(19) Europäisches Patentamt
European Patent Office
Office européen des brevets

(11) EP 4 026 904 A1

(12) EUROPEAN PATENT APPLICATION
published in accordance with Art. 153(4) EPC

(43) Date of publication:
13.07.2022 Bulletin 2022/28

(21) Application number: 20860748.1

(22) Date of filing: 03.09.2020

(51) International Patent Classification (IPC):
C12N 15/10 (2006.01)          G01N 30/72 (2006.01)
G01N 33/68 (2006.01)          C40B 40/08 (2006.01)
C40B 50/06 (2006.01)

(52) Cooperative Patent Classification (CPC):
C12N 15/10; C40B 40/08; C40B 50/06;
G01N 30/72; G01N 33/68

(86) International application number:
PCT/KR2020/011885

(87) International publication number:
WO 2021/045541 (11.03.2021 Gazette 2021/10)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 03.09.2019  KR 20190108722
28.08.2020  KR 20200108930

(71) Applicants:
• Osong Medical Innovation Foundation
Cheongju-si, Chungcheongbuk-do 28160 (KR)
• Kim, Sung Sub
Daejeon 34049 (KR)
(72) Inventors:
• KIM, Sung Sub
Daejeon 34049 (KR)
• CHOI, So Young
Sejong 30130 (KR)
• PARK, Ji Hoon
Sejong 30100 (KR)
• JIN, Jonghwa
Seoul 04369 (KR)
• JANG, Myung-Hee
Sejong 30150 (KR)

(74) Representative: V.O.
P.O. Box 87930
2508 DH Den Haag (NL)

(54) **METHOD FOR ULTRA-RAPIDLY SELECTING SIGNAL PEPTIDE TO WHICH INDIVIDUAL BARCODE SYSTEM FOR INCREASING PROTEIN PRODUCTIVITY IS INTRODUCED**

(57) The present invention relates to a composition for screening various signal peptides to select specific ones that allow efficient secretion of a target protein to out of host cells. The present invention also relates to a method for selecting specific signal peptides that express a target protein in host cells and efficiently secrete the target protein to out of the host cells. The use of the composition and/or method according to the present invention enables ultrafast selection of optimal signal peptides for a target protein through barcoding sequences corresponding to the signal peptides, leading to the maximization of the production yield of the recombinant protein.

[Fig. 5]

C-terminus of target protein · Barcoding sequence · His tag

....... GKGXXXXXGHHHHHHR

Trypsin cleavage site

## Description

### Technical Field

The present invention relates to a composition including barcoding sequences for screening various signal peptides to select specific ones that allow efficient secretion of a target protein to out of host cells.

### Background Art

With recent advances in biotechnology, a great deal of research around the world has focused on the structure and function of proteins. The explosive growth of the market for recombinant protein pharmaceuticals, including therapeutic antibodies, that are specific to target diseases and have few side effects is shaking up the global pharmaceutical market that has been dominated by small-molecule drugs.

Recombinant protein drugs refer to therapeutic proteins that are mass-produced in microbial or animal cell systems using genetic recombination technology. Therapeutic proteins have previously been difficult to obtain *in vivo.* Recombinant proteins can be modified such that they are expressed intracellularly or secreted extracellularly. When overexpressed intracellularly, recombinant proteins often accumulate into inactive insoluble aggregates in cells. Although recombinant proteins do not accumulate, there may arise many factors negatively affecting productivity, such as cumbersome cell disruption and difficult isolation and purification from numerous other proteins present in cells. These negative factors can be easily avoided by extracellular secretion of produced proteins. Extracellular secretion of proteins requires correct folding and modification of proteins after transcription, enabling the production of soluble proteins that are activated and have accurate tertiary structures for therapeutic protein production. Thus, the production of recombinant proteins via extracellular secretion is advantageous in terms of protein production yield as well as protein quality control. Therefore, optimization for efficient extracellular secretion of recombinant proteins is important in the manufacture of recombinant protein drugs.

Signal peptides are amino acid sequences that are located at the N-termini of secretory or membrane proteins and function as targeting signals for the secretory or membrane proteins. Very diverse signal peptides are known so far. For example, approximately 4,000-5,000 signal peptides are found in eukaryotes. The extracellular secretion rate of a target protein may vary depending on which signal peptides are applied for the production of the target protein. In this connection, several studies have been reported aimed at selecting signal peptides matched to target proteins and optimizing the extracellular secretion of the target proteins *(*Acta Biochim Biophys Sin (Shanghai) (2011) 43 (2): 96-102, Signal peptide replacements enhance expression and secretion of hepatitis C virus envelope glycoproteins. Biochem Biophys Res Commun. 2010 Jan 1;391(1): 931-5, Signal peptide design for improving recombinant protein secretion in the baculovirus expression vector system).

In conclusion, ultrafast screening of currently known 4,000 or more signal peptides to select the most suitable ones for the expression of target proteins and introduction of the selected signal peptides into the target proteins in the development of protein drugs can artificially induce the maximum production of recombinant protein drugs.

The description of the Background Art is merely provided for better understanding of the background of the invention and should not be taken as corresponding to the prior art already known to those skilled in the art.

### Detailed Description of the Invention

### Problems to be Solved by the Invention

The present inventors have earnestly and intensively conducted research to find a method for rapidly searching the most suitable signal peptides that allow highly efficient extracellular secretion of a target protein. As a result, the present inventors have found that when specific amino acids are combined into barcoding sequences corresponding to signal peptides, a library of the barcoding sequences and the signal peptides is constructed and expressed in host cells, and the amounts of a target protein in culture media of the host cells are determined, optimal signal peptides for efficient extracellular secretion of the target protein can be selected. Based on this finding, the present invention has been accomplished.

Therefore, it is one object of the present invention to provide a composition for screening various signal peptides to select specific ones that allow efficient secretion of a target protein to out of host cells.

It is a further object of the present invention to provide a method for selecting specific signal peptides that allow secretion of a target protein to out of host cells after expression of the target protein in the host cells.

Other objects and advantages of the invention become more apparent from the following detailed description, claims, and drawings.

**Means for Solving the Problems**

One aspect of the present invention provides a composition for screening various signal peptides to select specific ones that allow efficient secretion of a target protein to out of host cells.

The present inventors have made efforts to find a method for rapidly searching for the most suitable signal peptides that allow highly efficient extracellular secretion of a target protein. As a result, the present inventors have found that when specific amino acids are combined into barcoding sequences corresponding to signal peptides and the barcoding sequences are applied a target protein, optimal signal peptides for efficient extracellular secretion of the target protein can be selected.

As used herein, the term "signal peptide" refers to a peptide that is fused to the N-terminus (front end) of a secretory protein secreted from cells and allows the protein to pass through the cell membrane. The signal peptide is usually composed of 10 to 30 amino acids and is cleaved off by a specific transmembrane protease. The signal peptide allows only the secretory protein to secrete extracellularly. A total of 4137 signal peptides are known so far to be present in eukaryotic cells (Uniprot, June, 2017).

Extracellularly secreted proteins have their own signal peptides. That is, secreted proteins are expected to have different signal peptides depending on the type of expressing cells, the role of the proteins after expression or the expression of appropriate amounts of the proteins.

As used herein, the term "target protein" refers to a protein that is to be produced in proper host cells with high efficiency. The introduction of optimal signal peptides for a target protein into the recombinant protein in the development of a biopharmaceutical can artificially induce the maximum production of the target protein.

According to a preferred embodiment of the present invention, the composition includes polypeptides containing signal peptide tags (SP-tags) or nucleic acid molecules encoding the polypeptides.

As used herein, the term "signal peptide tag" or "SP-tag" refers to a tag that contains a barcoding sequence site consisting of three or more amino acids barcoding the corresponding signal peptide. The signal peptide tag can be fused to the C-terminus (rear end) of the target protein either directly or via a linker.

According to a preferred embodiment of the present invention, the three or more amino acids are selected from the group consisting of leucine (L), proline (P), alanine (A), tryptophan (W), tyrosine (Y), threonine (T), serine (S), glutamate (E), and aspartate (D).

Three out of the nine amino acids may be combined into a codon. In this case, a total of 729 ($9\times9\times9$) signal peptides can be barcoded with the codons. Alternatively, five out of the nine amino acids may be combined into a codon. In this case, a total of 59,049 ($9\times9\times9\times9\times9$) signal peptides can be barcoded with the codons. Considering that 4,137 signal peptides have been discovered so far in eukaryotes, each of the barcoding sequences preferably consists of 3 to 8 amino acids, more preferably 3 to 5 amino acids.

According to a preferred embodiment of the present invention, each of the barcoding sequences independently consists of the same three amino acids selected from the nine amino acids.

For example, each of the barcoding sequences may be independently selected from the group consisting of LLL, PPP, AAA, WWW, YYY, TTT, SSS, EEE, and DDD.

According to a preferred embodiment of the present invention, each of the barcoding sequences independently consists of the same two amino acids and one different amino acid selected from the nine amino acids.

For example, each of the barcoding sequences may independently have a configuration in which two identical amino acids are repeated consecutively, such as LLP, PPA, AAW, WWY, YYT, TTS, SSE, EED or DDL, or in which one different amino acid is inserted between two identical amino acids, such as LDL, PEP, ASA, WTW, YLY, TPT, SAS, EWE or DYD.

According to a preferred embodiment of the present invention, each of the barcoding sequences independently consists of three different amino acids selected from the nine amino acids.

For example, the barcoding sequence may be selected from LPA, PAW, AWY, WYT, YTS, TSE, SED, EDL, and DLP, each of which consists of a series of different amino acids.

According to a preferred embodiment of the present invention, each of the barcoding sequences independently consists of four different amino acids selected from the nine amino acids.

According to a preferred embodiment of the present invention, each of the barcoding sequences independently consists of a combination of four amino acids selected from the nine amino acids wherein some of the four amino acids are identical and some of the four amino acids are different.

According to a preferred embodiment of the present invention, each of the barcoding sequences independently consists of five identical amino acids selected from the nine amino acids.

According to a preferred embodiment of the present invention, each of the barcoding sequences independently consists of a combination of five amino acids selected from the nine amino acids wherein some of the five amino acids are identical and some of the five amino acids are different.

According to a preferred embodiment of the present invention, each of the barcoding sequences independently includes or is independently the sequence set forth in SEQ ID NO: 1:

$[L]_1[L]_2[L]_3$ (SEQ ID NO: 1) with the proviso that $[L]_1$, $[L]_2$ or $[L]_3$ is optionally replaced with an amino acid selected from the group consisting of P, A, W, Y, T, S, E, and D.

According to a preferred embodiment of the present invention, each of the barcoding sequences independently includes or is independently the sequence set forth in SEQ ID NO: 2:

$[L]_1[L]_2[L]_3[L]_4$ (SEQ ID NO: 2) with the proviso that $[L]_1$, $[L]_2$, $[L]_3$ or $[L]_4$ is optionally replaced with an amino acid selected from the group consisting of P, A, W, Y, T, S, E, and D.

According to a preferred embodiment of the present invention, each of the barcoding sequences independently includes or is independently the sequence set forth in SEQ ID NO: 3:

$[L]_1[L]_2[L]_3[L]_4[L]_5$ (SEQ ID NO: 3) with the proviso that $[L]_1$, $[L]_2$, $[L]_3$, $[L]_4$ or $[L]_5$ is optionally replaced with an amino acid selected from the group consisting of P, A, W, Y, T, S, E, and D.

In the Examples section that follows, the nine amino acids were selected as constituents for the barcoding sequences as follows:

(1) First, 18 amino acids were selected. K and R as trypsin cleavage sites were excluded from the 20 amino acids, which will be described later.

(2) 12 out of the 18 amino acids were selected. Specifically, V, F, G, C, H, and N with high or low hydropathy indices were excluded from the 18 amino acids. First, the expression levels of peptides, each containing a different one of the 18 amino acids, were measured by LC-MS analysis. As a result, the amino acids V, F, G, C, H, and N were found to have relatively high or low hydropathy indices (particularly, H is likely to affect His tags and change its charge).

(3) M was excluded from the 12 amino acids. M is susceptible to oxidation modification during protein digestion. A total of 11 amino acids were selected.

(4) I and Q were excluded from the amino acids I, Q, N, L, E, and D with similar masses (N was already excluded in (2)) and L, E and D only were used. Finally, the nine selected amino acids L, P, A, W, Y, T, S, E, and D were used to design the barcoding sequences.

According to a preferred embodiment of the present invention, each of the signal peptide tags may independently further include a proteolytic cleavage site at the N-terminus (front end) of the barcoding sequence such that the barcoding sequence is cleaved by the protein.

As used herein, the term "proteolytic cleavage site" refers to a site that is present in a protein or peptide to be cleaved and is recognizable by a specific protein, resulting in cleavage of the target protein or peptide.

Various proteolytic cleavage sites are known in the art. Examples of preferred proteolytic cleavage sites include, but are not limited to, trypsin cleavage sites, thrombin cleavage sites, enterokinase cleavage sites, Factor Xa cleavage sites, collagenase cleavage sites, and TEV protease cleavage sites.

According to a preferred embodiment of the present invention, the proteolytic cleavage site is a trypsin cleavage site.

Trypsin is a proteolytic enzyme that cleaves the peptide chain next to lysine (K) or arginine (R). The proteolytic cleavage site may be selected from the group consisting of K and R.

The proteolytic cleavage site may be directly linked to the barcoding sequence. Alternatively, the proteolytic cleavage site may be linked to the barcoding sequence via a linker. The linker may be any of those known in the art but is preferably glycine (G).

In the Examples section that follows, glycine (G) was selected as the linker. This selection is based on the following considerations. The presence of P next to K causes incomplete trypsin cleavage and the acidic residues D and E next to K slow down hydrolysis rate, resulting in miscleavage. Accordingly, P, D, and E are unsuitable as linkers. L, P, A, W, Y, T, S, E, and D used in the barcoding sequences are excluded for stable cleavage.

According to a preferred embodiment of the present invention, the linker includes at least one glycine (G).

According to a preferred embodiment of the present invention, each of the polypeptides containing signal peptide tags independently further includes an affinity tag at the end of the barcoding sequence. The affinity tag is used to isolate and purify the barcoding sequence from culture media of the host cells.

As used herein, the term "affinity tag" refers to a tag that binds to a molecule of interest *(e.g.,* a barcoding sequence) to allow isolation and purification of the molecule of interest using an affinity tag receptor. Various affinity tags known in the art can be used to implement the present invention.

The affinity tag may be selected from the group consisting of histidine tag (His-tag), myc-tag, FLAG-tag, small ubiquitin-like modifier tag (SUMO-tag), covalent yet dissociable NorpD peptide tag (CYD-tag), heavy chain of protein C tag (HPC-tag), calmodulin binding peptide tag (CBP-tag), and hemagglutinin-tag (HA-tag). The affinity tag is preferably a His-tag composed of 2 to 15 histidine residues.

Each of the signal peptide tags may be independently represented by Structure 1:

<Structure 1>

Trypsin cleavage site-linker-barcoding sequence-linker-affinity tag-trypsin cleavage site

Each of the signal peptide tags may be independently represented by Structure 2:

<Structure 2>

Lysine (K) or arginine (R)-glycine (G)-barcoding sequence-glycine (G)-affinity tag-lysine (K) or arginine (R)

More preferably, each of the signal peptide tags is independently represented by Structure 3:

<Structure 3>

Lysine (K)-glycine (G)-barcoding sequence-glycine (G)-affinity tag-arginine (R) The introduction of arginine (R) as a trypsin cleavage site at the C-terminus of the peptide ensures ionization stability of the peptide.

In the Examples section that follows, when a His tag was used as the affinity tag, arginine (R) was added to the C-terminus to ensure ionization stability because the terminal H is likely to change its charge. Arginine (R) was used also when isotope-labeled peptides were synthesized as internal standards to ensure quantitation. For reference, the arginine residue was $^{15}$N- and $^{13}$C-labeled.

According to a preferred embodiment of the present invention, the target protein is fused to the N-termini (front ends) of the signal peptide tags of the polypeptides.

According to a preferred embodiment of the present invention, the signal peptides are fused to the N-terminus (front end) of the target protein.

A further aspect of the present invention provides a vector including each of the nucleic acid molecules encoding the polypeptides containing signal peptide tags.

The nucleic acid molecule may be an isolated or recombinant one. Examples of such nucleic acid molecules include single- and double-stranded DNA and RNA and their corresponding complementary sequences. The isolated nucleic acid may be isolated from a naturally occurring source. In this case, the isolated nucleic acid is separated from the peripheral gene sequence present in the genome of a subject from which the nucleic acid is to be isolated. The isolated nucleic acid may be a nucleic acid, for example, a PCR product, a cDNA molecule or an oligonucleotide, that is enzymatically or chemically synthesized from a template. In this case, the nucleic acid produced from this procedure can be understood as the isolated nucleic acid molecule. The isolated nucleic acid molecule represents a nucleic acid molecule in the form of a separate fragment or as a component of a larger nucleic acid construct. A nucleic acid is "operably linked" when arranged in a functional relationship with another nucleic acid sequence. For example, the DNA of a presequence or secretory leader is operably linked to the DNA of the polypeptide when expressed as a preprotein, which is a presecretory polypeptide. A promoter or an enhancer affecting the transcription of the polypeptide sequence is operably linked to a coding sequence or a ribosome-binding site is operably linked to a coding sequence when it is arranged such that translation is promoted. Generally, the term "operably linked" means that DNA sequences to be linked are located adjacent to each other. In the case of secretory leaders, the term "operably linked" means that the secretory leaders are present adjacent to each other in the same leading frame. However, an enhancer needs not be contiguous. The linkage is performed by ligation at a convenient restriction enzyme site. In the case where this site does not exist, a synthetic oligonucleotide adaptor or a linker is used according to a suitable method known in the art.

As used herein, the term "vector" is used to refer to a carrier into which a nucleic acid sequence can be inserted for introduction into a cell where it can be replicated. A nucleic acid sequence may be "exogenous," or "heterologous". Examples of such vectors include, but are not limited to, plasmids, cosmids, and viruses (e.g., bacteriophage). One of skill in the art may construct a vector through standard recombinant techniques (Maniatis et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc, NY, 1994, etc.).

As used herein, the term "expression vector" refers to a vector containing a nucleic acid sequence coding for at least part of a gene product capable of being transcribed. In some cases, RNA molecules are then translated into a protein, polypeptide, or peptide. Expression vectors can contain a variety of regulatory sequences. In addition to regulatory sequences that govern transcription and translation, vectors and expression vectors may contain nucleic acid sequences that serve other functions as well.

As described above, a nucleic acid sequence encoding a polypeptide containing a signal peptide tag including a barcoding sequence encoded with specific amino acid codes corresponding to a signal peptide is constructed to have a "signal peptide-target protein-signal peptide tag" structure. The nucleic acid sequence can be inserted into a vector and expressed in a suitable host cell. In the case where the signal peptide allows efficient secretion of the "target protein-signal peptide tag" to the cell membrane of the host cell, the type of the signal peptide allowing efficient secretion of the target protein can be readily determined by validating the barcoding sequence of the signal peptide tag.

Another aspect of the present invention provides a method for selecting specific signal peptides that express a target protein in host cells and secrete the target protein to out of the host cells, the method including:

> 1) constructing vectors for various signal peptides to establish a library;
> 2) transforming host cells with the vectors;
> 3) expressing polypeptides containing signal peptide tags from the transformed host cells; and
> 4) quantifying the polypeptides containing signal peptide tags, the signal peptide tags or barcoding sequences of the signal peptide tags secreted to out of the transformed host cells.

As used herein, the term "host cell" refers to any transgenic organism that is capable of replicating the vector or expressing the gene encoded by the vector. Suitable organisms include eukaryotes and prokaryotes.

As used herein, the term "transformation" is intended to include "transfection" and "transduction". The host cell may be transfected, transduced or transformed with the vector. This process means the delivery or introduction of the exogenous nucleic acid molecule into the host cell.

The host cell is preferably a bacterial cell, yeast cell, animal cell (e.g., CHO cell) or human cell (e.g., HeLa cell, HEK293 cell, BHK cell, COS7 cell, COP5 cell, A549 cell, NIH3T3 cell, MDCK cell or WI38 cell) but is not limited thereto.

According to a preferred embodiment of the present invention, the method may further include 3-1) isolating and purifying the polypeptides containing signal peptide tags using affinity tags after step 3).

The method may further include 3-2) treating the polypeptides containing signal peptide tags with trypsin after step 3). The trypsin cleavage enables efficient isolation of the target protein and the barcoding sequences.

Steps 3-1) and 3-2) may be carried out individually or sequentially or in the reverse order after step 3).

In step 4), the polypeptides containing signal peptide tags, the signal peptide tags or the barcoding sequences of the signal peptide tags may be quantified by any suitable technique known in the art. Examples of such quantification techniques include, but are not limited to, protein chip analysis, immunoassay, ligand binding assay, matrix desorption/ionization time of flight (MALDI-TOF) mass spectrometry, surface enhanced laser desorption/ionization time of flight (SELDI-TOF) mass spectrometry, radioimmunoassay (RIA), radioimmunodiffusion, Ouchterlony immunodiffusion, rocket immunoelectrophoresis, immunohistochemical analysis, immunocytochemical analysis, immunoprecipation, complement fixation assay, two-dimensional electrophoresis analysis, liquid chromatography-mass spectrometry (LCMS), liquid chromatography-mass spectrometry/mass spectrometry (LC-MS/MS), Western blot, and enzyme linked immunosorbent assay (ELISA).

In the Examples section that follows, the results of quantification of the polypeptides containing signal peptide tags, the signal peptide tags or the barcoding sequences of the signal peptide tags by Western blot were compared with the results of ultrafast quantification of the polypeptides containing signal peptide tags, the signal peptide tags or the barcoding sequences of the signal peptide tags by LC-MS/MS. The ultrafast quantification of the barcoding sequences by LC-MS/MS revealed that three of the signal peptides had the highest expression levels. This result was consistent with the result of quantification by Western blot, which validated the efficacy of ultrafast quantification by LC-MS/MS.

For ease of ultrafast peptide quantification by LC-MS/MS, it is preferable that the signal peptide tags are up to 20 amino acids in length. It is more preferable that in Structure 1, each of the linkers consists of 1 to 3 amino acids, the barcoding sequence consists of 3 to 5 amino acids, and the affinity tag consists of 2 to 10 amino acids such that the full length is up to 20 amino acids. This construction is suitable for quantification by LC-MS/MS.

**Effects of the Invention**

The features and advantages of the present invention are summarized as follows:

> (i) The composition of the present invention is effective in screening various signal peptides to select optimal signal peptides for efficient extracellular secretion of a target protein.
> (ii) The method of the present invention is effective in selecting specific signal peptides that express a target protein in host cells and secrete the target protein to out of the host cells.
> (iii) The use of the composition and/or method according to the present invention enables ultrafast selection of optimal signal peptides for a target protein through barcoding sequences corresponding to the signal peptides, leading to the maximization of the production yield of the recombinant protein.

**Brief Description of the Drawings**

Fig. 1 schematically shows the principle of how a signal peptide tag (SP-tag) is used to determine a corresponding signal peptide.

Fig. 2 schematically shows a method for screening signal peptides capable of increasing protein production using SP-tags.

Fig. 3 exemplifies the construction of a vector expressing a signal peptide-target protein-SP-tag structure.

Fig. 4 exemplifies the construction of a library of 1200 signal peptides and 1200 SP-tags.

Fig. 5 shows a method for designing an SP-tag.

Fig. 6 shows the results of expression and purification of 18 SP-tagged proteins, each of which contains a different one of the 18 amino acids.

Fig. 7 shows the results of relative quantification for SP-tags, each of which contains a different one of the 18 amino acids.

Fig. 8 shows the results of validation of a method for constructing a library of 10 signal peptide vectors.

Fig. 9 schematically shows an experiment to validate a method for screening optimal signal peptides by LC-MS/MS.

Fig. 10 compares the experimental results of LC-MS/MS and Western blot analyses.

Fig. 11 shows the rankings of signal peptides based on the results of LC-MS/MS and Western blot.

Fig. 12 shows LC-MS/MS peaks representing the expression levels of 9 SP-tags.

Fig. 13 shows the results of screening of signal peptides using SP-tags and LC-MS/MS.

Fig. 14 shows relative quantitative peaks sorted according to peak area and intensity.

Fig. 15 is a table showing selected 10 signal peptides.

Fig. 16 shows values for correcting the results of MRM analysis for 16 groups with a global standard peptide (EQVTNVGGAVVTGVTAVAQK) based on an isotope labeled peptide that was absent in the samples.

Fig. 17 shows the results of screening of signal peptides suitable for aflibercept expression.

Fig. 18 compares the quantitative results of Western blot and OCTET for the individual expression levels of the top 24 peptides and the bottom 24 peptides, which were determined from the results of LC-MS/MS analysis.

Fig. 19 is a table showing signal peptides as secondary hits obtained by Western blotting and OCTET analyses.

## Mode for Carrying out the Invention

The present invention will be more specifically explained with reference to the following examples. It will be evident to those skilled in the art that the scope of the present invention is not limited by these examples according to the gist of the present invention.

## EXAMPLES

### *Experimental materials and methods*

### 1. Selection of amino acids for use in barcoding sequences

For efficient and equivalent ionization and detection in LC-MS/MS, 18 amino acids were selected and used in barcoding sequences. K and R as trypsin cleavage sites were excluded from a total of 20 amino acids. Tags, each containing a different one of the 18 amino acids, were fused to the C-terminus of one target protein. As a result, a total of 18 clones were constructed.

A target gene was constructed by overlapping PCR (SET15-R500, Solgent). The vectors and the gene were treated with restriction enzymes AscI (R0558S, New England Biolabs)/XhoI (R0146S, New England Biolabs). The vectors and the PCR products were mixed with a DNA ligation mixture (6023, Takara), ligated at room temperature for 10 min, and transformed into DH5α (CP010, Enzynomics) for cloning. 18 plasmids were expressed in FreeStyle 293-F cells (R79007, ThermoFisher Scientific) and the cell culture media were harvested 3 days later. The target protein in the cell culture media was isolated and purified by His affinity chromatography on Ni-NTA resin (05893801001, Roche). After pretreatment with the 18 proteins (each 1 μg), peak area values were calculated by LC-MS/MS quantification and were compared.

### 2. Signal peptide and SP-tag vector construction

Primers for 1200 SP-tags were synthesized (Cosmogenetech, Republic of Korea) and SP-tag genes were constructed by overlapping PCR (SET15-R500, Solgent). The vectors and the PCR products were treated with restriction enzymes AscI (R0558S, New England Biolabs)/XhoI (R0146S, New England Biolabs), followed by DNA ligation (6023, Takara). The ligation mixture was transformed into DH5α (CP010, Enzynomics) for cloning.

**3. Validation of protocol for target protein library construction**

10 vectors carrying 10 signal peptides and one target protein gene were separately treated with restriction enzymes AscI (R0558S, New England Biolabs)/XhoI (R0146S, New England Biolabs). The 10 vectors and the inserts were subjected to DNA ligation (6023, Takara). The ligation mixture was transformed into DH5α (CP010, Enzynomics) to construct a mini-library. To demonstrate the diversity of the mini-library, retransformation was performed and 100 colonies were picked and sequenced (Solgent, Republic of Korea).

**4. Validation of signal peptide screening**

Vectors carrying 9 SP-tags and one target protein gene were separately cloned to construct 9 clones. The 9 clones were mixed together or independently expressed in FreeStyle 293-F cells (R79007, ThermoFisher Scientific). 3 days later, cell culture media were harvested. Western blot was performed using anti-6×His antibody (ab18184, Abcam) to detect the target protein in the cell culture media. After pretreatment of the cell culture media by His affinity chromatography on Ni-NTA resin (05893801001, Roche), LC-MS/MS was performed to obtain peak area values. The Western blot results and LC-MS/MS results were analyzed and compared.

**5. Quantitation by multiple reaction monitoring (MRM)**

MRM was performed on a nano LC system (nanoACQUITY UPLC System Nano-LC, Waters) connected to a hybrid triple quadruple/ion trap mass spectrometer (6500QTRAP, AB SCIEX, USA) with a nanoelectrospray interface. The mass spectrometer was operated in positive ion MRM mode in which Q1 and Q3 were set to transmit different precursor/product ion pairs.

The LC buffer system was as follows: mobile phase A, 2% acetonitrile/0.1% formic acid (Fisher Scientific, Cat# LS118-4) and mobile phase B, 98% acetonitrile/0.1% formic acid (Fisher Scientific, Cat# LS120-212). The peptides were separated and eluted at a flow rate of 300 nl/min on a linear concentration gradient of mobile phase B from 5% to 95% in 45 min. The total LC run time was 60 min. A trap (100 Å, 5 μm, 180 μm×20 mm, 2G, V/M, Cat# 186006527) and an analytical column (130 Å, 1.7 μm, 100 μm×100, Cat# 186003546) were used.

Typical instrument settings were as follows: ion spray (IS) voltage 2.3 kV, interface heater temperature 300 °C, GS1 (nebulizer gas) setting 15, and curtain gas setting 20. MS coefficients for declustering potential (DP) and collision energy (CE) were determined by linear regression of optimized values for corresponding peptides and manual tuning. MRM experiments were conducted with a scan time of 20 ms and a scan width of 0.002 m/z using resolutions of 0.2 and 0.7 Da (FWHM) for Q1 and Q3, respectively. In the MRM runs, the scan time was maintained at 20 ms for each transition and the pause between transition scans was set to 1 ms. MRM transitions were selected to monitor fragment ions with the highest intensity.

**6. Sample pretreatment (in solution digestion)**

Proteins were treated for MRM analysis as follows. Sample proteins were quantified by bicinchoninic acid (Pierce™ BCA Protein Assay Kit, Thermo Scientific™, 23225) assay. The protein samples (each 100 μg) were denatured with 6 M urea (GE Healthcare, PN 17-1319-01), 50 mM Tris, pH 8.0 (Invitrogen, AM9855G), and 30 mM dithiothreitol (DTT, Sigma-Aldrich, PN D0632-10G) at 37 °C for 60 min and alkylated with 50 mM iodoacetamide (IAA, Sigma-Aldrich, PN I1149-25G) at room temperature in the dark for 30 min. Urea was diluted 15-fold with 50 mM Tris (pH 8.0), trypsin/Lys-C (Promega, PN V0571) was added in a 1:50 (w/w) enzyme-to-protein concentration ratio, followed by culture at 37 °C overnight.

Tryptic digestion was stopped by the addition of formic acid (SIGMA Aldrich, F0507) at a final concentration of 1% and desalting was performed on OASIS cartridges (Waters, PN WAT094225). The cartridges were equilibrated with 3 ml of water containing 0.1% formic acid and 3 ml of methanol before use. After use, the cartridges were washed with 3 ml of 0.1% formic acid and eluted with 1 ml of 60% ACN and 0.1% formic acid. The eluted samples were dried on a speed vacuum and frozen. Prior to MRM analysis, the samples were dissolved to a concentration of 0.2 μg/μl in 0.1% formic acid.

**7. SP-tag design**

Each SP-tag consists of a cleavage site, a barcoding sequence, and a His tag. At least 14-20 amino acids can be used in SP-tags. Up to 20 amino acids can be quantified by LC-MS analysis. The present inventors utilized 14 amino acids in the following manner. SP-tags without quantitative differences were designed from 1200 peptides with different masses while maintaining their length at 14 amino acids.

**(1) Barcoding sequence selection**

(1) Protein peptides containing at least 8 amino acids and up to 20 amino acids were used for MRM analysis. R or K as a trypsin cleavage site was excluded from the peptides. That is, 18 out of the 20 amino acids were selected.
(2) Then, 12 out of the 18 amino acids were selected. Specifically, V, F, G, C, H, and N with high or low hydropathy indices were excluded from the 18 amino acids. First, the expression levels of peptides, each containing a different one of the 18 amino acids, were measured by LC-MS analysis to determine whether they were the same or different. As a result, the amino acids V, F, G, C, H, and N were found to have relatively high or low hydropathy indices (Particularly, H is likely to affect His tags and change its charge).
(3) M was excluded from the 12 amino acids. M is susceptible to oxidation modification during protein digestion. A total of 11 amino acids were selected.
(4) I and Q were excluded from the amino acids I, Q, N, L, E, and D with similar masses (N was already excluded in (2)) and L, E and D only were used. Finally, the nine selected amino acids L, P, A, W, Y, T, S, E, and D were used to design barcoding sequences consisting of 5 amino acids.

**(2) Cleavage site and His tag design**

For trypsin cleavage, lysine (K) as a cleavage site was introduced in front of the peptide. For peptide ionization stability and quantification, arginine (R) was introduced at the end of the peptide.
G was introduced after the lysine (K) to prevent the appearance of continuous sequences such as KP, KD, and KE. The reasons for the selection of G were as follows: (1) P, D, and E were excluded because the presence of P next to K causes incomplete trypsin cleavage and the acidic residues D and E next to K slow down hydrolysis rate, resulting in miscleavage; (2) L, P, A, W, Y, T, S, E, and D were excluded because they were used in the barcoding sequences; and (3) G was suitable for stable cleavage.

6×histidine tags were attached for selective isolation and purification of the target protein.

*Experimental results*

**1. Design of method using signal peptide tags (SP-tags) to find corresponding signal peptides**

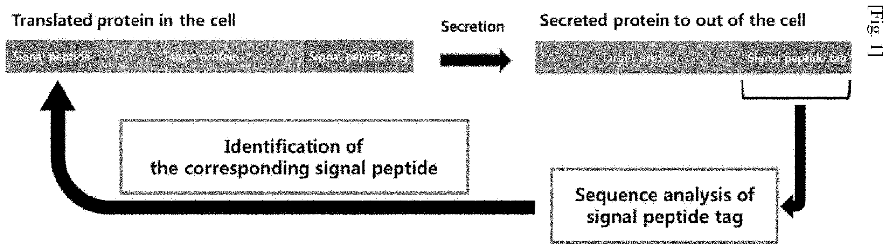

Signal peptides at the N-terminus of a protein that is translated in cells play a very important role in protein secretion. Grafting of various signal peptides for recombinant protein production promotes extracellular protein secretion, leading to an increase in production yield. The present inventors have succeeded in developing an ultrafast search method to find optimal signal peptides for protein production. Since signal peptides at the N-terminus of a protein are cleaved in cells, the signal peptides are not present in the secreted protein. Thus, the present inventors have found that when signal peptide tags (SP-tags) are attached to the C-terminus of a protein and are identified, the corresponding signal peptides can be determined (Fig. 1).

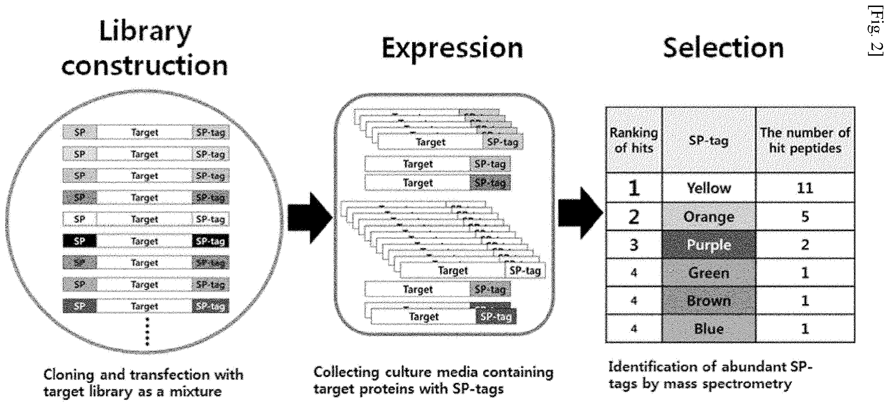

Fig. 2 schematically shows a method for screening signal peptides capable of increasing protein production using SP-tags. Individual steps of the method will be explained below.

1) Target protein library construction: A target protein is cloned into vectors carrying various signal peptides and corresponding SP-tags with different molecular weights to construct a target protein library.
2) Expression of the target protein library: Several days after transfection of the target protein library into animal cells, cell culture media containing the protein secreted to out of the cells are collected. The amount of the secreted protein varies depending on the type of the signal peptide and can be determined by the SP-tag corresponding to the signal peptide.
3) Selection of signal peptides capable of increasing protein production: The SP-tags in the cell culture media containing the produced protein are identified and their relative amounts are quantified by LC-MS/MS. The identified SP-tags are ranked according to their relative amounts and the corresponding signal peptides are determined to find optimal ones capable of increasing protein production.

## 2. Construction of library of signal peptides and SP-tags

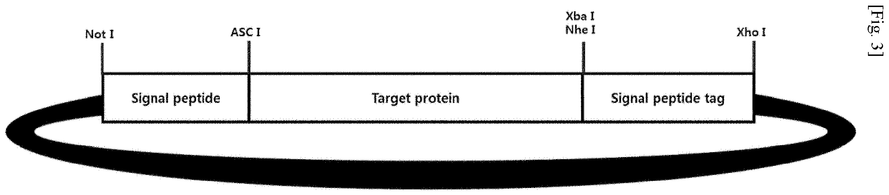

The target protein between the signal peptides and the SP-tags was cloned using restriction enzymes ASC I-Xba I/Nhe I to construct vectors having signal peptide-target protein-SP-tag structures (Fig. 3).

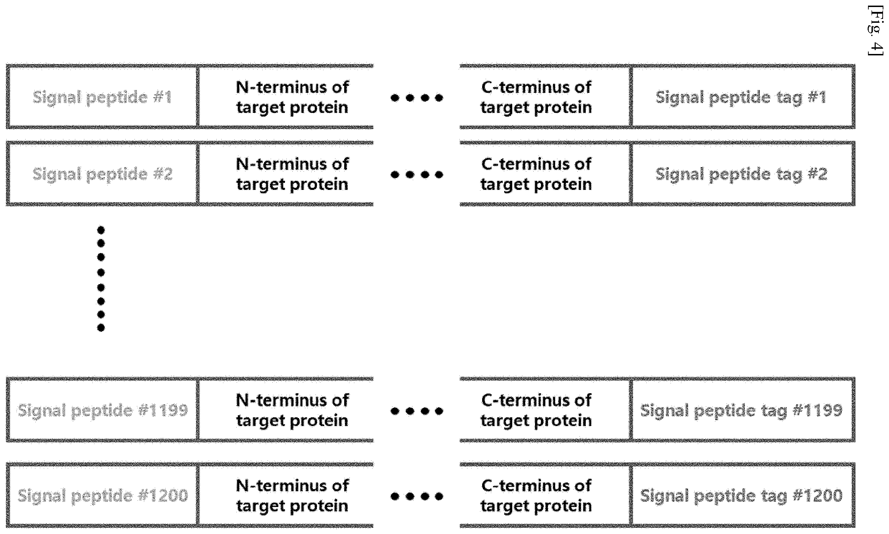

As shown in Fig. 4, vectors carrying 1200 signal peptides were cloned with 1200 SP-tags corresponding to the signal peptides to construct a library. The 1200 signal peptides used for library construction were signal peptides of already known human secretory proteins (Table 1).

[Table 1] 1200 human signal peptides constituting the library

| No. | Name of signal peptides | Amino acid sequence |
|---|---|---|
| 1 | Afamin | MKTT KTTGFIFFLFFLTESLT |
| 2 | Alkaline phosphatase | MQGPWVLLLLGLRLQLSLG |
| 3 | Alpha-1-antitrypsin | MPSSVSWGILLLAGLCCLVPVSLA |
| 4 | Alpha-2-macro globulin | MGKNKLLHPSLVLLLL VLLPTDA |
| 5 | Angiogenin | MVMGLGVLLLVFVLGLGLTPPTLA |
| 6 | Apolipoprotein E | MKVLWAALLVTFLAGCQA |
| 7 | Appetite-regulating hormone | MPSPGTVCSLLLLGMLWLDLAMA |
| 8 | Arylsulfatase A | MGAPRSLLLALAAGLAVA |
| 9 | Azurocidin | MTRLTVLALLAGLLASSRAGSSPLLD |
| 10 | Cadherin-2 | MCRIAGALRTLLPLLAALLQASVEA |
| 11 | Calcitonin | MGFQKFSPFLALSILVLLQAGSLHA |
| 12 | Carboxypeptidase Al | MRGLLVLSVLLGAVFG |
| 13 | Cathepsin B | MWQLWASLCCLLVLANA |
| 14 | Chromogranin-A | MRSAAVLALLLCAGQVTA |
| 15 | Cortistatin | MPLSPCTT T T T T SGATAT |
| 16 | Elastin | MAGLTAAAPRPGVLLLLLSILHPSRP |
| 17 | Fibroblast growth factor 4 | MSGPGTAAVALLPAVLLALLAPWAGRGGAA |
| 18 | Fibronectin | MLRGPGPGLLLLAVQCLGTAVPSTGASKSKR |
| 19 | Fibulin-1 | MERAAPSRRVPLPLLLLGGLALLAAGVDA |
| 20 | Gastric triacylglycerol lipase | MWLLLTMASLISVLGTTHG |
| 21 | Gastrin | MQRLCVYVLIFALALAAFSEA |
| 22 | Glucosylceramidase | MAGSLTGLLLLQAVSWASG |
| 23 | High IgG Fc receptor I | MWFLTTLLLWVPVDG |
| 24 | Histatin-3 | MKFFVFALILALMLSMTGA |
| 25 | Human Serum Albumin | MKWVTFISLLFLFSSAYS |
| 26 | Insulin | MALWMRLLPLLALLALWGPDPAAA |
| 27 | Insulin-like growth factor 1 | MGKISSLPTQLFKCCFCDFLK |
| 28 | Interferon alpha-17 | MALSFSLLMAVLVLSYKSICSLG |
| 29 | Interleukin-2 | MQLLSCIALILALV |
| 30 | Lactotransferrin | MKLVFLVLLFLGALGLCLA |
| 31 | Nephronectin | MDFLLALVLVSSLYLQAAA |
| 32 | Neuropeptide B | MARSATLAAAALALCLLLAPPGLA |

(continued)

| No. | Name of signal peptides | Amino acid sequence |
|---|---|---|
| 33 | Neurotrophin 4 | MLPLPSCSLPILLLFLLPSVPIES |
| 34 | Neutrophil defensin 1 | MRTLAILAAILLVALQAQA |
| 35 | Oxytocin | MAGSSLACCLLGLLALTSA |
| 36 | Pancreatic alpha amylase | MKFFLLLFTIGFCWA |
| 37 | Pancreatic prohormone | MAAARLCLSLLLLSTCVALLLQPLLGAQG |
| 38 | Pepsin A4 | MKWLLLLGLVALSEC |
| 39 | Proenkephalin-A | MARFLTLCTWLLLLGPGLLATVRA |
| 40 | Resistin | MKALCLLLLPVLGLLVSS |
| 41 | slit3 | MAPGWAGVGAAVRARLALALALASVLSGPPAVA |
| 42 | SOD3 | MLALLCSCLLLAAGASDA |
| 43 | Statherin | MKFLVFAFILALMVSMIGA |
| 44 | Thromopoietin | MELTELLLVVMLLLTARLTLS |
| 45 | Tissue-type plasminogen activator | MDAMKRGLCCVLLLCGAVFVSP |
| 46 | Trypsinogen-2 | MNLLLILTFVAAAVA |
| 47 | Tyrosinase | MLLAVLYCLLWSFQTSAG |
| 48 | Uteroglobin | MKLAVTLTLVTLALCCSSASA |
| 49 | Vascular endothelial growth factor A | MNFLLSWVHWSLALLLYLHHAKWSQA |
| 50 | Wnt-1 | MGLWALLPGWVSATLLLALAALPAALA |
| 51 | Acidic mammalian chitinase | MTKTTTT TGLVLILNLQLGSA |
| 52 | Acrosin | MVEMLPTAILLVLAVSVVA |
| 53 | Acrosomal protein SP-10 | MNRFLLLMSLYLLGSARGTSS |
| 54 | Acyloxyacyl hydrolase | MQSPWKILTVAPLFLLLSLQSSA |
| 55 | ADAM DEC 1 | MLRGISQLPAVATMSWVLLPVLWLIVQTQA |
| 56 | ADAMTS-like protein 1 | MECCRRATPGTLLLFLAFLLLSSRTARS |
| 57 | Adipolin | MRRWAWAAVVVLLGPQLVLL |
| 58 | Adiponectin | MLLLGAVLLLLALPGHDQ |
| 59 | Adrenomedullin | MKLVSVALMYLGSLAFLGADT |
| 60 | Agouti-related protein | MLTAAVLSCALLLALPATRG |
| 61 | Agouti-signaling protein | MDVTRLLLATLLVFLCFFTANS |
| 62 | Agrin | MAGRSHPGPLRPLLPLLVVAACVLPGAGG |
| 63 | Alpha-1B-glycoprotein | MSMLVVFLLLWGVTWGPVTEA |
| 64 | Alplia-2-niacroglobulin-like protein 1 | MWAQLLLGMLALSPAIA |
| 65 | Alpha-fetoprotein | MKWVESIFLIFLLNFTES |
| 66 | Alpha-lactalbumin | MRFFVPLFLVGILFPAILA |
| 67 | Ameloblastin | MSASKIPLFKMKDLILILCLLEMSFA |
| 68 | Amelogenin | MGTWILFACLLGAAFA |
| 69 | Amelotin | MRSTILLFCLLGSTRS |
| 70 | Amiloride-sensitive amine oxidase | MGRGTLALGWAGAALLLLQMLAAA |

(continued)

| No. | Name of signal peptides | Amino acid sequence |
|---|---|---|
| 71 | Amphiregulin | MRAPLLPP APVVLSLLILG |
| 72 | Amyloid beta A4 protein | MLPGLALLLLAAWTARA |
| 73 | Angiotensinogen | MRKRAPQSEMAPAGVSLRATILCLLAWAGLAAG |
| 74 | Anosmin-1 | MVPGVPGAVLTLCLWLAASSGCLA |
| 75 | Antileukoproteinase | MKSSGLFPFLVLLALGTLAPWAVEG |
| 76 | Apelin | MNLRLCVQALLLLWLSLTAVCG |
| 77 | Apolipoprotein A-I | MKAAVLTLAVLFLTGSQA |
| 78 | Apolipoprotein B-100 | MDPPRPALLALLALPALLLLLLAGARA |
| 79 | Aspartylglucosaminidase | MARKSNLPVLLVPFLLCQALVRC |
| 80 | Asporin | MKEYVLLLFLALCS |
| 81 | Augurin | MAASPARPAVLALTGGALLLLLCWGPGGISG |
| 82 | Basigin | MAAALFVLLGFALLGTHGASG |
| 83 | Beta-2-glycoprotein 1 | MISPVLILFSSFLCHVAIA |
| 84 | Beta -2-micro globulin | MSRSVALAVLALLSLSGLEA |
| 85 | Beta-hexosaminidase subunit alpha | MTSSRLWFSLLLAAAFAGRATA |
| 86 | Beta-microseminoprotein | MNVLLGSVVIFATFVTLCNA |
| 87 | Beta-secretase 2 | MGALARALLLPLLAQWLLRA |
| 88 | Biglycan | MWPLWRLVSLLALSQA |
| 89 | Bone marrow proteoglycan | MKLPLLLALLFGAVSA |
| 90 | Brevican core protein | MAQLFLPLLAALVLAQAPAALA |
| 91 | Calreticulin | MLLSVPLLLGLLGLAVA |
| 92 | Calsequestrin-2 | MKRTHLFI VGIYFLSSCRA |
| 93 | Carboxvlesterase 1 | MWLRAFILATLSASAAW |
| 94 | Cartilage intermediate layer protein 1 | MVGTKAWVFSFLVLEVTSVLG |
| 95 | Cerebellin-1 | MLGVLELLLLGAAWLAGPARG |
| 96 | Ceruloplasmin | MKILILGIFLFLCSTPAWA |
| 97 | Cholecvstokinin | MNSGVCLCVLMAVLAAGALT |
| 98 | Cholinesterase | MHSKVTIICIRFLFWFLLLCMLIGKSHT |
| 99 | Chondroadherin-like protein | MEGPRSSTHVPLVLPLLVLLLLAPARQAAA |
| 100 | Chordin | MPSLPAPPAPLLLLGLLLLGSRPARG |
| 101 | Choriogonadotropin subunit beta 3 | MEMFQGLLLLLLLSMGGTWA |
| 102 | Chorionic somatomammotropin hormone 2 isoform 2 | MAAGSRTSLLLAFALLCLPWLQEAGA |
| 103 | Chvmase | MLLLPLPLLLFLLCSRAEA |
| 104 | Clusterin | MMKTLLLFVGLLLTWESGQVLG |
| 105 | Coagulation factor V | MFPGCPRLWVLVVLGTSWVGWGSQGTEA |
| 106 | Colipase | MEKILILLLVALSVAYA |
| 107 | Collagen alpha-1(I) chain | MFSFVDLRLLLLLAATALLTHG |

(continued)

| No. | Name of signal peptides | Amino acid sequence |
|---|---|---|
| 108 | Complement C3 | MGPTSGPSLLLLLLTHLPLALG |
| 109 | Contactin-1 | MKMWLLVSHLVIISITTCLA |
| 110 | Corneodesmosin | MGSSRAPWMGRVGGHGMMALLLAGLLLPGTLA |
| 111 | Corticoliberin | MRLPLLVSAGVLLVALLPCPPCRA |
| 112 | Corticosteroid-binding globulin | MPLLLYTCLLWLPTSGLWTVQA |
| 113 | Corticotropin-releasing factor-binding protein | MSPNFKLOCHFILIFLTALRGESR |
| 114 | C-reactive protein | MEKLLCFLVLTSLSHAFG |
| 115 | Cubilin | MMNMSLPFLWSLLTLLIFAEVNG |
| 116 | Cvstatin-C | MAGPLRAPLLLLAILAVALAVSPAAG |
| 117 | Deoxyribonuclease-1 | MRGMKLLGALLALAALLQGAVS |
| 118 | Dermatopontin | MDLSLLWVLLPLVTMAWG |
| 119 | Dermcidin | MRFMTLLFLTALAGALVCA |
| 120 | Dickkopf-related protein 1 | MMALGAAGATRVFVAMVAAALGGHPLLGVSA |
| 121 | Dystrolycan | MRMSVGLSLLLPLSGRTFLLLLSVVMAQS |
| 122 | EGF-like domain-containing protein 1 | MGAAAVRWHLCVLLALGTRGRLA |
| 123 | Enamelin | MLVLRCRLGTSFPKLDNLVPKGKMKILLVFLGLLGNSVA |
| 124 | Endothelin-1 | MDYLLMIFSLLFVACQG |
| 125 | Endothelin-2 | MVSVPTTWCSVALALLVALHEGKG |
| 126 | Ephrin-A2 | MAPAQRPLLPLT T T T T PLPPPPF A |
| 127 | Epiphycan | MKTLAGLVLGLVIFDAAVT |
| 128 | Eppin | MGSSGLLSLLVLFVLLANVQG |
| 129 | Erythroferrone | MAPARRPAGARLLLVYAGLLAAAAAGLG |
| 130 | Erythropoietin | MGVHECPAWLWLLLSLLSLPLGLPVLG |
| 131 | E-selectin | MIASQFLSALTLVLLIKESGA |
| 132 | Fibromodulin | MQWTSLLLLAGLFSLSQA |
| 133 | Follicular dendritic cell secreted peptide | MKKVLLLITAILAVAVG |
| 134 | Follistatin | MVRARHQPGGLCLT T T T T CQFMEDRSAQA |
| 135 | Furin | MELRPWLLWVVAATGTLVLLAADA |
| 136 | Galanin peptides | MARGSALLLASLLLAAALS |
| 137 | Galanin-like peptide | MAPPSVPLVLLLVLLLSLAETPAS |
| 138 | Gastrokine-1 | MLAYSSVHCFREDKMKFTIVFAGLLGVFLAPALA |
| 139 | Glucagon | MKSIYFVAGLFVMLVOGSW0 |
| 140 | Glycophorin-A | MYGKIIFVLLLSEIVSISA |
| 141 | Glypican-1 | MELRARGWWLLCAAAALVACARG |
| 142 | GPHB5 protein | MKLAFLFLGPMALLLLAGYGCVLG |
| 143 | Granulysin | MATWALLLLAAMLLGNPGLVFS |

(continued)

| No. | Name of signal peptides | Amino acid sequence |
|---|---|---|
| 144 | Granzyme B | MQPILLLLAFLLLPRADA |
| 145 | Growth hormone variant | MAAGSRTSLLLAFGLLCLSWLQEGSA |
| 146 | Guanvlin | MNAFLLSALCLLGAWAALAGG |
| 147 | Haptoglobin | MSALGAVIALLLWGQLFA |
| 148 | Heparanase | MLLRSKPALPPPLMLLLLGPLGPLSPGALPRPAQA |
| 149 | Hepatocyte growth factor | MWVTKLLPALLLQHVLLHLLLLPIAIPYAEG |
| 150 | Hepcidin | MALSSQIWAACLLLLLLLASLTSG |
| 151 | Ig heavy chain V-III region 23 | MEFGLSWLFLVAILKGVQC |
| 152 | Ig kappa chain V-III region HIC | METPAQLLFLLLLWLPDTTG |
| 153 | Insulin-like peptide INSL6 | MPRLLRLSLLWLGLLLVRFS |
| 154 | Integrin alpha-2 | MGPERTGAAPLPLLLVLALSQGILNCCLA |
| 155 | Intelectin-1 | MNQLSFLLFLIATTRGWS |
| 156 | Interferon alpha-inducible protein 27-like protein 2 | MMKRAAAAAVGGALAVGAVPVVLS |
| 157 | Interleukin-2 | MYRMQLLSCIALSLALVTNS |
| 158 | Interleukin-6 | MNSFSTSAFGPVAFSLGLLLVLPAAFPAP |
| 159 | Kallikrein -1 | MWFLVLCLALSLGGTGAA |
| 160 | Kallikrein -6 | MKKLMVVLSLIAAAWA |
| 161 | Keratocan | MAGTICFIMWVLFITDTVWS |
| 162 | Kininogen-1 | MKLITILFLCSRLLLSLT |
| 163 | Klotho | MPASAPPRRPRPPPPSLSLLLVLLGLGGRRLRA |
| 164 | Lactoperoxidase | MRVLLHLP ALLASLILLQAAASTTRA |
| 165 | Legumain | MVWKVAVFLSVALGIGA |
| 166 | Leptin | MHWGTLCGFLWLWPYLFYVQA |
| 167 | Lumican | MSLSAFTLFLALIGGTSG |
| 168 | Lymphotactin | MRLLILALLGICSLTAYIVEG |
| 169 | Lvsyl oxidase homolog 2 | MERPLCSHLCSCLAMLALLSPLSLA |
| 170 | Mammalian ependymin-related protein 1 | MPGRAPLRTVPGALGAWLLGGLWAWTLCGLCSLGAVG |
| 171 | Mannose-binding protein C | MSLFPSLPLLLLSMVAASYS |
| 172 | Matrilin-2 | MEKMLAGCFLLILGQIVLLP AEA |
| 173 | Metalloproteinase inhibitor 2 (TIMP2) | MGAAARTLRLALGLLLLATLLRPADA |
| 174 | Methyltransferase-like protein 7B | MDILVPLLQLLVLLLTLPLHLMA |
| 175 | Midkine | MQHRGFLLLTLLALLALTSA |
| 176 | Mucin-5AC | MSVGRRKLALLWALALALACTRHTGHA |
| 177 | Muellerian-inhibiting factor | MRDLPLTSLALVLSALGA |
| 178 | Multimerin-1 | MKGARLFVLLSSLWSGGIG |
| 179 | Myostatin | MQKLQLCVYIYLFMLIVA |

(continued)

| No. | Name of signal peptides | Amino acid sequence |
|-----|-------------------------|---------------------|
| 180 | Napsin-A | MSPPPLLQPLLLLLPLLNVEPSGAT |
| 181 | Natriuretic peptides A | MSSFSTTTVSFLLLLAFQLLGQTRA |
| 182 | Natriuretic peptides B | MDPQTAPSRALLLLLFLHLAFLGGRS |
| 183 | Netrin-4 | MGSCARLLLLWGCTVVAA |
| 184 | Neural cell adhesion molecule 1 | MLQTKDLIWTLFFLGTAVS |
| 185 | Neurexophilin-2 | MRLRPLPLVVVPGLLQLLFCDS |
| 186 | Neurocan core protein | MGAPFVWALGLLMLQMLLFVAG |
| 187 | Neuroendocrine convertase 1 | MERRAWSLQCTAFVLFCAWCALNSAKA |
| 188 | Neuroendocrine convertase 2 | MKGGCVSQWKAAAGFLFCVMVFASA |
| 189 | Neuromedin-B | MARRAGGARMFGSLLLFALLAAGV |
| 190 | Neurosecretory protein VGF | MKALRLSASALFCLLLINGLGA |
| 191 | Niemann-Pick CI protein | MTARGLALGLLLLLLCPAQVFS |
| 192 | Noggin | MERCPSLGVTLYALVVVLGLRATPAGG |
| 193 | Norrin | MRKHVLAASFSMLSLLVIMGDTDS |
| 194 | Nyctalopin | MKGRGMLVLLLHAVVLGLPSAWA |
| 195 | Olfactomedin-4 | MRPGLSFLLALLFFLGQAAG |
| 196 | Osteo glycin | MKTLQSTLLLLLLVPLIKP |
| 197 | Otospiralin | MQACMVPGLALCLLLGPLAGA |
| 198 | Ovochymase-2 | MLISRNKLILLLGIVFFERGKS |
| 199 | Palmitoyl-protein thioesterase 1 | MASPGCLWLLAVALLPWTCASRALQHL |
| 200 | Pancreatic lipase-related protein 3 | MLGIWIVAFLFFGTSRG |
| 201 | Parathyroid hormone | MIPAKDMAKVMIVMLAICFL TKSDG |
| 202 | Peptide YY | MVFVRRPWPALTTVLLALLVCLGALVDA |
| 203 | Persephin | MAVGKFLLGSLLLLSLQLGQG |
| 204 | Pikachurin | MDLIRGVLLRLLLLASSLGPGAVS |
| 205 | Pituitary adenylate cyclase-activating polypeptide | MTMCSGARLALLVYGIIMHSSVYS |
| 206 | Placenta-specific protein 1 | MKVFKFIGLMILLTSAFSAGSG |
| 207 | Plasma serine protease inhibitor | MQLFLLLCLVLLSPQGASL |
| 208 | Platelet-derived growth factor receptor beta | MRLPGAMPALALKCELLLLSLLLLLFPQISQG |
| 209 | Prenylcysteine oxidase 1 | MGRVVAELVSSLLGLWLLLCSCGCPEG |
| 210 | Prepronociceptin | MKVLLCDLLLLSLFSSVFS |
| 211 | Procollagen-lysine,2 -oxoglutarate 5 -dioxygenase 3 | MTSSGPGPRFLLLLPLLLPPAASA |
| 212 | Proenkephalin-B | MAWQGLVLAACLLMFPSTTA |
| 213 | Pro-epidermal growth factor | MLLTLIILLPVVSKFSFVSLSA |
| 214 | Proepiregulin | MTAGRRMEMLCAGRVPALLLCLGFHLLQA |
| 215 | Progonadoliberin-1 | MKPIQKLLAGLILLTWCVEGCSS |

(continued)

| No. | Name of signal peptides | Amino acid sequence |
|---|---|---|
| 216 | Prokineticin-1 | MRGATRVSIMLLLVTVSDC |
| 217 | Prokineticin-2 | MRSLCCAPLLLLLLLPPLLLTPRAGDA |
| 218 | Prolactin releasing hormone | MKVLRAWLLCLLMLGLALRGAAS |
| 219 | Prolactin-inducible protein | MRLLQLLFRASPATLLLVLCLQLGANKA |
| 220 | Prolyl 3-hydroxylase 2 | MRERIWAPPLLLLPLLLPPPLWG |
| 221 | Prolyl 4-hydroxylase subunit alpha-1 | MIWYILIIGILLPQSLA |
| 222 | Promotilin | MVSRKAVAALLVVHVAAMLASQTEA |
| 223 | Pro-neuropeptide Y | MLGNKRLGLSGLTLALSLLVCLGALAEA |
| 224 | Pro-opiomelanocortin | MPRSCCSRSGALLLALLLQASMEVRG |
| 225 | Prorelaxin H2 | MPRLFFFHLLGVCLLLNQFSRAVA |
| 226 | ProSAAS | MAGSPLLWGPRAGGVGLLVLLLLGLFRPPPALC |
| 227 | Prosalusin | MAAATRGCRPWGSLLGLLGLVSAAAA |
| 228 | Prostaglandin-H2 D-isomerase | MATHHTLWMGLALLGVLGDLQA |
| 229 | Prostate and testis expressed protein 2 | MLVLFLLGTVFLLCPYWGELHDPIKA |
| 230 | Protachykinin-1 | MKFLVALAVFFLVSTQLFA |
| 231 | Protein disulfide-isomerase | MLRRALLCLAVAALVRA |
| 232 | Protein O-glucosyltransferase 1 | MEWWASSPLRLWLLLFLLP SAQG |
| 233 | Prothrombin | MAHVRGLQLPGCLALAALCSLVHS |
| 234 | Pro-thyrotropin-releasing hormone | MPGPWLLLALALTLNLTGVPGGRA |
| 235 | Pulmonary surfactant-associated protein D | MLLFLLSALVLLTQPLGYLE |
| 236 | Relaxin-3 | MARYMLLLLLAVWVLTGELWPGAEA |
| 237 | Renalase | MAQVLIVGAGMTGSLCA |
| 238 | Renin | MDGWRRMPRWGLLLLLWGSCTFG |
| 239 | Reticulocalbin-2 | MRLGPRTAALGLLLLCAAAAGA |
| 240 | Sarcalumenin | MRALVLLGCLLASLLFSGQA |
| 241 | Sclerostin | MQLPLALCLVCLLVHTAFRVVEG |
| 242 | Secretin | MAPRPLLLLLLLGGSAA |
| 243 | Secretoglobin family 1D member 1 | MRLSVCLLLLTLALCCYRANA |
| 244 | Secretogranin-1 | MQPTLLLSLLGAVGLAAVNS |
| 245 | Selenoprotein M | MSLLLPPLALLLLAALVAPATA |
| 246 | Semenogelin-1 | MKPNIIFVLSLLLILFKQAAVMG |
| 247 | Serglycin | MMQKLLKCSRLVLALALILVLESSVQG |
| 248 | Serotransferrin | MRLAVGALLVCAVLGLCLA |
| 249 | Serum amyloid A-1 protein | MKLLTGLVFCSLVLGVSS |
| 250 | Serum amyloid P-component | MNKPLLWISVLTSLLEAFA |
| 251 | Somatomedin-B and thrombospondin type-1 domain-containing protein | MRTLWMALCALSRLWPGAQA |
| 252 | Somatotropin | MATGSRTSLLLAFGLLCLPWLQEGSA |

(continued)

| No. | Name of signal peptides | Amino acid sequence |
|---|---|---|
| 253 | Sperm acrosome-associated protein 5 | MKAWGTVVVTLATLMVVTVDA |
| 254 | Spexin | MKGLRSLAATTLALFLVFVFLGNSSC |
| 255 | Spondin-1 | MRLSPAPLKLSRTPALLALALPLAAALA |
| 256 | Stanniocalcin-1 | MLQNSAVLLVLVISASA |
| 257 | Stereocilin | MALSLWPLLLLLLLLLLLSFAV |
| 258 | Stervl-sulfatase | MPLRKMKTPFLLLFFLWEAES |
| 259 | Submaxillary gland androgen-regulated protein 3A | MKSLTWILGLWALAACFTPGES |
| 260 | Sulfatase 1 | MKYSCCALVLAVLGTELLGSLC |
| 261 | Syncollin | MSPLRPLLLALALASVPCAQG |
| 262 | Tachykinin-3 | MRIMLLFTAILAFSLA |
| 263 | Tenascin | MGAMTQLLAGVFLAFLALATEG |
| 264 | Thyroglobulin | MALVLEIFTLLASICWVSA |
| 265 | Thyroid peroxidase | MRALAVLSVTLVMACTEA |
| 266 | Transcobalamin-1 | MRQSHQLPLVGLLLFSFFPSQLC |
| 267 | Transforming growth factor beta-1 | MPPSGLRLLLLLLPLLWLLVLTPGRPAAG |
| 268 | Transthyretin | MASHRLLLLCLAGLVFVSEA |
| 269 | Trehalase | MPGRTWELCLLLLLGLGLGSQEA |
| 270 | TSHR protein | MRPADLLQLVLLLDLPRDLGG |
| 271 | Tsukushin | MPWPLLLLLAVSGAQT |
| 272 | Urocortin | MRQAGRAALLAALLLLVQLCPGSSQ |
| 273 | Urotensin-2B | MNKILSSTVCFGLLTLLSVLSFLQSVHG |
| 274 | Versican core protein | MFINIKSILWMCSTLIVTHA |
| 275 | Vitrin | MRTVVLTMKASVIEMFLVLLVTGVHS |
| 276 | Vitronectin | MAPLRPLLILALLAWVALA |
| 277 | WAP four-disulfide core domain protein 2 | MPACRLGPLAAALLLSLLLFGFTLVSGTGA |
| 278 | Zymogen granule membrane protein 16 | MLTVALLALLCASASG |
| 279 | Cystatin-S | MARPLCTLLL LMATLAGALA |
| 280 | A disintegrin and metalloproteinase...1 | MQRAVPEGFGRRKLGSDMGNAERAPGSRSFGPVPTL LLLAAALLAVSDA |
| 281 | Leucine-rich alpha-2-glycoprotein | MSSWSRQRPKSPGGIQPHVSRTLFLLLLLAASAWG |
| 282 | Bone morphogenetic protein 2 | MVAGTRCLLALLLPQVLLGGAAG |
| 283 | Bone morphogenetic protein 7 | MHVRSLRAAAPHSFVALWAPLFLLRSALA |
| 284 | Complement C1q tumor necrosis factor-related protein 8 | MAAPALLLLALLLPVGA |
| 285 | Carboxypeptidase E | MAGRGGSALLALCGALAACGWLLGA |
| 286 | Cholesteryl ester transfer protein | MLAATVLTLALLGNAHA |
| 287 | Complement factor I | MKLLHVFLLFLCFHLRFC |
| 288 | Collagen alpha-3(VI) chain | MRKHRHLPLVAVFCLFLSGFPTTHA |

(continued)

| No. | Name of signal peptides | Amino acid sequence |
|---|---|---|
| 289 | Cysteine-rich motor neuron 1 protein | MYLVAGDRGLAGCGHLLVSLLGLLLLLARSGTRA |
| 290 | U·PF0672 protein CXorf36 | MEPQLGPEAAALRPGWLALLLWVSALSCSFS |
| 291 | Cystatin-D | MMWPMHTPLLLLTALMVAVA |
| 292 | Beta-defensin 109 | MRLHLLLLILLLFSILLSPVRG |
| 293 | Dickkopf-related protein 2 | MAALMRSKDSSCCLLLLAAVLMVESSQIGSSRA |
| 294 | EMILIN-1 | MAPRTLWSCYLCCLLTAAAGA |
| 295 | Fibulin-2 | MVLLWEPAGAWLALGLALALGPSVAAA |
| 296 | FRAS 1-related extracellular matrix protein 3 | MAGASRHPTGTPRQLLVALACLLLSRPALQ |
| 297 | Gelsolin | MAPHRPAPALLCALSLALCALSLPVRA |
| 298 | Beta-galactosidase-1-like protein | MAPKKLSCLRSLLLPLSLTLLLPQADT |
| 299 | Plasma protease C1 inhibitor | MASRLTLLTLLLLLLAGDRASS |
| 300 | Interleukin-10 | MHSSALLCCLVLLTGVRA |
| 301 | Interleukin-12 subunit alpha | MCPARSLLLVATLVLLDHLSLA |
| 302 | Interleukin-31 | MASHSGPSTSVLFLFCCLGGWLA |
| 303 | Interleukin-1 receptor-like 1 | MGFWILAILTILMYSTAA |
| 304 | Early placenta insulin-like peptide | MASLFRSYLPAIWLLLSQLLRESLA |
| 305 | Kallikrein-14 | MSLRVLGSGTWPSAPKMF |
| 306 | Extracellular glycoprotein lacritin | MKFTTLLFLAAVAGALVYA |
| 307 | Left-right determination factor 1 | MQPLWLCWALWVLPLASPGAA |
| 308 | Lysyl oxidase homolog 3 | MRPVSVWQWSPWGLLLCLLCSSCLG |
| 309 | 1-O-acylceramide synthase | MGLHLRPYRVGLLPDGLLFLLLLLMLLADPALP |
| 310 | Matrilin-4 | MRGLLCWPVLLLLLQPWE |
| 311 | Matrix metalloproteinase-26 | MQLVILRVTIFLPWCFA |
| 312 | Neutrophil collagenase | MFSLKTLPFLLLLHVQISKA |
| 313 | Netrin-2-like protein | MPGWPWGLLLTAGTLFAALSPGPPAPA |
| 314 | Neuropeptide S | MISSVKLNLILVLSLSTMHVFWC |
| 315 | Proprotein convertase subtilisin/kexin type 5 | MGWGSRCCCPGRLDLLCVLALLGGCLLPVCRT |
| 316 | Parathyroid hormone-related protein | MQRRLVQQWSVAVFLLSYAVPSCG |
| 317 | Peroxidasin-like protein | MEPRLFCWTTLFLLAGWCLPGLP |
| 318 | Sulfhydryl oxidase 2 | MAAAGAAVARSPGIGAGPALR |
| 319 | Proactivator polypeptide | MYALFLLASLLGAALA |
| 320 | Pulmonary surfactant-associated protein A1 | MWLCPLALNLILMAASGAVC |
| 321 | Tectonic-1 | MRPRGLPPLLVVLLGCWASVSA |
| 322 | Protein TMEM155 | MASDLIRTILAVALISKLGTAVDA |
| 323 | Twisted gastrulation protein homolog 1 | MKLHYVAVLTLAILMFLTWLPESLS |

(continued)

| No. | Name of signal peptides | Amino acid sequence |
|---|---|---|
| 324 | Urokinase plasminogen activator surface receptor | MGHPPLLPLLLLLHTCVPASWG |
| 325 | VIP peptides | MDTRNKAQLLVLLTLLSVLF |
| 326 | Von Willebrand factor D and EGF domain-containing protein | MPGGACVLVIALMFLAWGEA |
| 327 | WAP four-disulfide core domain protein 6 | MGLSGLLPILVPFILLGDIQEPGHA |
| 328 | Protein Wnt-8a | MGNLFMLWAALGICCAAFS |
| 329 | Putative uncharacterized protein FLJ46089 | MRCVTRTRNWWRRAARMPRAGSSAWWVAVCKQVCT |
| 330 | HLA class I histocompatibility antigen, Cw-16alphachain | MRVMAPRTLILLLSGALAL TETWA |
| 331 | Acrosin-binding protein | MRKPAAGFLPSLLKVLLLPLAPAAA |
| 332 | Angiopoietin-related protein 1 | MKTFTWTLGVLFFLL VDTGHCRG |
| 333 | Apolipoprotein L1 | MEGAALLRVSVLCIWMSALFLGVGVRA |
| 334 | Brain-specific serine protease 4 | MVVSGAPPALGGGCLGTFTSLLLLASTAILNA |
| 335 | Cocaine- and amphetamine-regulated transcript protein | MESSRVRLLPLLGAALLLMLPLLGTRA |
| 336 | C-C motif chemokine 14 | MKISVAAIPFFLLITIALG |
| 337 | C-C motif chemokine 4 | MKLCVTVLSLLMLVAAFCSPALS |
| 338 | Complement component C6 | MARRSVLYFILLNALINKGQA |
| 339 | Cvtokine-like protein 1 | MRTPGPLPVLLLLAGAPAARP |
| 340 | Dickkopf-like protein 1 | MGEASPPAPARRHLLVLLLLLSTLVIPSAA |
| 341 | Deoxyribonuclease gamma | MSRELAPLLLLLLSIHSALA |
| 342 | Dentin sialophosphoprotein | MKIITYFCIWAVAWA |
| 343 | EGF-like domain-containing protein 7 | MRGSQEVLLMWLLVLAVGGTEHA |
| 344 | Endonuclease domain-containing 1 protein | MGTARWLALGSLFALAGLLEG |
| 345 | Ectonucleotide pyrophosphatase/ phosphodiesterase family member 2 | MARRSSFQSCQIISLFTFAVGVNICLG |
| 346 | Abhydrolase domain-containing protein FAM108B1 | MNNLSFSELCCLFCCPPCPG |
| 347 | EGF-containing fibulin-like extracellular matrix protein 2 | MLPCASCLPGSLLLWALLLLLLGSA |
| 348 | Complement factor H-related protein 5 | MLLLFSVILISWVSTVGG |
| 349 | Glypican-4 | MARFGLPALLCTLAVLSA |
| 350 | Ig heavy chain V-I region V35 | MDWTWRILFLVAAATGAHS |
| 351 | Interferon alpha-7 | MARSFSLLMVVLVLSYKSICSLG |
| 352 | Plasma kallikrein-like protein 4 | MVSAAGLSGDGKMRGVLLVLLGLLYSSTSC |
| 353 | Laminin subunit beta-2 | MELTSRERGRGQPLPWELRLGLLLSVLAATLA |
| 354 | Laminin subunit gamma-3 | MAAAALLLGLALLAPRAAG |

(continued)

| No. | Name of signal peptides | Amino acid sequence |
|---|---|---|
| 355 | Lipase member I | MRVYIFLCLMCWVRS |
| 356 | Lactadherin | MPRPRLLAALCGALLCAPSLLVA |
| 357 | Mannose receptor-like protein 1 | MLHPETSPGRGHLLAVLLALLGTTWA |
| 358 | Mucin-4 | MKGARWRRVPWVSLSCLCLCLLPHVVPG |
| 359 | NKG2D ligand 2 | MAAAAATKILLCLPLLLLLSGWSRA |
| 360 | Noelin-3 | MSPPLLKLGAVLSTMAMISNWMS |
| 361 | Epididymal secretory protein E1 | MRFLAATFLLLALSTAAQA |
| 362 | Otogelin | MGVLASALCWLLCVWLPWGEQA |
| 363 | PATE-like protein B | MRKMNTLLLVSLSFLYLKE |
| 364 | Perforin-1 | MAARLLLLGILLLLLPLPVPA |
| 365 | Peptidoglycan recognition protein I-alpha | MGTLPWLLAFFILGLQA |
| 366 | Prostatic acid phosphatase | MRAAPLLLARAASLSLGFLFLLFFWLDRSVLA |
| 367 | Basic salivary proline-rich protein 4 | MLLILLSVALLALSSA |
| 368 | Prolactin receptor | MKENVASATVFTLLLFLNTCLLNG |
| 369 | Serum amyloid A-4 protein | MRLFTGIVFCSLVMGVTS |
| 370 | Stromal cell-derived factor 2 | MAVVPLLLLGGLWSAVGA |
| 371 | Serpin E3 | MPPFLITLFLFHSCCLRANG |
| 372 | Tenascin-N | MSLQEMFRFPMGLLLGSVLLVASAPATL |
| 373 | Thioredoxin domain-containing protein 16 | MFSGFNVFRVGISFVIMCIFYMPTVNS |
| 374 | Vascular endothelial growth factor receptor 2 | MQSKVLLAVALWLCVETRA |
| 375 | von Willebrand factor A domain-containing protein 5B 1 | MPGLLNWITGAALPLTAS |
| 376 | WAP four-disulfide core domain protein 1 | MPLTGVGPGSCRRQIIRALCLLLLLLHAGSA |
| 377 | Putative uncharacterized protein UNQ5830/PRO19650/PRO19816 | MAILMLSLQLILLLIPSIS |
| 378 | Putative uncharacterized protein ENSP000003 80674 | MRPLLCALAGLALLCAVGALA |
| 379 | Putative uncharacterized protein LOC116349 | MPAVFMLASSSALQCGRG |
| 380 | Anterior gradient protein 2 homolog | MEKIPVSAFLLLVALSYTLA |
| 381 | Insulin-like growth factor-binding protein complex acid labile chain | MALRKGGLALALLLLSWVALGPRSLEG |
| 382 | Apolipoprotein C-III | MQPRVLLVVALLALLASARA |
| 383 | A disintegrin and metalloproteinase with thrombospondin motifs 16 | MKPRARGWRGLAALWMLLAQVAEQ |
| 384 | A disintegrin and metalloproteinase with thrombospondin motifs 8 | MLPAPAAPRWPPLLLLLLLLLPLARGA |
| 385 | Bone morphogenetic protein 1 | MPGVARLPLLLGLLLLPRPGRP |

(continued)

| No. | Name of signal peptides | Amino acid sequence |
|---|---|---|
| 386 | Bactericidal/permeability-increasing protein-like 1 | MAWASRLGLLLALLLPVVGA |
| 387 | Scavenger receptor cysteine-rich type 1 protein M160 | MMLPQNSWHIDFGRCCCHQNLFSAVVTCILLLNSCFLISS |
| 388 | Cathepsin D | MQPSSLLPLALCLLAAPA |
| 389 | Putative cat eye syndrome critical region protein 9 | MQSHLAPLACAAAAGRAGGSCQA |
| 390 | UPF0670 protein C8orf55 | MLGLLVALLALGLAVFA |
| 391 | C-C motif chemokine 3-like 1 | MQVSTAALAVLLCTMALCNQVLS |
| 392 | Collagen alpha-2(VIII) chain | MLGTLTPLSSLLLLLLVLVLGCGPRASS |
| 393 | Uncharacterized protein C18orf20 | MINLHRLCIIHVVATLLSTLLSLISVAIS |
| 394 | C-X-C motif chemokine 14 | MSLLPRRAPPVSMRLLAAALLL |
| 395 | Beta-defencin 106 | MRTFLFLFAVLFFLTPAKNA |
| 396 | Coagulation factor X | MGRPLHLVLLSASLAGLLLLGESLFIRREQA |
| 397 | INT-2 proto-oncogene protein | MGLIWLLLLSLLEPGWP |
| 398 | Leucine-rich repeat transmembrane protein FLRT2 | MGLQTTKWPSHGAFFLKSWLIISLGLYSQVSKLLA |
| 399 | Interleukin-18-binding protein | MTMRHNWTPDLSPLWVLLLCAHVVTLLV |
| 400 | Insulin-like growth factor-binding protein 7 | MERPSLRALLLGAAGLLLLLLLPLSSS |
| 401 | Serine protease inhibitor Kazal-type 7 | MKITGGLLLLCTVVYFCSS |
| 402 | Isthmin | MVRLAAELLLLLGLLLLTLHITVLRGSGA |
| 403 | Laminin subunit beta-4 | MQFQLTLFLHLGWLSYSKA |
| 404 | Endothelial lipase | MSNSVPLLCFWSLCYCFAAG |
| 405 | Secretoglobin-like protein LOC284402 | MRVTSATCALLLALICSVQLGDA |
| 406 | Lymphocyte antigen 86 | MKGFTATLFLWTLIFPSCSG |
| 407 | Netrin-1-like protein | MPVTFALLLLLGQATA |
| 408 | NHL repeat-containing protein 3 | MARFWVCVAGAGFFLAFLVLHSRFC |
| 409 | Protocadherin-15 | MFRQFYLWTCLASGIILGSLFEICLG |
| 410 | Pregnancy-specific beta-1-glycoprotein 5 | MGPLSAPPCTQHITWKGLLLTASLLNFWNLPITA |
| 411 | Putative peptide YY-2 | MATVLLALLVYLGALVDA |
| 412 | Ribonuclease K6 | MVLCFPLLLLLLVLWGPVCPLHA |
| 413 | Ribonuclease-like protein 12 | MIIMVIIFL VLLFWENEVND |
| 414 | Signal peptide, CUB and EGF-like domain-containing protein 2 | MGVAGRNRPGAAWAVLLLLLLLPPLLLLAGA |
| 415 | Serpin A9 | MASYLYGVLFAVGLCAPIYCVSP |
| 416 | Kunitz-tvpe protease inhibitor 4 | MKSAKLGFLLRFFIFCSLNTLLLG |
| 417 | Stanniocalcin-2 | MCAERLGQFMTLALVLATFDPARG |
| 418 | Thrombospondin type-1 domain-containing protein 4 | MVSHFMGSLSVLCFLLLLGFQFVCP |

(continued)

| No. | Name of signal peptides | Amino acid sequence |
|---|---|---|
| 419 | Uromodulin | MGQPSLTWMLMVVVASWFITTAAT |
| 420 | Protein Wnt-4 | MSPRSCLRSLRLLVFAVFSAAA |
| 421 | Protein Wnt-7b | MHRNFRKWIFYVFLCFGVLYVKLGALSSVVA |
| 422 | Peptidase S1 domain-containing protein LOC136242 | MKYVFYLGVLAGTFFFADS |
| 423 | Complement C1q subcomponent subunit C | MDVGPSSLPHLGLKLLLLLLLLPLRGQA |
| 424 | Uncharacterized protein C2orf66 | MIIDSSRIPSFTQLHSTMTRAPLLLLCVALVLLGHVNG |
| 425 | Cerebellin-4 | MGSGRRALSAVP A VLL VL TLPGLPVW A |
| 426 | Coiled-coil domain-containing protein 3 | MLRQLLLAALCLAGPPAPARA |
| 427 | Eotaxin | MKVSAALLWLLLIAAAFSPQGLA |
| 428 | C-C motif chemokine 23 | MKVSVAALSCLMLVTALGSQA |
| 429 | C-C motif chemokine 3 | MQVSTAALAVLLCTMALCNQFSA |
| 430 | Colipase-like protein C6orf127 | MMLPQWLLLLFLLFFFLFLLTRG |
| 431 | Retinoic acid early transcript 1G protein | MAAAASPAFLLRLPLLLLLSSWCRT |
| 432 | C-type lectin domain family 11 member A | MQAAWLLGALVVPQLLGFGHG |
| 433 | Collagen alpha-4(IV) chain | MWSLHIVLMRCSFRLTKSLATGPWSLILILFSVQYVYG |
| 434 | C3 and PZP-like alpha-2-macroglobulin domain-containing protein8 | MSGALLWPLLPLLLLLSARDGVRA |
| 435 | Cysteine-rich secretory protein 2 | MALLPVLFLVTVLLPSLPAEG |
| 436 | Chymotrypsinopen B | MASLWLLSCFSLVGAAFG |
| 437 | Cvstatin-SA | MAWPLCTLLLLLLATQAVALA |
| 438 | Coagulation factor VII | MVSQALRLLCLLLGLQGCLA |
| 439 | Fibroblast growth factor-binding protein 1 | MKICSLTLLSFLLLAAQVLLVEG |
| 440 | Growth/differentiation factor 5 | MRLPKLLTFLLWYLAWLDLEFICTVLG |
| 441 | Glican-2 | MSATRPLLLLLLLPLCPGPGPGPG |
| 442 | Hemicentin-1 | MISWEVVHTVFLFALLYSSLA |
| 443 | Hvaluronan and proteoglycan link protein 4 | MVCARAALGPGALWAAAWGVLLLTAPAGA |
| 444 | Interferon beta | MTNKCLLQIALLLCFSTTALS |
| 445 | Gastric intrinsic factor | MAWFALYLLSLLWATAGT |
| 446 | Immunoglobulin lambda-like polypeptide 1 | MRPGTGQGGLEAPGEPGPNLRQRWPLLLLGLAVVTHG |
| 447 | Interleukin-21 | MERIVICLMVIFLGTL VHKSSS |
| 448 | Interleukin-29 | MAAAWTVVLVTLVLGLAVA |
| 449 | Interleukin-4 receptor alpha chain | MGWLCSGLLFPVSCLVLLQVASSGN |
| 450 | Kin of IRRE-like protein 3 | MKPFQLDLLFVCFFLFSQELG |
| 451 | Kallikrein -13 | MWPLALVIASLTLALS |
| 452 | Kallikrein-9 | MKLGLLCALLSLLAG |

(continued)

| No. | Name of signal peptides | Amino acid sequence |
|-----|------------------------|---------------------|
| 453 | Leukemia inhibitory factor | MKVLAAGVVPLLLVLHWKHGAG |
| 454 | Long palate, lung and nasal epithelium carcinoma-associated protein 4 | MWMAWCVAALSVVAVCGT |
| 455 | Stromelvsin-3 | MAPAAWLRSAAARALLPPMLLLLLQPPPLLA |
| 456 | Matrix metalloproteinase-19 | MNCQQLWLGFLLPMTVSG |
| 457 | Endomucin | MELLQVTILFLLPSICSS |
| 458 | Nucleobindin-2 | MRWRTILLQYCFLLITCLLTALEA |
| 459 | Polvmeric immunoglobulin receptor | MLLFVLTCLLAVFPAIST |
| 460 | Serine protease 27 | MRRPAAVPLLLLLCFGSQRAKA |
| 461 | Stromal cell-derived factor 1 | MNAKVVVVLVLVLTALCLSDG |
| 462 | Signal-regulatory protein delta | MPIPASPLHPPLPSLLLYLLLFLAGVTHV |
| 463 | Slit homolog 1 protein | MALTPGWGSSAGPVRPELWLLLWAAAWRLGASA |
| 464 | Sortilin-related receptor | MATRSSRRESRLPFLFTLVALLPPGALC |
| 465 | Short palate, lung and nasal epithelium carcinoma-associated protein 3 | MMCPL WRLLIFLGLLALPLAP |
| 466 | Tryptase beta-2 | MLNLLLLALPVLASRAYA |
| 467 | Protein Wnt-11 | MRARPQVCEALLFALALQTGVCYG |
| 468 | Fractalkine | MAPISLSWLLRLATFCHLTVLLAG |
| 469 | Ig-like domain-containing protein ENSP00000270642 | MPPPAPGARLRLLAAAALAGLAVISRGLLS |
| 470 | Apolipoprotein M | MFHQIWAALLYFYGIILLNSIYQ |
| 471 | Artemin | MELGLGGLSTLSHCPWPRQQPALWPTLAALALLSSV AEA |
| 472 | Acid sphingomyelinase-like phosphodiesterase 3a | MALVRALVCCLLTAWHCRSGLG |
| 473 | Complement Clq tumomecrosis factor-related protein 1 | MGSRGQGLLLAYCLLLAFASGLVLS |
| 474 | C-C motif chemokine 28 | MQQRGLAIVALAVCAALHA |
| 475 | T-cell surface glycoprotein CD8 alpha chain | MALPVTALLLPLALLLHAARP |
| 476 | Cat eye syndrome critical region protein 1 | MLVDGPSERPALCFLLLAVAMSFFGSALS |
| 477 | Collagen alpha-1(II) chain | MIRLGAPQTLVLLTLLVAAVLRCQG |
| 478 | Collagen alpha-3(IX) chain | MAGPRACAPLLLLLLLGELLAAAGA |
| 479 | Collagen alpha-1 (XX) chain | MSSGDPAHLGLCLWLWLGATLG |
| 480 | Peptidase inhibitor R3HDML | MPLLPSTVGLAGLLFWAGOAVNAL |
| 481 | Crumbs homolog 1 | MALKNINYLLIFYLSFSLLIYIKNS |
| 482 | Beta-dpfpncin 116 | MSVMKPCLMTIAILMILAQKTPG |
| 483 | Beta-defensin 125 | MNILMLTFIICGLLTRVTKG |
| 484 | Protein FAM55D | MKISMINYKSLLALLFILASWIIFTVF |

(continued)

| No. | Name of signal peptides | Amino acid sequence |
|-----|------------------------|---------------------|
| 485 | Fibronectin type III domain-containing protein 7 | MAGGRETCLPLIGFILICLKMVASAK |
| 486 | Insulin growth factor-like family member 4 | MVPRISAAIFIFELLGSNS |
| 487 | Immunoglobulin superfamily member 21 | MRTAPSLRRCVCLLLAAILDLARG |
| 488 | Interleukin-17 A | MTPGKTSLVSLLLLLSLEAIVKA |
| 489 | Inhibin beta B chain | MDGLPGRALGAACLLLLAAGWLGPEAWG |
| 490 | Intelectin-2 | MLSMLRTMTRLCFLLFFSVATSGCSA |
| 491 | Leucine-rich repeat and calponin homology domain-containing protein 3 | MAAAGLVAVAAAAEYSGTVASG |
| 492 | Neurotrypsin | MTLARFVLALMLGALPEVVG |
| 493 | Neurturin | MQRWKAAALASVLCSSVLS |
| 494 | Protease-associated domain-containing protein of 21 kDa | MVPGAAGWCCLVLWLPACVAA |
| 495 | Proprotein convertase subtilisin/kexin type 9 | MGTVSSRRSWWPLPLLLLLLLLLLGPAGARA |
| 496 | Pigment epithelium-derived factor | MQALVLLLCIGALLGHSSC |
| 497 | Peptidase inhibitor 15 | MIAISAVSSALLFSLLCEA |
| 498 | Plasminogen-related protein A | MEHKEVVLLLLLFLKSGQG |
| 499 | Podocan | MAQSRVLLLLLLLPPQLHL |
| 500 | Putative testis-specific prion protein | MQHSLVFFFAVILHLSHL |
| 501 | Salivary acidic proline-rich phosphoprotein 1/2 | MLLILLSVALLAFSSA |
| 502 | PC3-secreted microprotein | MALRMLWAGQAKGILGGWGIICLVMSLLLQHPGVY |
| 503 | Secretogranin-2 | MAEAKTHWLGAALSLIPLIFLISGAEA |
| 504 | Secreted and transmembrane protein 1 | MQTCPLAFPGHVSQALGTLLFLAASLSA |
| 505 | Semaphorin-3D | MNANKDERLKARSQDFHLFPALMMLSMTMLFLPVTG |
| 506 | Secretoglobin family 3A member 2 | MKLVTIFLLVTISLCSYSATA |
| 507 | Sialate O-acetylesterase | MVAPGLVLGLVLPLILWADRSAG |
| 508 | Sialic acid-binding Ig-like lectin 6 | MQGAQEASASEMLPLLLPLLWAGALA |
| 509 | SPARC | MRAWIFFLLCLAGRALA |
| 510 | Hepatocellular carcinoma-associated protein TD26 | MPVPALCLLWALAMVTRPASA |
| 511 | Trypsin-X3 | MKFILLWALLNLTVALA |
| 512 | Urocortin-2 | MTRCALLLLMVLMLGRVLV |
| 513 | Proto-oncogene protein Wnt-3 | MEPHLLGLLLGLLLGGTRVLA |
| 514 | Uncharacterized protein FLJ37543 | MLAPLFLCCLRNLFRKLIS |
| 515 | Putative uncharacterized protein ENSP00000381830 | MSFEYRHYKREAKICTCRGGW AHVLLCIGVSQGAC |
| 516 | Alpha-2-antiplasmin | MALLWGLLVLSWSCLQGPCSVFSPVSA |

(continued)

| No. | Name of signal peptides | Amino acid sequence |
|-----|------------------------|---------------------|
| 517 | Angiotensin-converting enzyme | MGAASGRRGPGLLLPLPLLLLLPPQPALA |
| 518 | Angiopoietin-1 | MTVFLSFAFLAAIL T |
| 519 | Apolipoprotein C-IV | MSLLRNRLQALPALCLCVLVLACIGA |
| 520 | Brain-derived neurotrophic factor | MTILFL TMVISYFGCMKA |
| 521 | Uncharacterized protein C1orf54 | MDVLFVAIFAVPLILG |
| 522 | Cerebellin-3 | MLGAKPHWLPGPLHSPGLPLVLVLLALGAGWA |
| 523 | Carboxypeptidase B2 | MKLCSLAVLVPIVLFCEQHVFA |
| 524 | C-C motif chemokine 22 | MDRLQTALLVVLVLLAVALQATEA |
| 525 | Colipase-like protein C6orf126 | MAAALALVAGVLSGAVLPLWS |
| 526 | Uncharacterized protein C7orf34 | MPPLAPQLCRAVFLVPILLLLQVKPLNG |
| 527 | Protein canopy homolog 4 | MGPVRLGILLFLFLAVHEAWA |
| 528 | Collagen alpha-2(VI) chain | MLQGTCSVLLLWGILGAIQA |
| 529 | Tenascin-X | MMPAQYALTSSLVLLVLLSTARA |
| 530 | Endothelin-3 | MEPGLWLLFGLTVTSA |
| 531 | Endothelial cell-specific molecule 1 | MKSVLLLTTLLVPAHLVAA |
| 532 | Protein eyes shut homolog | MTDKSIVILSLMVFHSSFING |
| 533 | Coagulation factor XI | MIFLYQVVHFILFTSVSG |
| 534 | Immunoglobulin alpha Fc receptor | MDPKQTTLLCLVLCLGQRIQA |
| 535 | Low affinity immunoglobulin gamma Fc region receptor III-B | MWQLLLPTALLLLVSA |
| 536 | Granzyme A | MRNSYRFLASSLSVVVSLLLIPEDVC |
| 537 | Hyaluronan and proteoglycan link protein 3 | MGLLLLVPLLLLPGSYG |
| 538 | Interleukin-28B | MTGDCMPVLVLMAAVLTVTGAVPVA |
| 539 | Pancreatic secretory trypsin inhibitor | MKVTGIFLLSALALLSLSGNTGA |
| 540 | Kallistatin | MHLIDYLLLLLVGLLALSHG |
| 541 | Kallikrein -12 | MGLSIFLLLCVLGLSQA |
| 542 | Keratinocyte differentiation-associated protein | MKIPVLPAVVLLSLLVLHSAQG |
| 543 | Putative lipocalin 1-like protein 1 | MKPLLLAISLSLIAALQA |
| 544 | Lysozyme C | MKALIVLGLVLLSVTVQG |
| 545 | MAM domain-containing protein 2 | MLLRGVLLALQALQLAGA |
| 546 | Matrilysin | MRLTVLCAVCLLPGSLA |
| 547 | Oxytocin-neurophysin 1 | MAGPSLACCLLGLLALTSA |
| 548 | NPIP-like protein LOC440350 | MRLRFWLLIWLLLGFISH |
| 549 | Phospholipase A2, membrane associated | MKTLLLLAVIMIFGLLQAHG |
| 550 | Group XIIB secretory phospholipase A2-like protein | MKLASGFLVLWLSLGGGLA |
| 551 | Basement membrane-specific heparansulfateproteo glycancoreprotein | MGWRAAGALLLALLLHGRLLA |

(continued)

| No. | Name of signal peptides | Amino acid sequence |
|---|---|---|
| 552 | Plasma glutamate carboxypeptidase | MKFLIFAFFGGVHLLSLCSG |
| 553 | Melanocyte protein Pmel 17 | MDLVLKRCLLHLAVIGALLAVGAT |
| 554 | Vitamin K-dependent protein Z | MAGCVPLLQGLVLVLALHRVEPS |
| 555 | Sulfhydryl oxidase 1 | MRRCNSGSGPPPST T T T T T WLLAVPGANA |
| 556 | Regenerating islet-derived protein 3 gamma | MLPPMALPSVSWMLLSCLILLCQVQG |
| 557 | Secretoglobin family 1C member 1 | MKGSRALLLVALTLFCICRMATG |
| 558 | Sex hormone-binding globulin | MESRGPLATSRLLLLLLLLLLLRHTRQGWA |
| 559 | Tomoregulin-2 | MVLWESPRQCSSWTLCEGFCWLLLLPVMLLIVARPV KLAA |
| 560 | Trefoil factor 2 | MGRRDAQLLAALLVLGLCALAGS |
| 561 | Tachykinin-4 | MLPCLALLLLMELSVCTVA |
| 562 | Tumor necrosis factor receptor superfamily member 1A | MGLSTVPDLLLPLVLLELLVG |
| 563 | Tumor necrosis factor receptor superfamily member 5 | MVRLPLQCVLWGCLLTAVHP |
| 564 | Usherin | MNCPVLSLGSGFLFQVIEMLIFAYFASISLT |
| 565 | Urotensin-2 | MYKLASCCLLFIGFLNPLLS |
| 566 | V-set and transmembrane domain-containing protein 2A | MMGIFLVYVGFVFFSVLYVQQGL |
| 567 | Protein Wnt-6 | MLPPLPSRLGLLLLLLLLCPAHVGG |
| 568 | Putative uncharacterized protein FP248 | MGTGGSLLCGCSLVLSCLCPSAS |
| 569 | Putative serine protease LOC138652 | MGYCQGVSQVAVVLLMFPKEKEA |
| 570 | Zona pellucida sperm-binding protein 3 | MELSYRLFICLLLWGSTELCYP |
| 571 | Signal peptide, CUB and EGF-like domain-containin protein 3 | MGSGRVPGLCLLVLLVHARA |
| 572 | C-type natriuretic peptide | MHLSOLLACALLLTLLSLRPSEA |
| 573 | Antithrombin-III | MYSNVIGTVTSGKRKVYLLSLLLIGFWDCVTC |
| 574 | Acid sphingomyelinase-like phosphodiesterase 3b | MRLLAWLIFLANWGGARA |
| 575 | A disintegrin and metalloproteinase with thrombospondin motifs 13 | MHQRHPRARCPPLCV AGILACGFLLGCWG |
| 576 | B melanoma antigen 1 | MAARAVFLALSAQLLQA |
| 577 | Complement C1q subcomponent subunit A | MEGPRGWLVLCVLAISLASMVT |
| 578 | Complement C1q tumor necrosis factor-related protein 2 | MIPWVLLACALPCAA |
| 579 | Uncharacterized protein C1orf 187 | MAGPAIHTAPMLFLVLLLPLELSLA |
| 580 | Carbonic anhydrase-related protein 11 | MGAAARLSAPRALVLWAALGAAA |
| 581 | C-C motif chemokine 19 | MALLLALSLLVLWTSPAPTLS |
| 582 | Monocyte differentiation antigen CD 14 | MERASCLLLLPLVHVSA |

(continued)

| No. | Name of signal peptides | Amino acid sequence |
|---|---|---|
| 583 | CUB domain-containing protein 1 | MAGLNCGVSIALLGVLLLGAARLPRGAEA |
| 584 | UPF0669 protein C6orf120 | MAAPRGRAAPWTTALLLASQVLSPGSCA |
| 585 | Chitinase-3-like protein 1 | MGVKASQTGFVVLVLLQCCSA |
| 586 | Uncharacterized protein C18orf54 | MAKSKTKHRLCSQESSVSALLASCTLSGSNS |
| 587 | Cvstatin-8 | MPRCRWLSLILLTIPLALVAR |
| 588 | Secreted frizzled-related protein 3 | MVCGSPGGMLLLRAGLLALAALCLLRVPGARA |
| 589 | Beta-defencin 118 | MKLLLLALPMLVLLPQVIP |
| 590 | Beta-defensin 133 | MKIHVFLFVLFFFL VPIATRVKC |
| 591 | Beta-defensin 1 | MRTSYLLLFTLCLLLSEMASG |
| 592 | Dermokine | MKFQGPLACLLLALCLGSGEA |
| 593 | Protein FAM24A | MAKMFDLRTKIMIGIGSSLLVAAMVLLSVVFC |
| 594 | Fibrinogen-like protein 1 | MAKVFSFILVTTALTMGREISA |
| 595 | Folate receptor gamma | MAWQMMQLLLLALVTAAGSAQPR |
| 596 | Growth/differentiation factor 11 | MVLAAPLLLGFLLLALELRPRGEA |
| 597 | Granzyme K | MTKFSSFSLFFLIVGAYMTHVCFN |
| 598 | Major histocompatibility complex class I-related gene protein | MGELMAFLLPLIIVLMVKHSDS |
| 599 | Immunoglobulin superfamily member 1 | MTLDRPGEGATMLKTFT |
| 600 | Interleukin-1 receptor antagonist protein | MEICRGLRSHLITLLLFLFHSETIC |
| 601 | Serine protease inhibitor Kazal-type 5 | MKIATVSVLLPLALCLIQDAAS |
| 602 | Immunoglobulin superfamily containing leucine-richrepeatprotein | MQELHLLWWALLLGLAQA |
| 603 | Putative killer cell immunoglobulin-like receptorlikeproteinKIR3DP1 | MSLMVISMACVGFFLLQGAWT |
| 604 | Laminin subunit alpha-4 | MALSSAWRSVLPLWLLWSAACSRA |
| 605 | Leucine zipper protein 2 | MKFSPAHYLLPLLPALVLS |
| 606 | Sushi, nidogen and EGF-like domain-containing protein 1 | MRHGVAWALLVAAALGLGARGVRG |
| 607 | Protein-lysine 6-oxidase | MRFAWTVLLLGPLQLCALVHC |
| 608 | Neuroblastoma suppressor of tumorigenicity 1 | MMLRVLVGAVLPAML |
| 609 | Neurotensin/neuromedin N | MMAGMKIQLVCMLLLAFSSWSLC |
| 610 | Group 3 secretory phospholipase A2 | MGVQAGLFGMLGFLGVALG |
| 611 | Placenta growth factor | MPVMRLFPCFLQLLAGLA |
| 612 | Pregnancy-specific beta-1-glycoprotein 11 | MGPLSAPPCTEHIKWKGLLLTALLLNFWNLPTTA |
| 613 | Ribonuclease T2 | MRPAALRGALLGCLCLALLCLGGA |
| 614 | R-spondin-1 | MRLGLCVVALVLSWTHLTIS |
| 615 | Semaphorin-3E | MASAGHIITLLLWGYLLELWTGGHT |
| 616 | Kunitz-type protease inhibitor 1 | MAPARTMARARLAPAGIPAVALWLLCTLGLQGTQA |

(continued)

| No. | Name of signal peptides | Amino acid sequence |
|---|---|---|
| 617 | Transmembrane protein 25 | MALPPGPAALRHTLLLLPALLSSGWG |
| 618 | WAP four-disulfide core domain protein 10A | MAPQTLLPVLVLCVLLLQAQG |
| 619 | WAP four-disulfide core domain protein 3 | MMLSCLFLLKALLALGSLESWITA |
| 620 | Protein Wnt-10a | MGSAHPRPWLRLRPQPQPRPALWVLLFFLLLLAAA |
| 621 | Abhvdrolase domain-containing protein 9 | MPELVVTALLAPSRLSLKLLRAFMWSLVFSVALVA |
| 622 | ADAM 12 | MAARPLPVSPARALLLALAGALLAPCEA |
| 623 | A disintegrin and metalloproteinase with thrombospondin motifs 2 | MDPPAGAARRLLCPALLLLLLLLPPPLLP |
| 624 | Bone morphogenetic protein 3b | MAHVPARTSPGPGPQLLLLLLLPLFLLLLRDVAG |
| 625 | Complement C1q-like protein 4 | MVLLLLVAIPLLVHS |
| 626 | Complement C1r subcomponent-like protein | MPGPRVWGKYLWRSPHSKGCPGAMWWLLLWGVLQA |
| 627 | Uncharacterized protein Clorf56 | MVPAAGALLWVLLLNLGPRAAGA |
| 628 | Cathelicidin antimicrobial peptide | MKTQRDGHSLGRWSLVLLLLGLVMPLAIIA |
| 629 | Carboxypeptidase A2 | MAMRLILFFGALFGHI |
| 630 | C-C motif chemokine 4-like | MKLCVTVLSLLVLVAAFCSLALS |
| 631 | C-C motif chemokine 13 | MKVSAVLLCLLLMTAA |
| 632 | Neural cell adhesion molecule L1-like protein | MEPLLLGRGLIVYLMFLLLKFSKA |
| 633 | Complement C4-B | MRLLWGLIWASSFFTLSLQ |
| 634 | Collagen alpha-1(IX) chain | MKTCWKIPVFFFVCSFLEPWASA |
| 635 | Cartilage oligomeric matrix protein | MVPDTACVLLLTLAALGASG |
| 636 | Cysteine-rich secretory protein LCCL domain-containing 1 | MKCTAREWLRVTTVLFMARAIPA |
| 637 | UPF0556 protein C19orf10 | MAAPSGGWNGVGASLWAALLLGAVALRPAEA |
| 638 | Uncharacterized protein C19orf36 | MALLLCLVCLTAALA |
| 639 | Granulocyte-macrophage colony-stimulating factorreceptorsubunitalpha | MLLLVTSLLLCELPHPAFLLIP |
| 640 | Beta-defensin 103 | MRIHYLLFALLFLFLVPVPGHG |
| 641 | Beta-defencin 123 | MKLLLLTLTVLLLLSQLTPG |
| 642 | Beta-defencin 130 | MKLHSLISVLLLFVTLIPKGKT |
| 643 | Beta-defensin 2 | MRVLYLLFSFLFIFLMPLPGVFG |
| 644 | Ectonucleoside triphosphate diphosphohydrolase 5 | MATSWGTVFFMLVVSCVCSA |
| 645 | Protein FAM55A | MSSNTMLQKTLLILISFSVVT |
| 646 | Protein FAM3B | MRPLAGGLLKVVFVVFASLCAWYSGYLLA |
| 647 | Complement factor H-related protein 4 | MLLLINVILTLWVSCANGQ |
| 648 | Glucose-fructose oxidoreductase domain-containing protein 1 | MLPGVGVFGTSLTARVIIPLL |

(continued)

| No. | Name of signal peptides | Amino acid sequence |
|-----|------------------------|---------------------|
| 649 | Hemopexin | MARVLGAPVALGLWSLCWSLAIA |
| 650 | Ig heavy chain V-I region HG3 | MDWTWRVFCLLAVAPGAHS |
| 651 | Insulin-like growth factor-binding protein 4 | MLPLCLVAALLLAAGPGPSLG |
| 652 | Interleukin-17B | MDWPHNLLFLLTISIFLGLG |
| 653 | Interleukin-1 receptor type I | MKVLLRLICFIALLISS |
| 654 | Interleukin-5 | MRMLLHLSLLALGAAYVYA |
| 655 | Inhibin beta A chain | MPLLWLRGFLLASCWIIVRS |
| 656 | Epididymal-specific lipocalin-12 | MRLLCGLWLWLSLLKVLQA |
| 657 | Ly-6/neurotoxin-like protein 1 | MTPLL TLIL VVLMGLPLAQA |
| 658 | 72 kDa type IV collagenase | MEALMARGALTGPLRALCLLGCLLSHAAA |
| 659 | Serine protease MPN2 | MAAPASVMGPLGPSALGLLLLLLVVAPPRVAA |
| 660 | Neuronal pentraxin-like protein C16orf38 | MGCSWRKTLSFFLVFVPIYLHGASS |
| 661 | Neurexophilin-1 | MQAACWYVLFLLQPTVYLVTC |
| 662 | Group IIE secretory phospholipase A2 | MKSPHVLVFLCLLVALVTG |
| 663 | Platelet-derived growth factor D | MHRLIFVYTLICANFCSC |
| 664 | Platelet factor 4 | MSSAAGFCASRPGLLFLGLLLLPLVVAFASA |
| 665 | Periostin | MIPFLPMFSLLLLLIVNPINA |
| 666 | Basic salivary proline-rich protein 3 | MLLILLSVALLALSSA |
| 667 | Pro stasin | MAQKGVLGPGQLGAV AILL YLGLLRSGTG |
| 668 | Poliovirus receptor | MARAMAAAWPLLLVALLVLS |
| 669 | Retinol-binding protein 3 | MMREWVLLMSVLLCGLA |
| 670 | WAP, kazal, immunoglobulin, kunitz and NTRdomain-containing protein 1 | MPALRPLLPLLLLLRLTSG |
| 671 | Protein Wnt-5a | MKKSIGILSPGVALGMAGSAMSSKFFLVALAIFFS |
| 672 | Uncharacterized lipocalin UNQ2541/PR06093 | MMSFLLGAILTLLWAPTAQA |
| 673 | Zona pellucida sperm-binding protein 4 | MWLLRCVLLCVSLSLAVS |
| 674 | Alpha-amylase 1 | MKLFWLLFTIGFCWA |
| 675 | Complement C1q-like protein 2 | MALGLLIAVPLLLQAAPRGAA |
| 676 | Complement C1q tumor necrosis factor-related protein 9 | MRIWWLLLAIEICTGNINS |
| 677 | C-C motif chemokine 24 | MAGLMTIVTSLLFLGVCAHHIIPTGS |
| 678 | Uncharacterized protein C4orf26 | MARRHCFSYWLLVCWLVVTVAEG |
| 679 | CD 166 antigen | MESKGASSCRLLFCLLISATVFRPGLG |
| 680 | Uncharacterized protein C14orf93 | MSFSATILFSPPSGSEA |
| 681 | Collagen alpha-5(VI) chain | MKILLIIFVLIIWTETLA |
| 682 | Complement component C8 gamma chain | MLPPGTATLLTLLLAAGSLG |
| 683 | Collagen alpha-1(X) chain | MLPQIPFLLLVSLNL VHG |
| 684 | Putative uncharacterized protein C17orf69 | MHPWLGSALGFPKCRG |

(continued)

| No. | Name of signal peptides | Amino acid sequence |
|---|---|---|
| 685 | Cysteine-rich motor neuron 2 protein | MAGVGAAALSLLLHLGALALAAGAEG |
| 686 | Cvsteine-rich secretory protein 3 | MTLFPVLLFLVAGLLPSFPA |
| 687 | Macrophage colony-stimulating factor 1 | MTAPGAAGRCPPTTWLGSLLLLVCLLASRSIT |
| 688 | Beta-defensin 108B | MRIAVLLFAIFFFMSQVLPARG |
| 689 | Complement decay-accelerating factor | MTVARPSVPAALPLLGELPRLLLLVLLCLPAVWG |
| 690 | Estradiol 17-beta-dehydrogenase 11 | MKFLLDILLLLPLLIVCSL |
| 691 | EMILIN-2 | MWQPRRPWPRVPWRWALALLALVGAGLCHA |
| 692 | EGF-containing fibulin-like extracellular matrix protein 1 | MLKALFLTMLTLALVKS |
| 693 | Fibroblast growth factor 5 | MSLSFLLLLFFSHLILS |
| 694 | Fibroblast growth factor receptor 4 | MRLLLALLGVLLSVPGPPVLS |
| 695 | Glia-derived nexin | MNWHLPLFLLASVTLPSIC |
| 696 | Gamma-glutamyl hydrolase | MASPGCLLCVLGLLLCGAASLELS |
| 697 | Glutathione peroxidase 3 | MARLLQASCLLSLLLAGFVS |
| 698 | Histatin-1 | MKFFVFALVLALMISMISA |
| 699 | Insulin-like growth factor II | MGIPMGKSMLVLLTFLAFASCCIA |
| 700 | Interleukin-19 | MKLQCVSLWLLGTILILCSVDNHG |
| 701 | Interleukin-32 | MCFPKVLSDDMKKTKARMVMLLPTSAQGLG |
| 702 | Inter-alpha-trypsin inhibitor heavy chain H5-like protein | MSGWRYLICVSFLL TILLEL TYQ |
| 703 | Inter-alpha-trypsin inhibitor heavy chain H4 | MKPPRPVRTCSKVLVLLSLLAIHQTTTA |
| 704 | Uncharacterized protein KIAA0495 | MCLLSSSAASDLAATSLTA |
| 705 | Plasma kallikrein | MILFKQATYFISLFATVSC |
| 706 | Ig kappa chain V-I region HK101 | MDMRVLAQLLGLLLLCFPGARC |
| 707 | Laminin subunit gamma-1 | MRGSHRAAPALRPRGRLWPVLAVLAAAAAGCA |
| 708 | Left-right determination factor 2 | MWPLWLCWALWVLPLAGPGAA |
| 709 | Lipase member H | MLRFYLFISLLCLSRSDA |
| 710 | Noelin | MSVPLLKIGVVLSTMA |
| 711 | Protocadherin alpha-10 | MVSRCSCLGVQCLLLSLLLLAAWEVGSG |
| 712 | Plexin-B1 | MPALGPALLQALWAGWVLT |
| 713 | Prolactin | MNIKGSPWKGSLLLLLVSNLLLCQSVAP |
| 714 | Poliovirus receptor-related protein 4 | MPLSLGAEMWGPEAWLLLLLLLASFTGRCPA |
| 715 | Secretoglobin family 1D member 4 | MRLSVCLLMVSLALCCYQAHA |
| 716 | Slit homolog 2 protein | MRGVGWQMLSLSLGLVLAILNKVAPQACPA |
| 717 | Trefoil factor 1 | MATMENKVICALVLVSMLALGTLA |
| 718 | Scavenger receptor cysteine-rich domain-containing protein LOC284297 | MRVLACLLAALVGIQA |
| 719 | Trefoil factor 3 | MAARALCMLGLVLALLSSSSA |
| 720 | Metalloproteinase inhibitor 1 | MAPFEPLASGILLLLWLIAPSRA |

(continued)

| No. | Name of signal peptides | Amino acid sequence |
|---|---|---|
| 721 | Vascular endothelial growth factor receptor 1 | MVSYWDTGVLLCALLSCLLLTGSSSG |
| 722 | WNT1-inducible-signaling pathway protein 1 | MRWFLPWTLAAVTAAAASTVLA |
| 723 | Uncharacterized protein ENSP00000244321 | MRLSRRPETFLLAFVLLCTLLGLGCP |
| 724 | Anthrax toxin receptor 2 | MVAERSPARSPGSWLFPGLWLLVLSGPGGLLRA |
| 725 | Apolipoprotein C-I | MRLFLSLPVLVVVLSIVLEGPAPAQG |
| 726 | Lymphotoxin-alpha | MTPPERLFLPRVCGTTLHLLLLGLLLVLLPGAQG |
| 727 | ADAMTS-like protein 2 | MDGRWQCSCWAWFLLVLAVVAG |
| 728 | ADAMTS-like protein 4 | MENWTGRPWLYLLLLLSLPQLCLD |
| 729 | Complement C1q-like protein 3 | MVLLLVILIPVLVSSAGTSA |
| 730 | Collagen alpha-6(IV) chain | MLINKLWLLLVTLCLTEELAAA |
| 731 | Collagen alpha-6(VI) chain | MMLLILFLVIICSHISVNQ |
| 732 | EGF-like domain-containing protein 6 | MPLPWSLALPLLLSWVAGGFG |
| 733 | EMILIN-3 | MGRRRLLVWLCAVAALLSGAQA |
| 734 | Ephrin type-A receptor 10 | METCAGPHPLRLFLCRMQLCLALLLGPWRPGTA |
| 735 | Fibroblast growth factor-binding protein 3 | MTPPKLRASLSPSLLLLLSGCLLAAA |
| 736 | Complement factor H-related protein 2 | MWLLVSVILISRISSVGG |
| 737 | Fibrinogen beta chain | MKRMVSWSFHKLKTMKHTLLLLLCVFLVKS |
| 738 | Fibronectin type III domain-containing protein 1 | MAPEAGATLRAPRRLSWAALLLLAALLPVASS |
| 739 | Growth/differentiation factor 6 | MDTPRVLLSAVFLISFLWDLPG |
| 740 | Hyaluronidase-1 | MAAHLLPICALFL TLLDMAQG |
| 741 | Interferon epsilon-1 | MIIKHFFGTVLVLLASTTIFS |
| 742 | Interleukin-1 receptor accessory protein | MTLLWCVVSLYFYGILQSDA |
| 743 | Ig kappa chain V-I region HK102 | MDMRVPAQLLGLLLLWLPGAKC |
| 744 | Ig kappa chain V-II region RPMI 6410 | MRLPAQLLGLLMLWVPGSSG |
| 745 | Epididymal-specific lipocalin-10 | MRQGLLVLALVLVLVLA |
| 746 | Leucine-rich repeat LGI family member 3 | MAGLRARGGPGPGLLALSALGFCLMLQVSA |
| 747 | Lysozyme-like protein 6 | MTKALLIYLVSSFLALNQA |
| 748 | Mucin-like protein 1 | MKFLAVLVLLGVSIFLVSAQ |
| 749 | Group IID secretory phospholipase A2 | MELALLCGLVVMAGVIPIQG |
| 750 | Procollagen C-endopeptidase enhancer 2 | MRGANAWAPLCLLLAAATQLSRQ |
| 751 | Basic salivary proline-rich protein 1 | MLLILLSVALLALSSA |
| 752 | Peroxidasin homolog | MAKRSRGPGRRCLLALVLFCAWGTLA |
| 753 | Ribonuclease 8 | MAPARAGCCPLLLLLLGLWVAEVLVRA |
| 754 | Semaphorin-3B | MGRAGAAA VIP GL ALL W AVGLGSA |
| 755 | Secreted Ly-6/uPAR-related protein 1 | MASRWAVQLLLVAAWSMGCGE |

(continued)

| No. | Name of signal peptides | Amino acid sequence |
|---|---|---|
| 756 | Tumor necrosis factor receptor superfamily member 6B | MRALEGPGLSLLCLVLALPALLPVPAVRG |
| 757 | Triggering receptor expressed on mveloid cells 1 | MRKTRLWGLLWMLFVSELRA |
| 758 | von Willebrand factor | MIPARFAGVLLALALILPGTLC |
| 759 | Protein Wnt-16 | MDRAALLGLARLCALWAALLVLFPYGAQG |
| 760 | Putative uncharacterized protein FLJ21075 | MGFHFCIWIIFLLPPPCKKC |
| 761 | Arylsulfatase F | MRPRRPLVFMSLVCALLNTCQA |
| 762 | ADAMTS-like protein 5 | MGKLRPGRVEWLASGHTERPHLFQNLLLFLWA |
| 763 | A disintegrin and metalloproteinase with thrombospondin motifs 17 | MCDGALLPPLVLPVLLLLVWGLDPGTA |
| 764 | A disintegrin and metalloproteinase with thrombospondin motifs 9 | MQFVSWATLLTLLVRDLA |
| 765 | Bactericidal/permeability-increasing protein-like 2 | MCTKTIPVLWGCFLLWNLYVSSS |
| 766 | Putative uncharacterized protein Clorfl91 | MWGFLVLKARWLVTPVRT |
| 767 | Carboxypeptidase B | MLALLVLVTVALASA |
| 768 | Uncharacterized protein C10orf25 | MVPGPPESVVRFFLWFCFLLPPTRKASC |
| 769 | UPF0454 protein C12orf49 | MVNLAAMVWRRLLRKRWVLALVFGLSLVYFLSS |
| 770 | Putative uncharacterized protein C14ort144 | MCRETAGYGWLLASTELLSLLEPLSP |
| 771 | Beta-Ala-His dipeptidase | MDPKLGRMAASLLAVLLLLLERGMFS |
| 772 | Collagen alpha-2(IV) chain | MGRDQRAVAGPALRRWLLLGTVTVG |
| 773 | Complement component C8 alpha chain | MFAVVFFILSLMTCQPGVTA |
| 774 | Collagen alpha-2(XI) chain | MERCSRCHRLLLLLPLVLGLSA |
| 775 | Complement component receptor 1-like protein | MAPPVRLERPFPSRRFPGLLLAALVLLLSSFS |
| 776 | Platelet basic protein | MSLRLDTTPSCNSARPLHALQVLLLLSLLLTALA |
| 777 | Follistatin-related protein 4 | MKPGGFWLHLTLLGASLPAALG |
| 778 | Protein G7c | MLPTEVPQSHPGPSALLLLQLLLPPTSA |
| 779 | Gastric inhibitory polypeptide | MVATKTFALLLLSLFLAVGLG |
| 780 | Gastrokine-1 | MLAYSSVHCFREDKMKFTIV |
| 781 | Glycosyltransferase 1 domain-containing protein 1 | MRLLFLAVLRPHTGNA |
| 782 | Interleukin-20 | MKASSLAFSLLSAAFYLLWTPSTG |
| 783 | Interleukin-27 subunit beta | MTPQLLLALVLWASCPPCSG |
| 784 | Kazal-type serine protease inhibitor domain-containing protein 1 | MLPPPRPAAALALPVLLLLLVVLTPPPTGA |
| 785 | Kallikrein -10 | MRAPHLHLSAASGARALAKLLPLLMAQLWA |
| 786 | Kallikrein-7 | MARSLLLPLQILLLSLALETAG |

(continued)

| No. | Name of signal peptides | Amino acid sequence |
|---|---|---|
| 787 | Ig kappa chain V region EV15 | MGSQVHLLSFLLLWISDTRA |
| 788 | Long palate, lung and nasal epithelium carcinoma-associated protein 1 | MAGPWTFTLLCGLLAATLIQA |
| 789 | Low-density lipoprotein receptor-related protein 8 | MGLPEPGPLRLLALLLLLLLLLLLQLQHLAAA |
| 790 | Cartilage matrix protein | MRVLSGTSLMLCSLLLLLQALC |
| 791 | Melanoma-derived growth regulatory protein | MARSLVCLGVIILLSAFSGPGVRG |
| 792 | Matrix metalloproteinase-16 | MILLTFSTGRRLDFVHHSGVFFLQTLLWILC |
| 793 | Multimerin-2 | MILSLLFSLGGPLGWGLLGAWA |
| 794 | Protein NOV homolog | MQSVQSTSFCLRKQCLCLTFLLLHLLGQVAA |
| 795 | Oocyte-secreted protein 1 homolog | MKTILGFKGLFYLHSLIWTCAGDWSA |
| 796 | Secretory phospholipase A2 receptor | MLLSPSLLLLLLLGAPRGCA |
| 797 | Proline-rich protein 1 | MKLTFFLGLLALISCFTPSES |
| 798 | Putative pregnancy-specific beta-1-glycoprotein 7 | MGPLSAPPCTQHITWKGLLLTASLLNFWNPPTTA |
| 799 | Poliovirus receptor-related protein 1 | MARMGLAGAAGRWWGLALGLTAFFLPGVHS |
| 800 | Glutaminyl-peptide cvclotransferase | MAGGRHRRVVGTLHLLLLVAALPWASRG |
| 801 | Trypsin-1 | MNPLLILTFVAAALA |
| 802 | Thyrotropin subunit beta | MTALFLMSMLFGLTCGQAMS |
| 803 | Putative testis serine protease 2 | MSSGGGSRGLLAWLLLLQPWPGQNWA |
| 804 | von Willebrand factor C domain-containing protein 2-like | MALHIHEACILLLVIPGLVTS |
| 805 | EGF-like repeat and discoidin I-like domain-containing protein 3 | MKRSVAVWLLVGLSLG |
| 806 | ADAM 28 | MLQGLLPVSLLLSVAVSA |
| 807 | B melanoma antigen 5 | MAAGAVFLALSAQLLQA |
| 808 | Probetacellulin | MDRAARCSGASSLPLLLALALGLVILHCVVA |
| 809 | Complement C1q tumor necrosis factor-related protein 4 | MLPLLLGLLGPAACWA |
| 810 | Carboxypeptidase N catalytic chain | MSDLLSVFLHLLLLFKLVAP |
| 811 | Coiled-coil domain-containing protein 80 | MTWRMGPRFTMLLAMWLVCGS |
| 812 | Protein CEI | MVAPAARVFLRAVRAALTSTVPDLLCLLARGSPRG |
| 813 | Bile salt-activated lipase | MGRLQLVVLGLTCCWAVASA |
| 814 | Putative cystatin-9-like 2 | MWSLPPSRALSCAPLLLLFSFQFLVTYA |
| 815 | Protein CYR61 | MSSRIARALALVVTLLHLTRLALS |
| 816 | Beta-defencin 135 | MATRSVLLALVVLNLLFYVPPGRS |
| 817 | Epidermal growth factor receptor | MRPSGTAGAALLALLAALCPASRA |
| 818 | Elastase-2A | MIRTLLLSTLVAGALS |
| 819 | Inactive carboxylesterase 4 | MWLPALVLATLAASAAWA |

(continued)

| No. | Name of signal peptides | Amino acid sequence |
|---|---|---|
| 820 | Fibrillin-2 | MGRRRRLCLQLYFLWLGCVVLWAQGTAG |
| 821 | Fibroleukin | MKLANWYWLSSAVLATYGFLVVA |
| 822 | Leucine-rich repeat transmembrane protein FLRT1 | MDLRDWLFLCYGLIAFL TEV |
| 823 | Follistatin-related protein 3 | MRPGAPGPLWPLPWGALAWAVGFVSS |
| 824 | Beta-galactosidase-1-like protein 2 | MTTWSLRRRPARTLGLLLLVVLGFLVLRRLDW |
| 825 | Gremlin-1 | MSRTAYTVGALLLLLGTLLPAAEG |
| 826 | Interferon alpha-1/13 | MASPFALLMVLVVLSCKSSCSLG |
| 827 | Indian hedgehog protein | MSPARLRPRLHFCLVLLLLLVVPAAWG |
| 828 | Interleukin-12 subunit beta | MCHQQLVISWFSLVFLASPLVA |
| 829 | Interleukin-27 subunit alpha | MGQTAGDLGWRLSLLLLPLLLVQAGVWG |
| 830 | Interleukin-3 | MSRLPVLLLLQLLVRPGLQ |
| 831 | Inhibin beta E chain | MRLPDVQLWLVLLWALVRA |
| 832 | Inter-alpha-trypsin inhibitor heavy chain H2 | MKRLTCFFICFFLSEVSG |
| 833 | Kallikrein - 5 | MATARPPWMWVLCALITALLLG |
| 834 | Ig kappa chain V-III region VG | MEAPAQLLFLLLLWLPDTTG |
| 835 | Apolipoprotein(a)-like protein 2 | MEHKEVVLLLLLFLKSAPTET |
| 836 | Complement-activating component of Ra-reactive factor | MRWLLLYYALCFSLSKASA |
| 837 | Microfibrillar-associated protein 5 | MSLLGPKVLLFLAAFIITSDW |
| 838 | Opticin | MRLLAFLSLLALVLQETGT |
| 839 | Pregnancy-specific beta-1-glycoprotein 4 | MGPLSAPPCTQRITWKGVLLTASLLNFWNPPTTA |
| 840 | Secretogranin-3 | MGFLGTGTWIL VL VLPIQA |
| 841 | Semaphorin-3G | MAPSAWAICWLLGGLLLHGGSS |
| 842 | Surfactant-associated protein G | MGSGLPLVLLLTLLGSSHG |
| 843 | SPARC-related modular calcium-binding protein 2 | MLLPQLCWLPLLAGLLPPVPA |
| 844 | Kunitz-type protease inhibitor 3 | MQLQASLSFLLILTLCLELRSELA |
| 845 | Protein TSPEAR | MSALLSLCFVLPLAAPGHG |
| 846 | Protein WFDC11 | MVSLMKLWIPMLMTFFCTVLLSVLG |
| 847 | Protein Wnt-10b | MLEEPRPRPPPSGLAGLLFLALCSRALS |
| 848 | Angiopoietin-related protein 4 | MSGAPTAGAALMLCAATAVLLSAQG |
| 849 | Apolipoprotein C-II | MGTRLLPALFLVLLVLGFEVQG |
| 850 | Protein ARMET | MRRMWATQGLAVALALSVLPG |
| 851 | A disintegrin and metalloproteinase with thrombospondin motifs 12 | MPCAQRSWLANLSVVAQLLNFGALC |
| 852 | Bone morphogenetic protein 10 | MGSLVLTLCALFCLAAYLVSG |
| 853 | Carboxypeptidase A5 | MQGTPGGGTRPGPSPVDRRTLLVFSFILAAALG |
| 854 | Carboxypeptidase Z | MPPPLPLLLLTVLVVAAA |

(continued)

| No. | Name of signal peptides | Amino acid sequence |
|---|---|---|
| 855 | C-C motif chemokine 18 | MKGLAAALLVLVCTMALCSC |
| 856 | Fibronectin type-III domain-containing protein C4orf31 | MVLLHWCLLWLLFPLSSRT |
| 857 | Uncharacterized protein C6orf 15 | MQGRVAGSCAPLGLLLVCLHLPGLFA |
| 858 | Complement factor B | MGSNLSPQLCLMPFILGLLSGGVTT |
| 859 | Calcium-activated chloride channel regulator family member 3 | MVFSLKVILFLSLLLSPVLK |
| 860 | Putative uncharacterized protein C13orf28 | MAVSQGDGTLCFVLLLCCWQETEL |
| 861 | Complement component C7 | MKVISLFILVGFIGEFQSFSSA |
| 862 | Cochlin | MSAAWIPALGLGVCLLLLPGPAGS |
| 863 | Chordin-like protein 1 | MGGMKYIFSLLFFLLLEGGKT |
| 864 | UPF0510 protein C19orf63 | MAAASAGATRLLLLLLMAVAAPSRARG |
| 865 | Granulocyte-macrophage colony-stimulating factor | MWLQSLLLLGTVACSIS |
| 866 | Cystatin-like 1 | MGIGCWRNPLLLLIALVLS |
| 867 | C-X-C motif chemokine 3 | MAHATLSAAPSNPRLLRVALLLLVAASRRAAG |
| 868 | 17-beta hydroxysteroid dehydrogenase 13 | MNIILEILLLLITIIYSYL |
| 869 | Elastase -1 | MLVLYGHS |
| 870 | EGF-like module-containing mucin-like hormonereceptor-like 3 | MQGPLLLPGLCFLLSLFGAVT |
| 871 | Ephrin type-A receptor 3 | MDCQLSILLLLSCSVLDSFG |
| 872 | Receptor tyrosine-protein kinase erbB-3 | MRANDALQVLGLLFSLARG |
| 873 | Fibulin-7 | MVPSSPRALFLLLLILACPFPRAS |
| 874 | Folate receptor beta | MVWKWMPLLLLLVCVA |
| 875 | Follistatin-related protein 1 | MWKRWLALALALVAVAWVRA |
| 876 | Growth hormone receptor | MDLWQLLLTLALAGSSDA |
| 877 | Glutathione peroxidase 7 | MVAATVAAAWLLLWAAACA |
| 878 | Hepatocyte growth factor-like protein | MGWLPLLLLLTQCLGVPG |
| 879 | Hyaluronidase-3 | MTTQLGPALVLGVALCLGCG |
| 880 | Interferon alpha-17 | MALSFSLLMAVLVLSYKSICSLG |
| 881 | Interferon omega-1 | MALLFPLLAAL VMTSYSPVGS |
| 882 | Interleukin-4 | MGLTSQLLPPLFFLLACAGNFVHG |
| 883 | Interleukin-9 | MLLAMVLTSALLLCSVAG |
| 884 | Serine protease inhibitor Kazal-type 5-like 3 | MAAFPHKIIFFLVCSTLTHVAFS |
| 885 | Laminin subunit alpha-3 | MAAAARPRGRALGPVLPPTPLLLLVLRVLPACGAT |
| 886 | Hepatic triacylglycerol lipase | MDTSPLCFSILLVLCIFIQSSA |
| 887 | Latent-transforming growth factor beta-binding protein 2 | MRPRTKARSPGRALRNPWRGFLPLTLALFVGAGHA |

(continued)

| No. | Name of signal peptides | Amino acid sequence |
|---|---|---|
| 888 | Matrix metalloproteinase-20 | MKVLPASGLAVFLIMALKFSTA |
| 889 | Mucin-7 | MKTLPLFVCICALSACFSFSEG |
| 890 | Matrix-remodeling-associated protein 5 | MPKRAHWGALSVVLILLWGHPRVALA |
| 891 | Ecto-ADP-ribosyltransferase 5 | MALAALMIALGSLGLHTWQAQA |
| 892 | Vasopressin-neurophysin 2-copeptin | MPDTMLPACFLGLLAFSSA |
| 893 | Nidogen-1 | MLASSSRIRAAWTRALLLPLLLAGPVGC |
| 894 | Neurexophilin-3 | MQLTRCCFVFLVQGSLYLVICG |
| 895 | Osteoclast associated immunoglobulin-like receptor | MALVLILQLLTLWPLCHT |
| 896 | Peptidoglycan recognition protein | MSRRSMLLAWALPSLLRLGAA |
| 897 | Plasminogen-related protein B | MEHKEVVLLLLLFLKSGQG |
| 898 | Protein Plunc | MFQTGGLIVFYGLLAQTMA |
| 899 | Podocan-like protein 1 | MAESGLAMWPSLLLLLLLPGPPPVAG |
| 900 | Placental protein 11 | MRACISLVLAVLCGLAWA |
| 901 | Receptor-type tyrosine-protein phosphatase zeta | MRILKRFLACIQLLCVCRLDWANG |
| 902 | Prorelaxin H2 | MPRLFFFHLLGVCLLLNQFSRAVA |
| 903 | Ribonuclease pancreatic | MALEKSLVRLLLLVLILLVLGWVQPSLG |
| 904 | Ribonuclease-like protein 9 | MMRTLITTHPLPLLLLPQQLLQLVQF |
| 905 | Ribonuclease-like protein 10 | MKLNLVQIFFMLLMLLLGLGMGLGLG |
| 906 | Secreted frizzled-related protein 1 | MGIGRSEGGRRGAALGVLLALGAALLAVGSA |
| 907 | Pulmonary surfactant-associated protein D | MLLFLLSALVLLTQPLGYLE |
| 908 | Spermatogenesis-associated protein 6 | MPKVKALQCALALEISSVTC |
| 909 | Tissue factor pathway inhibitor 2 | MDPARPLGLSILLLFLTEAALG |
| 910 | Thrombospondin-4 | MLAPRGAAVLLLHLVLQRWLAAGAQA |
| 911 | Protein Wnt-9b | MRPPPALALAGLCLLALPAAAA |
| 912 | Dickkopf-related protein 3 | MQRLGATLLCLLLAAAVPTAP |
| 913 | Alpha-1-acid glycoprotein 2 | MALSWVLTVLSLLPLLEA |
| 914 | Amiloride-sensitive amine oxidase [copper-containing | MPALGWAVAAILMLQTAMA |
| 915 | ADM2 | MARIPTAALGCISLLCLQLPGSLS |
| 916 | Amelogenin, Y isoform | MGTWILFACLVGAAFA |
| 917 | Angiopoietin-related protein 5 | MMSPSQASLLFLNVCIFICGEAVQG |
| 918 | Apolipoprotein A-IV | MFLKAVVLTLALVAVAGARA |
| 919 | ADAMTS-like protein 3 | MASWTSPWWVLIGMVFMHSPLPQTTA |
| 920 | A disintegrin and metalloproteinase with thrombospondin motifs 14 | MAPLRALLSYLLPLHCALCAAA |
| 921 | A disintegrin and metalloproteinase with thrombospondin motifs 7 | MPGGPSPRSPAPLLRPLLLLLCALAPG |

(continued)

| No. | Name of signal peptides | Amino acid sequence |
|-----|------------------------|---------------------|
| 922 | B melanoma antigen 3 | MAAGVVFLALSAQLLQA |
| 923 | Bone morphogenetic protein 15 | MVLLSILRILFLCEL VLF |
| 924 | Bone morphogenetic protein 4 | MIPGNRMLMVVLLCQVLLG |
| 925 | Mus musculus Ig H-chain V-regiongene, exonandpartialcds | MGWSYIILFL VAT |
| 926 | Complement C1q tumor necrosis factor-related protein 3 | MLWRQLIYWQLLALFFLPFCLC |
| 927 | Carbonic anhydrase 6 | MRALVLLLSLFLLGGQA |
| 928 | C-C motif chemokine 1 | MQIITTALVCLLLAGMWPEDVDS |
| 929 | C-C motif chemokine 5 | MKVSAAALAVILIATALCAPASA |
| 930 | CUB domain-containing protein 2 | MLAEWGACLLLAVALLGPGLQA |
| 931 | Uncharacterized protein C11orf44 | MVLLCLFLASLAATPRA |
| 932 | Collagen alpha-1(IV) chain | MGPRLSVWLLLLPAALLLHFFHSRAAA |
| 933 | Collagen alpha-1(VIII) chain | MAVLPGPLQLLGVLLTISLSSIRLIQA |
| 934 | Collagen alpha-1(XIV) chain | MKIFQRKMRYWLLPPFLAIVYFCTIVQG |
| 935 | Collagen triple helix repeat-containing protein 1 | MRPQGPAASPQRLRGLLLLLLLQLPAPSSA |
| 936 | Beta-defencin 134 | MKPLLVVFVFLFLWDPVLA |
| 937 | Defensin-6 | MRTLTILTAVLLVALQAKA |
| 938 | Dentin matrix protein 4 | MKMMLVRRFRVLILMVFLVAC |
| 939 | Epididymal sperm-binding protein 1 | MTRWSSYLLGWTTFLLYSYESSGG |
| 940 | Coagulation factor VIII | MQIELSTCFFLCLLRFCFS |
| 941 | Fibroblast growth factor 8 | MGSPRSALSCLLLHLLVLCLQA |
| 942 | Growth arrest-specific protein 6 | MAPSLSPGPAALRRAPQLLLLLLAAECALA |
| 943 | Growth/differentiation factor 9 | MARPNKFLLWFCCFAWLCFPISLG |
| 944 | Histidine-rich glycoprotein | MKALIAALLLITLQYSCA |
| 945 | Interleukin-17 receptor E | MGSSRLAALLLPLLLIVIDLSDS |
| 946 | Insulin-like growth factor-binding protein 6 | MTPHPLLPPLLLLLALLLAASPGGALA |
| 947 | Interleukin-11 | MNCVCRLVLVVLSLWPDTAVA |
| 948 | Interleukin-25 | MRERPRLGEDSSLISLFLQVVAFLAMVMGTHT |
| 949 | Serine protease inhibitor Kazal-type 6 | MKLSGMFLLLSLALFCFLTGVFS |
| 950 | Uncharacterized protein KIAA0564 | MQSRLLLLGAPGGHG |
| 951 | Putative killer cell immunoglobulin-like receptor -likeproteinKIR3DX1 | MAPKLITVLCLGFCLN |
| 952 | Laminin subunit alpha-5 | MAKRLCAGSALCVRGPRGPAPLLLVGLALLGAARA |
| 953 | Laminin subunit beta-3 | MRPFFLLCFALPGLLHA |
| 954 | Leukocyte immunoglobulin-like receptor subfamily A member 2 | MTPILTVLICLGLSLGPRTHVQA |
| 955 | Matrix metalloproteinase-21 | MLAASIFRPTLLLCWLAAPWPTQP |

(continued)

| No. | Name of signal peptides | Amino acid sequence |
|-----|-------------------------|---------------------|
| 956 | Neurexophilin-4 | MRLLPEWFLLLFGPWLLRKAVSA |
| 957 | Polvserase-3 | MKWCWGPVLLIAGATVLMEGLQA |
| 958 | Pregnancy-specific beta-1-glycoprotein 2 | MGPLSAPPCTEHIKWKGLLVTASLLNFWNLPTTA |
| 959 | R-spondin-2 | MQFRLFSFALIILNCMDYSHC |
| 960 | Semaphorin-3F | MLVAGLLLWASLLTGAWP |
| 961 | Sperm-associated antigen 11B | MRQRLLPSVTSLLLVALLFPGSSQ |
| 962 | SPARC-related modular calcium-binding protein 1 | MLPARCARLLTPHLLLVLVQLSPARG |
| 963 | Thrombospondin type-1 domain-containing protein 1 | MKPMLKDFSNLLLVVLCDYVLGEA |
| 964 | V-set and transmembrane domain-containing protein 1 | MTAEFLSLLCLGLCLG |
| 965 | von Willebrand factor A domain-containing protein 3A | MKKYRKISIGCFAMATQTSHVFHG |
| 966 | Wnt inhibitory factor 1 | MARRSAFPAAALWLWSILLCLLALRAEA |
| 967 | Protein Z-dependent protease inhibitor | MKVVPSLLLSVLLAQVWL VPG |
| 968 | A disintegrin and metalloproteinase with thrombospondin motifs 19 | MRLTHICCCCLLYQLGFLSNG |
| 969 | Bone morphogenetic protein 6 | MPGLGRRAQWLCWWWGLLCS |
| 970 | Complement C1q subcomponent subunit B | MMMKIPWGSIPVLMLLLGLIDIS |
| 971 | C-C motif chemokine 7 | MKASAALLCLLLTAAAFSPQGLA |
| 972 | Putative uncharacterized protein C10orf31 | MFCLLHLCFYLANFASSIKRTHA |
| 973 | Uncharacterized protein C12orf73 | MPAGVPMSTYLKMFAASLLAMCAGA |
| 974 | Collagen alpha-3(IV) chain | MSARTAPRPQVLLLPLLLVLLAAAPAAS |
| 975 | Complement component C8 beta chain | MKNSRTWAWRAPVELFLLCAALGCLSLPGSRG |
| 976 | Collectin-11 | MRGNLALVGVLISLAFLSLLPSGHP |
| 977 | Uncharacterized protein C16orf89 | MASLGLLLLLLLTALPPLWS |
| 978 | Uncharacterized protein C17orf67 | MASFKLADSVWEDSLSKR |
| 979 | Protein CREG2 | MSVRRGRRPARPGTRLSWLLCCSALLSPAAG |
| 980 | C-X-C motif chemokine 9 | MKKSGVLFLLGIILLVLIGVQG |
| 981 | Beta-defensin 107 | MPGAMKIFVFILAALILLAQIF |
| 982 | Beta-defensin 128 | MKLFLVLIILLFEVLTDG |
| 983 | Ephrin-A4 | MRLLPLLRTVLWAAFLGSPLRGGSS |
| 984 | EMI domain-containing protein 1 | MGGPRAWALLCLGLLLPGGGAA |
| 985 | Protein FAM131A | MFLATLSFLLPFAHPFGTVSC |
| 986 | UPF0528 protein FAM172A | MSISLSSLILLPIWINMA |
| 987 | Fas apoptotic inhibitory molecule 3 | MDFWLWPLYFLPVSGAL |
| 988 | Low affinity immunoglobulin gamma Fc region receptor III-A | MWQLLLPTALLLLVSA |

(continued)

| No. | Name of signal peptides | Amino acid sequence |
|---|---|---|
| 989 | Fibroblast growth factor receptor 3 | MGAPACALALCVAVAIVAGASS |
| 990 | Growth/differentiation factor 3 | MLRFLPDLAFSFLLILALGQAVQF |
| 991 | Gastrokine-2 | MKILVAFLVVLTIFGIQSHG |
| 992 | Gastrin-releasing peptide | MRGRELPLVLLALVLCLAPRGRA |
| 993 | Inducible T-cell costimulator | MKSGLWYFFLFCLRIKVLTG |
| 994 | Interphotoreceptor matrix proteoglycan 1 | MYLETRRAIFVFWIFLQVQG |
| 995 | Serine protease inhibitor Kazal-type 9 | MRATAIVLLLALTLATMFS |
| 996 | Kallikrein-8 | MGRPRPRAAKTWMFLLLLGGAWAGHSRA |
| 997 | Leucine-rich glioma-inactivated protein 1 | MESERSKRMGNACIPLKRIAYFLCLLSALLLTEG |
| 998 | Lipase member N | MMWLLL TTTCLICGTLNA |
| 999 | Long palate, lung and nasal epithelium carcinoma-associated protein 3 | MQPVMLALWSLLLLWGLATP |
| 1000 | Lysozyme-like protein 2 | MKAAGILTLIGCLVTGAES |
| 1001 | Stromelysin-2 | MMHLAFLVLLCLPVCSA |
| 1002 | Matrix metalloproteinase-17 | MRRRAARGPGPPPPGPGLSRLPLPLLLLLALGTRGGC A |
| 1003 | Nodal homolog | MHAHCLPFLLHAWWALLQAGAATVAT |
| 1004 | Neuronal cell adhesion molecule | MQLKIMPKKKRLSAGRVPLILFLC |
| 1005 | Glycodelin | MLCLLLTLGVALVCGVPA |
| 1006 | Plasminogen activator inhibitor 1 | MQMSPALTCLVLGLALVFGEGSA |
| 1007 | Platelet-derived growth factor subunit B | MNRCWALFLSLCCYLRLVSA |
| 1008 | Paired immunoglobulin-like type 2 receptor alpha | MGRPLLLPLLPLLLPPAFL |
| 1009 | Proline-rich acidic protein 1 | MRRLLLVTSLVVVLLWEAGA |
| 1010 | R-spondin-4 | MRAPLCLLLLVAHAVDMLA |
| 1011 | Svndecan-4 | MAPARLFALLLFFVGGVA |
| 1012 | Secreted frizzled-related protein 4 | MFLSILVALCLWLHLALG |
| 1013 | Vitelline membrane outer layer protein 1 homolog | MERGAGAKLLPLLLLLRATGFTCA |
| 1014 | Brorin | MPSSTAMAVGALSSSLLVTCCLMVALC |
| 1015 | WAP four-disulfide core domain protein 12 | MGSSSFLVLMVSLVLVTLVAVEG |
| 1016 | Zinc-alpha-2-glycoprotein | MVRMVPVLLSLLLLLGP |
| 1017 | Angiopoietin-related protein 7 | MLKKPLSAVTWLCIFIVAFVSHPAWL |
| 1018 | Neutrophil defensin 1 | MRTLAILAAILLVALQAQA |
| 1019 | Transforming growth factor-beta-induced protein ig-h3 | MALFVRLLALALALALGPAATLA |
| 1020 | Complement Clq tumor necrosis factor-related protein 5 | MRPLLVLLLLGLAAG |
| 1021 | Beta-casein | MKVLILACLVALALA |
| 1022 | Coiled-coil domain-containin protein 134 | MDLLQFLAFLFVLLLSGMGATG |

(continued)

| No. | Name of signal peptides | Amino acid sequence |
|---|---|---|
| 1023 | C-C motif chemokine 20 | MCCTKSLLLAALMSVLLLHLCGESEA |
| 1024 | Tryptase beta-1 | MLNLLLLALPVLASRAYA |
| 1025 | Cerberus | MHLLLFQLLVLLPLGKT |
| 1026 | Complement factor D | MHSWERLAVLVLLGAAACAA |
| 1027 | Chitinase-3-like protein 2 | MGATTMDQKSLWAGVVVLLLLQGGSA |
| 1028 | Contactin-associated protein-like 3 | MASVAWAVLKVLLLLPTQTWSPVGA |
| 1029 | Complement C2 | MGPLMVLFCLLFLYPGLADS |
| 1030 | Collagen alpha-1 (XVI) chain | MWVSWAPGLWLLGLWATFGHG |
| 1031 | Probable carboxypeptidase X1 | MWGLLLALAAFAPAVGPALG |
| 1032 | Cystatin-9 | MSSPQRRKAMPWALSLLLMGFQLLVTYA |
| 1033 | C-X-C motif chemokine 6 | MSLPSSRAARVPGPSGSLCALLALLLLLLTPPGPLASA |
| 1034 | Beta-defensin 136 | MNLCLSALLFFLVILLPSGKG |
| 1035 | Ectonucleotide pyrophosphatase/ phosphodiesterase family member 5 | MTSKFLLVSFILAALSLSTTFSLQ |
| 1036 | Ephrin type-B receptor 6 | MATEGAAQLGNRVAGMVCSLWVLLLVSSVLA |
| 1037 | Fibrillin-3 | MTLEGLYLARGPLARLLLAWSALLCMAGGQG |
| 1038 | Leucine-rich repeat transmembrane protein FLRT3 | MISAAWSIFLIGTKIGLFLQVAPLSVMA |
| 1039 | Embryonic growth/differentiation factor 1 | MPPPQQGPCGHHLLLLLALLLPSLPLTRA |
| 1040 | Ig heavy chain V-II region ARH-77 | MDLLHKNMKHLWFFLLLVA |
| 1041 | Interferon kappa | MSTKPDMIQKCLWLEILMGIFIAGTLS |
| 1042 | IgA-inducing protein homolog | MCSYYHMKKRSVSGCNITIFAVMFSHLSAG |
| 1043 | Interleukin-7 | MFHVSFRYIFGLPPLILVLLPVASS |
| 1044 | Inhibin alpha chain | MVLHLLFLLLTPQGGHS |
| 1045 | Lipase member M | MLETLSRQWIVSHRMEMWLLILVAYMFQRNVNS |
| 1046 | Ig lambda chain V region 4A | MAWTPLFLFLLTCCPGSNSQ |
| 1047 | Mannan-binding lectin serine protease 2 | MRLLTLLGLLCGSVA |
| 1048 | Stromelysin-1 | MKSLPILLLLCVAVCSA |
| 1049 | Mesothelin | MALPTARPLLGSCGTPALGSLLFLLFSLGWVQPSRT |
| 1050 | Mucin-1 | MTPGTQSPFFLLLLLTVLTVVTG |
| 1051 | Protein kinase C-binding protein NELL2 | MESRVLLRTFCLIFGLGAVWG |
| 1052 | Neutrophil gelatinase-associated lipocalin | MPLGLLWLGLALLGALHAQA |
| 1053 | Orexigenic neuropeptide QRFP | MVRPYPLIYFLFLPLGAC |
| 1054 | Pregnancy-specific beta-1-glycoprotein 6 | MGPLSAPPCTQHITWKGLLLTASLLNFWNLPTTA |
| 1055 | Putative peptide YY-3 | MVSVCRPWPAVAIALLALLVCLG |
| 1056 | Regenerating islet-derived protein 3 alpha | MLPPMALPSVSWMLLSCLMLLSQVQG |
| 1057 | Serpin A11 | MGPAWLWLLGTGILASVHC |
| 1058 | Sushi, von Willebrand factor type A, EGF and pentraxindomain-containing protein 1 | MWPRLAFCCWGLALVSG |

(continued)

| No. | Name of signal peptides | Amino acid sequence |
|---|---|---|
| 1059 | TGF-beta receptor type III | MTSHYVIAIFALMSSCLATA |
| 1060 | Thyroxine-binding globulin | MSPFLYLVLLVLGLHATIHC |
| 1061 | Tubulointerstitial nephritis antigen-like | MWRCPLGLLLLLPLAGHLALG |
| 1062 | Tumor necrosis factor receptor superfamily member 18 | MAQHGAMGAFRALCGLALLCALSLG |
| 1063 | WAP four-disulfide core domain protein 5 | MRTQSLLLLGALLAVGSQLPAVFG |
| 1064 | Apolipoprotein A-V | MASMAAVLTWALALLSAFSATQA |
| 1065 | Apolipoprotein O | MFKVIQRSVGPASLSLLTFKVYAAP |
| 1066 | Chymotrypsinogen B2 | MAFLWLLSCWALLGTTFG |
| 1067 | Bone morphogenetic protein 5 | MHLTVFLLKGIVGFLWSCWVLVGYAKGGLG |
| 1068 | Biotinidase | MAHAHIQGGRRAKSRFVVCIM |
| 1069 | Calumenin | MDLRQFLMCLSLCTAFALS |
| 1070 | Collagen alpha-1(VI) chain | MRAARALLPLLLQACWTAA |
| 1071 | Carboxypeptidase-like protein X2 | MSRPGTATPALALVLLAVTLAGVGA |
| 1072 | Protein CREG1 | MAGLSRGSARALLAALLASTLLALLVSPARG |
| 1073 | Granulocyte colony-stimulating factor | MAGPATQSPMKLMALQLLLWHSALWTVQE |
| 1074 | C-X-C motif chemokine 16 | MGRDLRPGSRVLLLLLLLLLLVYLTQPGNG |
| 1075 | Beta-defensin 127 | MGLFMIIAILLFQKPTVTEQ |
| 1076 | Dehydrogenase/reductase SDR family member 4-like 2 | MARLLGLCAWARKSVRLASS |
| 1077 | Extracellular matrix protein 1 | MGTTARAALVLTYLAVASA |
| 1078 | Eosinophil cationic protein | MVPKLFTSQICLLLLLGLMGVEGSLHA |
| 1079 | Elastase-2B | MIRTLLLSTLVAGALS |
| 1080 | Protein FAM150A | MRPLKPGAPLPALFLLALALSPHGAHG |
| 1081 | Protein FAM5B | MRWQCGTRFRGLRPAVAPWTALLALGLPGWVLA |
| 1082 | Growth/differentiation factor 2 | MCPGALWVALPLLSLLAGSLQG |
| 1083 | Glycoprotein hormones alpha chain | MDYYRKYAAIFLVTLSVFLHVLHS |
| 1084 | Progonadoliberin-2 | MASSRRGLLLLLLLAHLGPSEA |
| 1085 | Glypican-6 | MPSWIGAVILPLLGLLLSLPAGA |
| 1086 | Proheparin-binding EGF-like growth factor | MKLLPSVVLKLFLAAVLSA |
| 1087 | Protein HEG homolog 1 | MASPRASRWPPPLLLLLLLPLLLLPPAAPG |
| 1088 | Hyaluronan and proteoglycan link protein 2 | MPGWLTLPTLCRFLLWAFTIFHKAQG |
| 1089 | Insulin-like growth factor-binding protein 2 | MLPRVGCPALPLPPPPLLPLLLLLLGASGGGGGARAEVL |
| 1090 | Insulin-like 3 | MDPRLPAWALVLLGPALVFA |
| 1091 | Serine protease inhibitor Kazal-type 8 | MKGICSDAILVLATSMWMAFA |
| 1092 | Phosphatidylcholine-sterol acyltransferase | MGPP GSPWQWVTLLL GLLLPP AAP |

(continued)

| No. | Name of signal peptides | Amino acid sequence |
|---|---|---|
| 1093 | Leukocyte immunoglobulin-like receptor subfamily B member 1 | MTPILTVLICLGLSLGPRTHVQA |
| 1094 | Lvsozvme-like protein 1 | MKAAGILTLIGCLVTGAES |
| 1095 | Netrin-1 | MMRAVWEALAALAAVACLVGAVRG |
| 1096 | Neuropilin-2 | MDMFPLTWVFLALYFSRHQV |
| 1097 | Neurotrophin-3 | MSILFYVIFLAYLRGIQG |
| 1098 | Otoraplin | MARILLLFLPGLVAVCA |
| 1099 | Protocadherin alpha-6 | MVFTPEDRLGKQCLLLPLLLLAAWKVGSG |
| 1100 | Gastricsin | MKWMVVVLVCLQLLEA |
| 1101 | Platelet factor 4 variant | MSSAARSRLTRATRQEMLFLALLLLPVVVA |
| 1102 | Proline-rich protein 4 | MLLVLLSVVLALSSA |
| 1103 | Pregnancy-specific beta-1-glycoprotein 8 | MGLLSAPPCTQRITWKGLLLTASLLNFWNPPTTA |
| 1104 | Pulmonary surfactant-associated protein B | MAESHLLQWLLLLLPTLCGPGTAA |
| 1105 | Inactive serine protease RAMP | MELGCWTQLGLTFLQLLLISS |
| 1106 | Ribonuclease-like protein 13 | MAPAVTRLLFLQLVLGPTLV |
| 1107 | R-spondin-3 | MHLRLISWLFIILNFMEYIGS |
| 1108 | Suprabasin | MHLARLVGSCSLLLLLGALSGWAAS |
| 1109 | Metalloproteinase inhibitor 3 | MTPWLGLIVLLGSWSLGDWGAEA |
| 1110 | Thymic stromal lymphopoietin | MFPFALLYVLSVSFRKIFILQLVGLVLT |
| 1111 | Testis-expressed sequence 264 protein | MSDLLLLGLIGGLTLLLLLTLLAFAGYSGLLA |
| 1112 | Retino schisin | MSRKIEGFLLLLLLFGYEATLGLS |
| 1113 | C-type lectin domain-containing protein UNQ5810/PRO19627 | MQRWTLWAAAFLTLHSAQA |
| 1114 | Uncharacterized aarF domain-containing protein kinase 1 | MARKALKLASWTSMALA |
| 1115 | Attractin | MVAAAAATEARLRRR |
| 1116 | A disintegrin and metalloproteinase with thrombospondin motifs 6 | MEILWKTLTWILSLIMASSEF |
| 1117 | BMP-binding endothelial regulator protein | MLWFSGVGALAERYCRRSPGITCCVLLLLNCSGVPM SLA |
| 1118 | Complement C1q tumor necrosis factor-related protein 9-like | MRIWWLLLAIEICTGNINS |
| 1119 | Carboxypeptidase A6 | MKCLGKRRGQAAAFLPLCWLFLKILQPGHS |
| 1120 | Collagen alpha-3(V) chain | MGNRRDLGQPRAGLCLLLAALQLLPGTQA |
| 1121 | Complement component C9 | MSACRSFAVAICILEISILTA |
| 1122 | B melanoma antigen 2 | MAAGVVFLALSAQLLQA |
| 1123 | Chordin-like protein 2 | MVPEVRVLSSLLGLALLWFPLDSHA |
| 1124 | C-X-C motif chemokine 5 | MSLLSSRAARVPGPSSSLCALLVLLLLTQPGPIAS |
| 1125 | C-X-C motif chemokine 11 | MSVKGMAIALAVILCATVVQG |
| 1126 | Desert hedgehog protein | MALLTNLLPLCCLALLALPAQS |

(continued)

| No. | Name of signal peptides | Amino acid sequence |
|---|---|---|
| 1127 | Protein FAM180A | MHWKMLLLLLLLYYNAEA |
| 1128 | Protein FAM19A4 | MRSPRMRVCAKSVLLSHWLFLAYVLMVCCKLMSAS |
| 1129 | Fibrillin-1 | MRRGRLLEIALGFTVLLASYTSHGADA |
| 1130 | Fibroblast growth factor 10 | MWKWILTHCASAFPHLPGCCCCCFLLLFLVSSVPVTC |
| 1131 | Glypican-5 | MDAQTWPVGFRCLLLLALVGSARS |
| 1132 | Hyaluronan-binding protein 2 | MFARMSDLHVLLLMALVGKTACG |
| 1133 | Probable serine protease HTRA4 | MIRPQLRTAGLGRCLLPGLLLLLVPVLWAGA |
| 1134 | Islet amyloid polypeptide | MGILKLQVFLIVLSVALNHLKA |
| 1135 | Interleukin-15 | MRISKPHLRSISIQCYLCLLLNSHFLTEA |
| 1136 | Interferon-alpha/beta receptor beta chain | MLLSQNAFIFRSLNLVLMVYISLVFG |
| 1137 | Inhibin beta C chain | MTSSLLLAFLLLAPTTVA |
| 1138 | Ig kappa chain V-IV region | MVLQTQVFISLLLWIS GAYG |
| 1139 | Epididvmal-specific lipocalin-8 | MPGAAEALPTVTVTLVAGAVPPASG |
| 1140 | Leucine-rich repeat-containing protein 17 | MRVVTIVILLCFCKAAEL |
| 1141 | Collagen alpha-5(IV) chain | MKLRGVSLAAGLFLLALSLWGQPAEA |
| 1142 | Lysozyme g-like protein 1 | MSALWLLLGLLALMDLSES |
| 1143 | Multiple epidermal growth factor-like domains 6 | MSFLEEARAAGRAVVLALVLLLLPAV |
| 1144 | Microfibril-associated glycoprotein 4 | MKALLALPLLLLLSTPPCAPQ |
| 1145 | Protein kinase C-binding protein NELL 1 | MPMDLILVVWFCVCTA |
| 1146 | Nidogen-2 | MEGDRVAGRPVLSSLPVLLLLPLLMLRAAA |
| 1147 | Platelet-activating factor acetylhydrolase | MVPPKLHVLFCLCGCLAVVYP |
| 1148 | Peptidase inhibitor 16 | MHGSCSFLMLLLPLLLLLVATTGPVGA |
| 1149 | Phospholipase A1 member A | MPPGPWESCFWVGGLILWLSVGSSG |
| 1150 | Vitamin K-dependent protein C | MWQLTSLLLFVATWGISGTPAPLDSVFSSSER |
| 1151 | Properdin | MITEGAQAPRLLLPPLLLLL TLP A TGS |
| 1152 | Pregnancy-specific beta-1-glycoprotein 1 | MGTLSAPPCTQRIKWKGLLLTASLLNFWNLPTTA |
| 1153 | Pregnancy zone protein | MRKDRLLHLCLVLLLILLSASDSNS |
| 1154 | Regulated endocrine-specific protein 18 | MQHPLWPGSSEGLQLLVCFLLLNSCPGGCS |
| 1155 | FMRFamide-related peptides | MEIISSKLFILLTLATSSLLTSNIFC |
| 1156 | Sperm-associated antigen 11A | MRQRLLPSVTSLLLVALLFPGSSQA |
| 1157 | Thrombospondin and AMOP domain-containing isthmin-like protein 1 | MRALRDRAGLLLCVLLLAALLEAALG |
| 1158 | Collagen alpha-2(I) chain | MLSFVDTRTLLLLAVTLCLATCQS |
| 1159 | Testican-2 | MRAPGCGRLVLPLLLLAAAALA |
| 1160 | T-cell immunomodulatory protein | MAAAGRLPSSWALFSPLLAGLALLGVGPVPARA |
| 1161 | Tumor necrosis factor receptor superfamily member 11B | MNNLLCCALVFLDISIKWTTQ |
| 1162 | Putative trvpsin-6 | MNPLLILAFVGAAVA |

(continued)

| No. | Name of signal peptides | Amino acid sequence |
|---|---|---|
| 1163 | Urocortin-3 | MLMPVHFLLLLLLLLGGPRTG |
| 1164 | Vasorin | MCSRVPLLLPLLLLLALGPGVQG |
| 1165 | Protein WFDC10B | MAPQTLLLVLVLCVLLLQAQG |
| 1166 | Protein Wnt-5b | MPSLLLLFTAALLSSWA |
| 1167 | Angiopoietin-related protein 6 | MGKPWLRALQLLLLLGASWA |
| 1168 | A disintegrin and metalloproteinase with thrombospondin motifs 15 | MLLLGILTLAFAGRTAG |
| 1169 | B melanoma antigen 4 | MAAGAVFLALSAQLLQA |
| 1170 | UPF0672 protein C3orf58 | MWRLVPPKLGRLSRSLKLAALGSLLVLMVLHSPSL |
| 1171 | C-C motif chemokine 2 | MKVSAALLCLLLIAATFIPQGLA |
| 1172 | T-cell surface glycoprotein CD8 beta chain | MRPRLWLLLAAQLTVLHGNSV |
| 1173 | Putative uncharacterized protein C11orf45 | MLTRLVLSAHLSSTTSPPWTHA |
| 1174 | Complement C5 | MGLLGILCFLIFLGKTWG |
| 1175 | Collagen alpha-1 (XI) chain | MEPWSSRWKTKRWLWDFTVTTLALTFLFQAREVRGA |
| 1176 | Collectin-10 | MNGFASLLRRNQFILLVLFLLQIQSLG |
| 1177 | Uncharacterized protein C17orf99 | MGLPGLFCLAVLAASSFSKA |
| 1178 | Cartilage-associated protein | MEPGRRGAAALLALLCVACALRAGRA |
| 1179 | Granulocyte colony-stimulating factor receptor | MARLGNCSLTWAALIILLLPGSLE |
| 1180 | Cystatin-9-like | MLGLPWKGGLSWALLLLLLGSQILLIYA |
| 1181 | C-X-C motif chemokine 13 | MKFISTSLLLMLLVSSLSPVQG |
| 1182 | Beta-defensin 126 | MKSLLFTLAVFMLLAQLVSG |
| 1183 | Protein FAM24B | MPVIAGGILAALLLLIVVVLC |
| 1184 | Fibroblast growth factor receptor 2 | MVSWGRFICL VVVTMATLSLA |
| 1185 | Fibrinogen alpha chain | MFSMRIVCLVLSVVGTAWT |
| 1186 | Growth/differentiation factor 15 | MPGQELRTVNGSQMLLVLLVLSWLPHGGA |
| 1187 | Pancreatic secretory granule membrane majorglycoproteinGP2 | MPHLMERMVGSGLLWLALVSCILTQASA |
| 1188 | Insulin-like growth factor-binding protein-like 1 | MPRLSLLLPLLLLLLLPLLPLLPPLSPS |
| 1189 | Interleukin-26 | MLVNFILRCGLLLVTLSLAIA |
| 1190 | Interleukin-6 receptor subunit beta | MLTLQTWLVQALFIFLTTESTG |
| 1191 | Interleukin-7 receptor subunit alpha | MTILGTTFGMVFSLLQVVSG |
| 1192 | Kallikrein -4 | MATAGNPWGWFLGYLILGVAGSLVSG |
| 1193 | Ig kappa chain V-I region Daudi | MDMRVPAQLLGLLLLWLRGARC |
| 1194 | Latherin | MLNVSGLFVLLCGLLVSSSA |
| 1195 | Epididvmal-specific lipocalin-9 | MALLLLSLGLSLIAA |
| 1196 | Leukocyte immunoglobulin-like receptor subfamily A member 3 | MTPILTVLICLGLSLDPRTHVQA |

(continued)

| No. | Name of signal peptides | Amino acid sequence |
|---|---|---|
| 1197 | Lutropin subunit beta | MEMLQGLLLLLLLSMGGAWA |
| 1198 | Latent-transforming growth factor beta-binding protein 4 | MPRPGTSGRRPLLLVLLLPLFAAATSA |
| 1199 | Lvsozvme g-like protein 2 | MLSSVVFWGLIALIGTSRG |
| 1200 | Meteorin | MGFPAAALLCALCCGLLAPAARA |

## 3. Design of SP-tags capable of distinguishing 1200 signal peptides

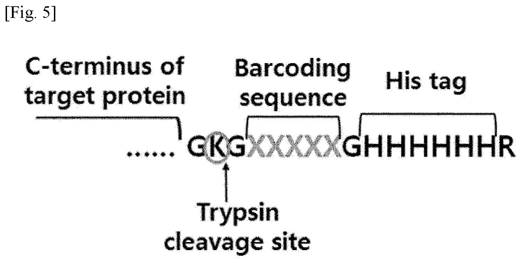

SP-tags were designed to consist essentially of barcoding sequences and histidine tags that produced 1200 or more diversities (Fig. 5). The barcoding sequences were designed to consist of 5 amino acids so as to correspond to 1200 signal peptides one by one. For the accuracy of subsequent experiments, 6×Histidine tags were attached to isolate and purify only the target protein from the cell culture media. Lysine (K) and arginine (R) were introduced at both ends for effective trypsin cleavage when LC-MS/MS was performed. Glycine residues as linker amino acids were inserted between the protein and the barcoding sequences and between the barcoding sequences and the His tags.

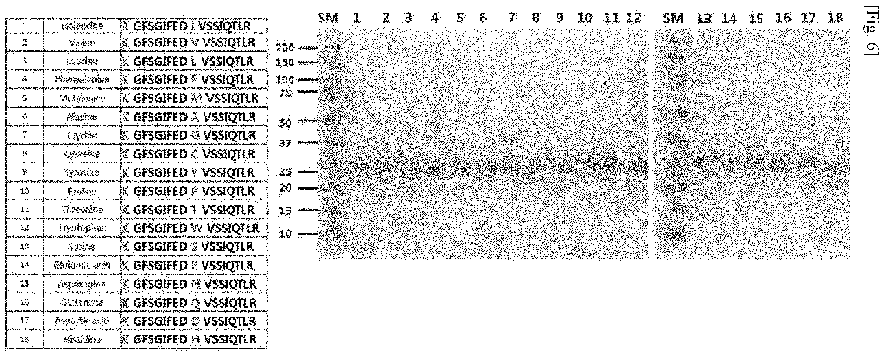

A total of 18 amino acids other than K and R as trypsin cleavage sites were selected as amino acids for the barcoding sequences. 18 SP-tagged proteins, each of which contains a different one of the 18 amino acids, were expressed and purified (Fig. 6).

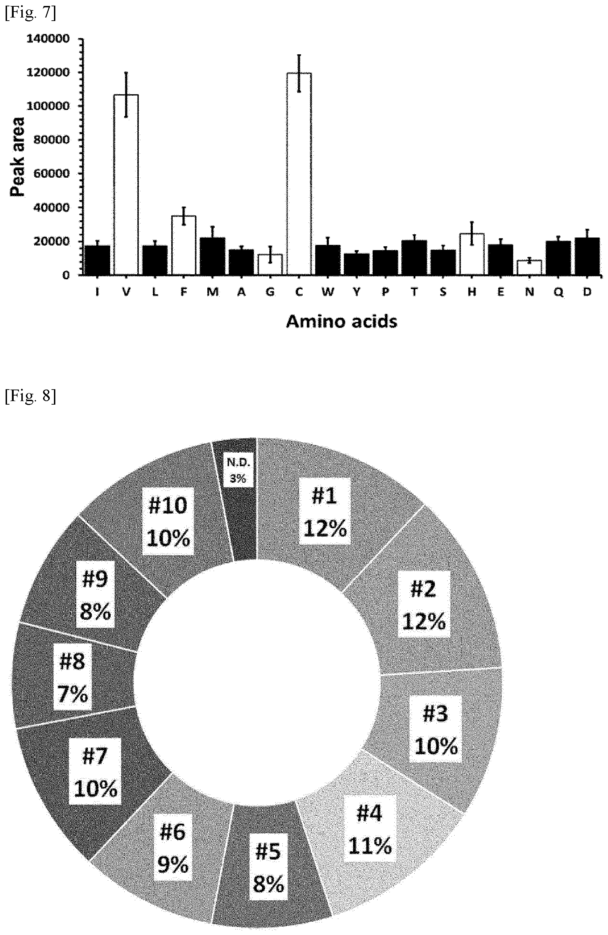

Equal amounts of the 18 proteins were determined by LC-MS/MS to select amino acids suitable for relative quantification (Fig. 7). As a result, 6 (V, F, G, C, H, and N) out of the 18 amino acids were found to be unsuitable for quantification due to their relatively high or low values. The other 12 amino acids (I, L, P, A, W, Y, P, T, S, E, Q, and D) did not affect quantification due to their similar peak area values. That is, the 12 amino acids were judged to be suitable for relative quantification because their peak area values were determined only by their amounts irrespective of their types. As a result, the 12 amino acids were primarily selected for barcoding sequence design.

## 4. Design of barcoding sequences consisting of five amino acids to construct library of 1200 SP-tags with different molecular weights

For effective discrimination on LC-MS/MS, 1200 SP-tags are required to have different molecular weights. Hence, barcoding sequences were designed such that all sequences differed by ≥2 in molecular weight. Among the 12 selected amino acids, I and L, E and Q, and D and N were identical to or different from each other (within ~±1) in molecular weight. Thus, I, Q, and N were excluded and only L, E, and D were used. Barcoding sequences consisting of five amino acids selected from L, P, A, W, Y, T, S, E, and D were designed to construct a library (Table 2).

[Table 2] Barcoding sequences consisting of five amino acids

| Number | Sequence | Number | Sequence | Number | Sequence | Number | Sequence |
|---|---|---|---|---|---|---|---|
| SP-tag #1 | SADAA | SP-tag #301 | WLELW | SP-tag #601 | EWPWP | SP-tag #901 | SLSYP |
| SP-tag #2 | TADAA | SP-tag #302 | EWELW | SP-tag #602 | YYPWP | SP-tag #902 | PLSYP |
| SP-tag #3 | ALDAA | SP-tag #303 | SAELY | SP-tag #603 | WYPWP | SP-tag #903 | PYSYP |
| SP-tag #4 | EADAA | SP-tag #304 | ALELY | SP-tag #604 | WWPWP | SP-tag #904 | EYSYP |
| SP-tag #5 | TLDAA | SP-tag #305 | DLELY | SP-tag #605 | AAPWT | SP-tag #905 | WLSYP |
| SP-tag #6 | LLDAA | SP-tag #306 | ELELY | SP-tag #606 | SAPWT | SP-tag #906 | YYSYP |
| SP-tag #7 | SYDAA | SP-tag #307 | WAELY | SP-tag #607 | TAPWT | SP-tag #907 | WYSYP |
| SP-tag #8 | WADAA | SP-tag #308 | EYELY | SP-tag #608 | EAPWT | SP-tag #908 | WWSYP |
| SP-tag #9 | SWDAA | SP-tag #309 | DWELY | SP-tag #609 | LLPWT | SP-tag #909 | AASYS |
| SP-tag #10 | EYDAA | SP-tag #310 | YYELY | SP-tag #610 | ELPWT | SP-tag #910 | SASYS |
| SP-tag #11 | WLDAA | SP-tag #311 | AAEWA | SP-tag #611 | SYPWT | SP-tag #911 | PASYS |
| SP-tag #12 | EWDAA | SP-tag #312 | SAEWA | SP-tag #612 | WLPWT | SP-tag #912 | DASYS |

(continued)

| Number | Sequence | Number | Sequence | Number | Sequence | Number | Sequence |
|---|---|---|---|---|---|---|---|
| SP-tag #13 | YYDAA | SP-tag #313 | PAEWA | SP-tag #613 | WYPWT | SP-tag #913 | EASYS |
| SP-tag #14 | WYDAA | SP-tag #314 | EAEWA | SP-tag #614 | AAPWW | SP-tag #914 | PLSYS |
| SP-tag #15 | WWDAA | SP-tag #315 | TLEWA | SP-tag #615 | SAPWW | SP-tag #915 | LLSYS |
| SP-tag #16 | SADAD | SP-tag #316 | YAEWA | SP-tag #616 | TAPWW | SP-tag #916 | YASYS |
| SP-tag #17 | DADAD | SP-tag #317 | SYEWA | SP-tag #617 | DAPWW | SP-tag #917 | ELSYS |
| SP-tag #18 | DLDAD | SP-tag #318 | WAEWA | SP-tag #618 | EAPWW | SP-tag #918 | SYSYS |
| SP-tag #19 | TYDAD | SP-tag #319 | SWEWA | SP-tag #619 | TLPWW | SP-tag #919 | WASYS |
| SP-tag #20 | TWDAD | SP-tag #320 | PWEWA | SP-tag #620 | LLPWW | SP-tag #920 | SWSYS |
| SP-tag #21 | WWDAD | SP-tag #321 | EYEWA | SP-tag #621 | YAPWW | SP-tag #921 | EYSYS |
| SP-tag #22 | TADAE | SP-tag #322 | WLEWA | SP-tag #622 | ELPWW | SP-tag #922 | WLSYS |
| SP-tag #23 | PLDAE | SP-tag #323 | EWEWA | SP-tag #623 | SYPWW | SP-tag #923 | EWSYS |
| SP-tag #24 | YADAE | SP-tag #324 | YYEWA | SP-tag #624 | PYPWW | SP-tag #924 | YYSYS |
| SP-tag #25 | SADAL | SP-tag #325 | WWEWA | SP-tag #625 | YLPWW | SP-tag #925 | WYSYS |
| SP-tag #26 | TADAL | SP-tag #326 | PAEWD | SP-tag #626 | PWPWW | SP-tag #926 | WWSYS |
| SP-tag #27 | WADAL | SP-tag #327 | DAEWD | SP-tag #627 | EYPWW | SP-tag #927 | TASYT |
| SP-tag #28 | TYDAL | SP-tag #328 | EAEWD | SP-tag #628 | WLPWW | SP-tag #928 | SLSYT |
| SP-tag #29 | EYDAL | SP-tag #329 | SYEWD | SP-tag #629 | EWPWW | SP-tag #929 | LLSYT |
| SP-tag #30 | DWDAL | SP-tag #330 | TYEWD | SP-tag #630 | AAPWY | SP-tag #930 | ELSYT |
| SP-tag #31 | WYDAL | SP-tag #331 | EYEWD | SP-tag #631 | ELPWY | SP-tag #931 | SYSYT |
| SP-tag #32 | SADAP | SP-tag #332 | DWEWD | SP-tag #632 | WAPWY | SP-tag #932 | WASYT |
| SP-tag #33 | DADAP | SP-tag #333 | AAEWE | SP-tag #633 | EYPWY | SP-tag #933 | TYSYT |
| SP-tag #34 | EADAP | SP-tag #334 | SAEWE | SP-tag #634 | EWPWY | SP-tag #934 | SWSYT |
| SP-tag #35 | WADAP | SP-tag #335 | PAEWE | SP-tag #635 | WWPYA | SP-tag #935 | EYSYT |
| SP-tag #36 | PWDAP | SP-tag #336 | ALEWE | SP-tag #636 | EAPYD | SP-tag #936 | EWSYT |
| SP-tag #37 | EYDAP | SP-tag #337 | EAEWE | SP-tag #637 | TLPYD | SP-tag #937 | YYSYT |
| SP-tag #38 | EWDAP | SP-tag #338 | TLEWE | SP-tag #638 | DYPYD | SP-tag #938 | WYSYT |
| SP-tag #39 | YYDAP | SP-tag #339 | LLEWE | SP-tag #639 | EYPYD | SP-tag #939 | WWSYT |
| SP-tag #40 | WYDAP | SP-tag #340 | YAEWE | SP-tag #640 | EWPYD | SP-tag #940 | AASYW |
| SP-tag #41 | WWDAP | SP-tag #341 | ELEWE | SP-tag #641 | SAPYS | SP-tag #941 | SASYW |
| SP-tag #42 | ELDAS | SP-tag #342 | SYEWE | SP-tag #642 | TAPYS | SP-tag #942 | TASYW |
| SP-tag #43 | SYDAT | SP-tag #343 | WAEWE | SP-tag #643 | LLPYS | SP-tag #943 | SYSYW |
| SP-tag #44 | AADAY | SP-tag #344 | TYEWE | SP-tag #644 | YAPYS | SP-tag #944 | WASYW |
| SP-tag #45 | SADAY | SP-tag #345 | SWEWE | SP-tag #645 | ELPYS | SP-tag #945 | TYSYW |
| SP-tag #46 | PADAY | SP-tag #346 | TWEWE | SP-tag #646 | SYPYS | SP-tag #946 | PWSYW |
| SP-tag #47 | DADAY | SP-tag #347 | WLEWE | SP-tag #647 | DYPYS | SP-tag #947 | YYSYW |
| SP-tag #48 | PLDAY | SP-tag #348 | EWEWE | SP-tag #648 | EWPYS | SP-tag #948 | ALTAA |
| SP-tag #49 | DLDAY | SP-tag #349 | YYEWE | SP-tag #649 | EAPYT | SP-tag #949 | DLTAA |
| SP-tag #50 | SYDAY | SP-tag #350 | WYEWE | SP-tag #650 | YAPYT | SP-tag #950 | SYTAA |

(continued)

| Number | Sequence | Number | Sequence | Number | Sequence | Number | Sequence |
|---|---|---|---|---|---|---|---|
| SP-tag #51 | EYDAY | SP-tag #351 | TAEWL | SP-tag #651 | SAPYW | SP-tag #951 | WATAA |
| SP-tag #52 | YYDAY | SP-tag #352 | EAEWL | SP-tag #652 | TAPYW | SP-tag #952 | TYTAA |
| SP-tag #53 | WYDAY | SP-tag #353 | TLEWL | SP-tag #653 | EAPYW | SP-tag #953 | TWTAA |
| SP-tag #54 | WWDAY | SP-tag #354 | YAEWL | SP-tag #654 | PLPYW | SP-tag #954 | DLTAD |
| SP-tag #55 | ALDLA | SP-tag #355 | SYEWL | SP-tag #655 | LLPYW | SP-tag #955 | ELTAE |
| SP-tag #56 | EADLA | SP-tag #356 | WAEWL | SP-tag #656 | YAPYW | SP-tag #956 | YLTAE |
| SP-tag #57 | TLDLA | SP-tag #357 | EYEWL | SP-tag #657 | SYPYW | SP-tag #957 | EWTAE |
| SP-tag #58 | DLDLA | SP-tag #358 | YYEWL | SP-tag #658 | SWPYW | SP-tag #958 | WATAL |
| SP-tag #59 | ELDLA | SP-tag #359 | WYEWL | SP-tag #659 | PWPYW | SP-tag #959 | EWTAL |
| SP-tag #60 | SYDLA | SP-tag #360 | AAEWP | SP-tag #660 | WLPYW | SP-tag #960 | SATAP |
| SP-tag #61 | SWDLA | SP-tag #361 | DAEWP | SP-tag #661 | YYPYW | SP-tag #961 | TATAP |
| SP-tag #62 | EWDLA | SP-tag #362 | SYEWP | SP-tag #662 | AAPYY | SP-tag #962 | DLTAP |
| SP-tag #63 | YYDLA | SP-tag #363 | DYEWP | SP-tag #663 | SAPYY | SP-tag #963 | ELTAP |
| SP-tag #64 | WWDLA | SP-tag #364 | WLEWP | SP-tag #664 | TAPYY | SP-tag #964 | SYTAP |
| SP-tag #65 | SADLL | SP-tag #365 | WYEWP | SP-tag #665 | ALPYY | SP-tag #965 | WATAP |
| SP-tag #66 | PADLL | SP-tag #366 | SAEWS | SP-tag #666 | EAPYY | SP-tag #966 | TYTAP |
| SP-tag #67 | SLDLL | SP-tag #367 | TAEWS | SP-tag #667 | PLPYY | SP-tag #967 | DYTAP |
| SP-tag #68 | TLDLL | SP-tag #368 | EAEWS | SP-tag #668 | YAPYY | SP-tag #968 | EYTAP |
| SP-tag #69 | LLDLL | SP-tag #369 | SYEWS | SP-tag #669 | ELPYY | SP-tag #969 | WLTAP |
| SP-tag #70 | ELDLL | SP-tag #370 | DWEWS | SP-tag #670 | WAPYY | SP-tag #970 | EWTAP |
| SP-tag #71 | SYDLL | SP-tag #371 | LLEWT | SP-tag #671 | TYPYY | SP-tag #971 | YYTAP |
| SP-tag #72 | WADLL | SP-tag #372 | YAEWT | SP-tag #672 | YLPYY | SP-tag #972 | WYTAP |
| SP-tag #73 | TYDLL | SP-tag #373 | WAEWT | SP-tag #673 | EYPYY | SP-tag #973 | WWTAP |
| SP-tag #74 | SWDLL | SP-tag #374 | DYEWT | SP-tag #674 | DWPYY | SP-tag #974 | DATAS |
| SP-tag #75 | PWDLL | SP-tag #375 | EYEWT | SP-tag #675 | EWPYY | SP-tag #975 | LLTAS |
| SP-tag #76 | EYDLL | SP-tag #376 | WLEWT | SP-tag #676 | YYPYY | SP-tag #976 | SYTAS |
| SP-tag #77 | DWDLL | SP-tag #377 | WYEWT | SP-tag #677 | WYPYY | SP-tag #977 | TYTAS |
| SP-tag #78 | EWDLL | SP-tag #378 | AAEWW | SP-tag #678 | ALSAA | SP-tag #978 | SWTAS |
| SP-tag #79 | YYDLL | SP-tag #379 | SAEWW | SP-tag #679 | EASAA | SP-tag #979 | TWTAS |
| SP-tag #80 | WWDLL | SP-tag #380 | TAEWW | SP-tag #680 | PLSAA | SP-tag #980 | EWTAS |
| SP-tag #81 | SADLP | SP-tag #381 | ALEWW | SP-tag #681 | DLSAA | SP-tag #981 | AATAT |
| SP-tag #82 | TADLP | SP-tag #382 | EAEWW | SP-tag #682 | ELSAA | SP-tag #982 | SATAT |
| SP-tag #83 | DADLP | SP-tag #383 | TLEWW | SP-tag #683 | PYSAA | SP-tag #983 | DATAT |
| SP-tag #84 | EADLP | SP-tag #384 | YAEWW | SP-tag #684 | SWSAA | SP-tag #984 | SLTAT |
| SP-tag #85 | TLDLP | SP-tag #385 | ELEWW | SP-tag #685 | PWSAA | SP-tag #985 | TLTAT |
| SP-tag #86 | DLDLP | SP-tag #386 | SYEWW | SP-tag #686 | EYSAA | SP-tag #986 | YATAT |
| SP-tag #87 | ELDLP | SP-tag #387 | PYEWW | SP-tag #687 | WLSAA | SP-tag #987 | SYTAT |
| SP-tag #88 | SYDLP | SP-tag #388 | YLEWW | SP-tag #688 | EWSAA | SP-tag #988 | SWTAT |

(continued)

| Number | Sequence | Number | Sequence | Number | Sequence | Number | Sequence |
|---|---|---|---|---|---|---|---|
| SP-tag #89 | PYDLP | SP-tag #389 | PWEWW | SP-tag #689 | YYSAA | SP-tag #989 | EYTAT |
| SP-tag #90 | SWDLP | SP-tag #390 | EYEWW | SP-tag #690 | WYSAA | SP-tag #990 | EWTAT |
| SP-tag #91 | EYDLP | SP-tag #391 | DWEWW | SP-tag #691 | WWSAA | SP-tag #991 | YYTAT |
| SP-tag #92 | WLDLP | SP-tag #392 | SAEWY | SP-tag #692 | TASAD | SP-tag #992 | WYTAT |
| SP-tag #93 | EWDLP | SP-tag #393 | TAEWY | SP-tag #693 | DLSAD | SP-tag #993 | WWTAT |
| SP-tag #94 | WWDLP | SP-tag #394 | DAEWY | SP-tag #694 | WASAD | SP-tag #994 | SATAW |
| SP-tag #95 | DWDLS | SP-tag #395 | SAEYA | SP-tag #695 | TYSAD | SP-tag #995 | TATAW |
| SP-tag #96 | DADLT | SP-tag #396 | PAEYA | SP-tag #696 | TWSAD | SP-tag #996 | ALTAW |
| SP-tag #97 | SYDLT | SP-tag #397 | EAEYA | SP-tag #697 | WLSAD | SP-tag #997 | YATAW |
| SP-tag #98 | DYDLT | SP-tag #398 | PLEYA | SP-tag #698 | TYSAE | SP-tag #998 | SYTAW |
| SP-tag #99 | SADLW | SP-tag #399 | YAEYA | SP-tag #699 | DYSAE | SP-tag #999 | WATAW |
| SP-tag #100 | LLDLW | SP-tag #400 | ELEYA | SP-tag #700 | TWSAE | SP-tag #1000 | SWTAW |
| SP-tag #101 | ELDLW | SP-tag #401 | PYEYA | SP-tag #701 | DWSAE | SP-tag #1001 | EYTAW |
| SP-tag #102 | TWDLW | SP-tag #402 | EYEYA | SP-tag #702 | WWSAE | SP-tag #1002 | YYTAW |
| SP-tag #103 | EWDLW | SP-tag #403 | EWEYA | SP-tag #703 | SWSAL | SP-tag #1003 | TATAY |
| SP-tag #104 | SADLY | SP-tag #404 | WWEYA | SP-tag #704 | WYSAL | SP-tag #1004 | DATAY |
| SP-tag #105 | TADLY | SP-tag #405 | SYEYD | SP-tag #705 | EASAP | SP-tag #1005 | TLTAY |
| SP-ta #106 | EADLY | SP-tag #406 | DYEYD | SP-tag #706 | YASAP | SP-tag #1006 | YATAY |
| SP-tag #107 | PLDLY | SP-tag #407 | DWEYD | SP-tag #707 | ELSAP | SP-tag #1007 | ELTAY |
| SP-tag #108 | ELDLY | SP-tag #408 | AAEYE | SP-tag #708 | WASAP | SP-tag #1008 | SYTAY |
| SP-tag #109 | WADLY | SP-tag #409 | SAEYE | SP-tag #709 | YLSAP | SP-tag #1009 | TYTAY |
| SP-tag #110 | PWDLY | SP-tag #410 | TAEYE | SP-tag #710 | EYSAP | SP-tag #1010 | YYTAY |
| SP-tag #111 | DWDLY | SP-tag #411 | DAEYE | SP-tag#711 | WLSAP | SP-tag #1011 | WYTAY |
| SP-tag #112 | WWDLY | SP-tag #412 | EAEYE | SP-tag #712 | EWSAP | SP-tap#1012 | AATLA |
| SP-tag #113 | AADWD | SP-tag #413 | PLEYE | SP-tag #713 | YYSAP | SP-tag #1013 | SATLA |
| SP-tag #114 | TADWD | SP-tag #414 | DLEYE | SP-tag #714 | WYSAP | SP-tag #1014 | TATLA |
| SP-tag #115 | DADWD | SP-tag #415 | ELEYE | SP-tag #715 | TASAS | SP-tag #1015 | EATLA |
| SP-tag #116 | EADWD | SP-tag #416 | SYEYE | SP-tag #716 | DASAS | SP-tag #1016 | TLTLA |
| SP-tag # 117 | TLDWD | SP-tag #417 | WAEYE | SP-tag #717 | SLSAS | SP-tag #1017 | YATLA |
| SP-tag # 118 | ELDWD | SP-tag #418 | TYEYE | SP-tag #718 | TLSAS | SP-tag #1018 | ELTLA |
| SP-tag #119 | SYDWD | SP-tag #419 | SWEYE | SP-tag #719 | YASAS | SP-tag #1019 | SYTLA |
| SP-tag #120 | YYDWD | SP-tag #420 | EYEYE | SP-tag #720 | ELSAS | SP-tag #1020 | TYTLA |
| SP-tag #121 | AADWE | SP-tag #421 | DWEYE | SP-tag #721 | PYSAS | SP-tag #1021 | DYTLA |
| SP-tag # 122 | SADWE | SP-tag #422 | EWEYE | SP-tag #722 | SWSAS | SP-tag #1022 | EYTLA |
| SP-tag #123 | DLDWE | SP-tag #423 | YYEYE | SP-tag #723 | PWSAS | SP-tag #1023 | DWTLA |
| SP-tag #124 | ELDWE | SP-tag #424 | WYEYE | SP-tag #724 | EYSAS | SP-tag #1024 | YYTLA |
| SP-tag #125 | WADWE | SP-tag #425 | WWEYE | SP-tag #725 | WLSAS | SP-tag #1025 | WYTLA |
| SP-ta #126 | DYDWE | SP-tag #426 | AAEYL | SP-tag #726 | EWSAS | SP-tag #1026 | WWTLA |

(continued)

| Number | Sequence | Number | Sequence | Number | Sequence | Number | Sequence |
|---|---|---|---|---|---|---|---|
| SP-tag #127 | EWDWE | SP-tag #427 | TAEYL | SP-tag #727 | YYSAS | SP-tag #1027 | EWTLD |
| SP-tag #128 | AADWL | SP-tag #428 | EAEYL | SP-tag #728 | WYSAS | SP-tag #1028 | AATLL |
| SP-ta #129 | TADWL | SP-tag #429 | TLEYL | SP-tag #729 | WWSAS | SP-tag #1029 | SATLL |
| SP-tag #130 | DADWL | SP-tag #430 | SYEYL | SP-tag #730 | SASAT | SP-tag #1030 | TATLL |
| SP-tag #131 | EADWL | SP-tag #431 | SWEYL | SP-tag #731 | EASAT | SP-tag #1031 | DATLL |
| SP-tag #132 | PLDWL | SP-tag #432 | EWEYL | SP-tag #732 | TLSAT | SP-tag #1032 | EATLL |
| SP-tag #133 | YADWL | SP-tag #433 | YAEYT | SP-tag #733 | YASAT | SP-tag #1033 | PLTLL |
| SP-tag #134 | SYDWL | SP-tag #434 | YLEYT | SP-tag #734 | ELSAT | SP-tag #1034 | DLTLL |
| SP-tag #135 | PYDWL | SP-tag #435 | AAEYW | SP-tag #735 | WASAT | SP-tag #1035 | ELTLL |
| SP-tag #136 | SWDWL | SP-tag #436 | EAEYW | SP-tag #736 | WLSAT | SP-tag #1036 | SYTLL |
| SP-tag #137 | AADWP | SP-tag #437 | PLEYW | SP-tag #737 | YYSAT | SP-tag #1037 | PYTLL |
| SP-tag #138 | TADWP | SP-tag #438 | YAEYW | SP-tag #738 | WYSAT | SP-tag #1038 | SWTLL |
| SP-tag #139 | YADWP | SP-tag #439 | ELEYW | SP-tag #739 | WWSAT | SP-tag #1039 | PWTLL |
| SP-tag #140 | SWDWP | SP-tag #440 | SYEYW | SP-tag #740 | AASAW | SP-tag #1040 | EYTLL |
| SP-tag #141 | PWDWP | SP-tag #441 | WAEYW | SP-tag #741 | SASAW | SP-tag #1041 | WLTLL |
| SP-tag #142 | DWDWP | SP-tag #442 | SWEYW | SP-tag #742 | PASAW | SP-tag #1042 | EWTLL |
| SP-tag #143 | EWDWP | SP-tag #443 | EYEYW | SP-tag #743 | ALSAW | SP-tag #1043 | YYTLL |
| SP-tag #144 | WYDWP | SP-tag #444 | DWEYW | SP-tag #744 | EASAW | SP-tag#1044 | WYTLL |
| SP-tag #145 | TYDWS | SP-tag #445 | EWEYW | SP-tag #745 | TLSAW | SP-tag #1045 | WWTLL |
| SP-tag #146 | DYDWS | SP-tag #446 | YYEYW | SP-tag #746 | YASAW | SP-tag #1046 | AATLP |
| SP-tag #147 | DADWT | SP-tag #447 | AAEYY | SP-tag #747 | WASAW | SP-tag #1047 | PATLP |
| SP-tag #148 | DLDWT | SP-tag #448 | SAEYY | SP-tag #748 | TYSAW | SP-tag #1048 | ALTLP |
| SP-tag #149 | AADWW | SP-tag #449 | PAEYY | SP-tag #749 | YLSAW | SP-tag #1049 | EATLP |
| SP-tag #150 | SADWW | SP-tag #450 | DAEYY | SP-tag #750 | EWSAW | SP-tag #1050 | TLTLP |
| SP-tag #151 | TADWW | SP-tag #451 | SLEYY | SP-tag #751 | WWSAW | SP-tag #1051 | ELTLP |
| SP-tag #152 | DADWW | SP-tag #452 | PLEYY | SP-tag #752 | AASAY | SP-tag #1052 | SYTLP |
| SP-tag #153 | EADWW | SP-tag #453 | DLEYY | SP-tag #753 | SASAY | SP-tag #1053 | PYTLP |
| SP-tag #154 | PLDWW | SP-tag #454 | ELEYY | SP-tag #754 | ALSAY | SP-tag #1054 | DYTLP |
| SP-tag #155 | YADWW | SP-tag #455 | SYEYY | SP-tag #755 | SLSAY | SP-tag #1055 | WLTLP |
| SP-tag #156 | ELDWW | SP-tag #456 | WAEYY | SP-tag #756 | PLSAY | SP-tag #1056 | YYTLP |
| SP-tag #157 | SYDWW | SP-tag #457 | YLEYY | SP-tag #757 | YASAY | SP-tag #1057 | WYTLP |
| SP-tag #158 | PYDWW | SP-tag #458 | TWEYY | SP-tag #758 | SYSAY | SP-tag #1058 | WWTLP |
| SP-tag #159 | SWDWW | SP-tag #459 | WLEYY | SP-tag #759 | TYSAY | SP-tag #1059 | EATLS |
| SP-ta #160 | PWDWW | SP-tag #460 | EWEYY | SP-tag #760 | EYSAY | SP-tag #1060 | SYTLS |
| SP-tag #161 | EYDWW | SP-tag #461 | WYEYY | SP-tag #761 | EWSAY | SP-tag #1061 | SWTLS |
| SP-tag #162 | DWDWW | SP-tag #462 | TAPAA | SP-tag #762 | YYSAY | SP-tag #1062 | DWTLS |
| SP-tag #163 | EWDWW | SP-tag #463 | ALPAA | SP-tag #763 | WYSAY | SP-tag #1063 | AATLT |
| SP-tag #164 | AADWY | SP-tag #464 | SLPAA | SP-tag #764 | WWSAY | SP-tag #1064 | SATLT |

(continued)

| Number | Sequence | Number | Sequence | Number | Sequence | Number | Sequence |
|---|---|---|---|---|---|---|---|
| SP-tag #165 | EADWY | SP-tag #465 | TLPAA | SP-tag #765 | AASLA | SP-tag #1065 | EATLT |
| SP-tag #166 | TYDWY | SP-tag #466 | DLPAA | SP-tag #766 | SASLA | SP-tag #1066 | TLTLT |
| SP-ta #167 | ELDYA | SP-tag #467 | ELPAA | SP-tag #767 | PASLA | SP-tag #1067 | ELTLT |
| SP-tag #168 | WADYA | SP-tag #468 | SYPAA | SP-tag #768 | ALSLA | SP-tag #1068 | SYTLT |
| SP-tag #169 | SWDYA | SP-tag #469 | WAPAA | SP-tag #769 | PLSLA | SP-tag #1069 | TYTLT |
| SP-tag #170 | EWDYA | SP-tag #470 | SWPAA | SP-tag #770 | DLSLA | SP-tag #1070 | SWTLT |
| SP-tag #171 | AADYD | SP-tag #471 | PWPAA | SP-tag #771 | ELSLA | SP-tag #1071 | TWTLT |
| SP-tag #172 | SADYD | SP-tag #472 | EYPAA | SP-tag #772 | SYSLA | SP-tag #1072 | YYTLT |
| SP-tag #173 | PADYD | SP-tag #473 | WLPAA | SP-tag #773 | WASLA | SP-tag #1073 | WYTLT |
| SP-tag #174 | DADYD | SP-tag #474 | EWPAA | SP-tag #774 | TWSLA | SP-tag #1074 | WWTLT |
| SP-tag #175 | EADYD | SP-tag #475 | YYPAA | SP-tag #775 | WLSLA | SP-tag #1075 | TLTLW |
| SP-tag #176 | ELDYD | SP-tag #476 | WYPAA | SP-tag #776 | EWSLA | SP-tag #1076 | ELTLW |
| SP-tag #177 | SYDYD | SP-tag #477 | WWPAA | SP-tag #777 | YYSLA | SP-tag #1077 | DWTLW |
| SP-tag #178 | WADYD | SP-tag #478 | TAPAD | SP-tag #778 | WWSLA | SP-tag #1078 | EWTLW |
| SP-ta #179 | TYDYD | SP-tag #479 | TLPAD | SP-tag #779 | DASLD | SP-tag #1079 | WWTLW |
| SP-tag #180 | PWDYD | SP-tag #480 | SYPAD | SP-tag #780 | DLSLD | SP-tag #1080 | EATLY |
| SP-tag #181 | EYDYD | SP-tag #481 | TYPAD | SP-tag #781 | WASLD | SP-tag #1081 | TLTLY |
| SP-tag #182 | EWDYD | SP-tag #482 | SWPAD | SP-tag #782 | EWSLD | SP-tag #1082 | ELTLY |
| SP-tag #183 | YYDYD | SP-tag #483 | DWPAD | SP-tag #783 | TASLE | SP-tag #1083 | WATLY |
| SP-tag #184 | AADYE | SP-tag #484 | AAPAE | SP-tag #784 | TLSLE | SP-tag #1084 | TYTLY |
| SP-tag #185 | SADYE | SP-tag #485 | PAPAE | SP-tag #785 | DLSLE | SP-tag #1085 | EYTLY |
| SP-tag #186 | TADYE | SP-tag #486 | ALPAE | SP-tag #786 | ELSLE | SP-tag #1086 | EWTLY |
| SP-tag #187 | DADYE | SP-tag #487 | LLPAE | SP-tag #787 | WASLE | SP-tag #1087 | YYTLY |
| SP-tag #188 | SLDYE | SP-tag #488 | ELPAE | SP-tag #788 | TYSLE | SP-tag #1088 | WYTLY |
| SP-tag #189 | TLDYE | SP-tag #489 | WAPAE | SP-tag #789 | EYSLE | SP-tag #1089 | EATWA |
| SP-tag #190 | DLDYE | SP-tag #490 | EWPAE | SP-tag #790 | YYSLE | SP-tag #1090 | TLTWA |
| SP-tag #191 | ELDYE | SP-tag #491 | YYPAE | SP-tag #791 | WYSLE | SP-tag #1091 | ELTWA |
| SP-tag #192 | WADYE | SP-tag #492 | AAPAL | SP-tag #792 | WWSLE | SP-tag #1092 | EWTWA |
| SP-tag #193 | EYDYE | SP-tag #493 | TAPAL | SP-tag #793 | TASLL | SP-tag #1093 | WYTWA |
| SP-tag #194 | EWDYE | SP-tag #494 | ALPAL | SP-tag #794 | SLSLL | SP-tag #1094 | EYTWD |
| SP-tag #195 | WYDYE | SP-tag #495 | EAPAL | SP-tag #795 | TLSLL | SP-tag #1095 | YYTWD |
| SP-tag #196 | WWDYE | SP-tag #496 | PLPAL | SP-tag #796 | LLSLL | SP-tag #1096 | SATWE |
| SP-tag #197 | DADYL | SP-tag #497 | LLPAL | SP-tag #797 | TYSLL | SP-tag #1097 | TYTWE |
| SP-tag #198 | YADYL | SP-tag #498 | YAPAL | SP-tag #798 | EYSLL | SP-tag #1098 | EWTWE |
| SP-tag #199 | TYDYL | SP-tag #499 | ELPAL | SP-tag #799 | EWSLL | SP-tag #1099 | WWTWE |
| SP-tag #200 | SWDYL | SP-tag #500 | SYPAL | SP-tag #800 | SASLP | SP-tag #1100 | AATWL |
| SP-tag #201 | SADYP | SP-tag #501 | PYPAL | SP-tag #801 | TLSLP | SP-tag #1101 | SATWL |
| SP-tag #202 | TADYP | SP-tag #502 | SWPAL | SP-tag #802 | YASLP | SP-tag #1102 | TATWL |

(continued)

| Number | Sequence | Number | Sequence | Number | Sequence | Number | Sequence |
|---|---|---|---|---|---|---|---|
| SP-tag #203 | DLDYP | SP-tag #503 | PWPAL | SP-tag #803 | SYSLP | SP-tag #1103 | ALTWL |
| SP-tag #204 | ELDYP | SP-tag #504 | EYPAL | SP-tag #804 | TYSLP | SP-tag #1104 | EATWL |
| SP-tag #205 | SYDYP | SP-tag #505 | DWPAL | SP-tag #805 | SWSLP | SP-tag #1105 | YATWL |
| SP-tag #206 | PYDYP | SP-tag #506 | EWPAL | SP-tag #806 | PWSLP | SP-tag #1106 | SYTWL |
| SP-tag #207 | DWDYP | SP-tag #507 | YYPAL | SP-tag #807 | EYSLP | SP-tag #1107 | WATWL |
| SP-tag #208 | YYDYP | SP-tag #508 | WYPAL | SP-tag #808 | DWSLP | SP-tag #1108 | DYTWL |
| SP-tag #209 | WWDYP | SP-tag #509 | WWPAL | SP-tag #809 | WYSLP | SP-tag #1109 | EYTWL |
| SP-tag #210 | ELDYS | SP-tag #510 | AAPAP | SP-tag #810 | WWSLP | SP-tag #1110 | DATWP |
| SP-tag #211 | DWDYS | SP-tag #511 | SAPAP | SP-tag #811 | AASLS | SP-tag #1111 | TLTWP |
| SP-tag #212 | SADYW | SP-tag #512 | TAPAP | SP-tag #812 | TASLS | SP-tag #1112 | YATWP |
| SP-tag #213 | PADYW | SP-tag #513 | DAPAP | SP-tag #813 | ALSLS | SP-tag #1113 | TYTWP |
| SP-tag #214 | DADYW | SP-tag #514 | EAPAP | SP-tag #814 | TLSLS | SP-tag #1114 | TWTWP |
| SP-tag #215 | PLDYW | SP-tag #515 | PLPAP | SP-tag #815 | YASLS | SP-tag #1115 | EWTWP |
| SP-tag #216 | DLDYW | SP-tag #516 | LLPAP | SP-tag #816 | ELSLS | SP-tag #1116 | AATWT |
| SP-tag #217 | ELDYW | SP-tag #517 | YAPAP | SP-tag #817 | SWSLS | SP-tag #1117 | TATWT |
| SP-tag #218 | SYDYW | SP-tag #518 | ELPAP | SP-tag #818 | PWSLS | SP-tag #1118 | DATWT |
| SP-tag #219 | WADYW | SP-tag #519 | SYPAP | SP-tag #819 | EWSLS | SP-tag #1119 | LLTWT |
| SP-tag #220 | DYDYW | SP-tag #520 | WAPAP | SP-tag #820 | YYSLS | SP-tag #1120 | YATWT |
| SP-tag #221 | TWDYW | SP-tag #521 | TYPAP | SP-tag #821 | WYSLS | SP-tag #1121 | ELTWT |
| SP-tag #222 | WLDYW | SP-tag #522 | YLPAP | SP-tag #822 | AASLT | SP-tag #1122 | SYTWT |
| SP-tag #223 | YYDYW | SP-tag #523 | TWPAP | SP-tag #823 | TASLT | SP-tag #1123 | WATWT |
| SP-tag #224 | AADYY | SP-tag #524 | WLPAP | SP-tag #824 | TLSLT | SP-tag #1124 | EYTWT |
| SP-tag #225 | SADYY | SP-tag #525 | EWPAP | SP-tag #825 | YASLT | SP-tag #1125 | YYTWT |
| SP-tag #226 | TADYY | SP-tag #526 | YYPAP | SP-tag #826 | WASLT | SP-tag #1126 | WWTWT |
| SP-tag #227 | DADYY | SP-tag #527 | WYPAP | SP-tag #827 | EYSLT | SP-tag #1127 | AATWW |
| SP-tag #228 | EADYY | SP-tag #528 | WWPAP | SP-tag #828 | YYSLT | SP-tag #1128 | SATWW |
| SP-tag #229 | PLDYY | SP-tag #529 | PAPAS | SP-tag #829 | WYSLT | SP-tag #1129 | TATWW |
| SP-tag #230 | YADYY | SP-tag #530 | SYPAS | SP-tag #830 | PLSLW | SP-tag #1130 | DATWW |
| SP-tag #231 | ELDYY | SP-tag #531 | PWPAS | SP-tag #831 | LLSLW | SP-tag #1131 | EATWW |
| SP-tag #232 | SYDYY | SP-tag #532 | EAPAT | SP-tag #832 | ELSLW | SP-tag #1132 | TLTWW |
| SP-tag #233 | WADYY | SP-tag #533 | TLPAT | SP-tag #833 | TYSLW | SP-tag #1133 | DLTWW |
| SP-tag #234 | TYDYY | SP-tag #534 | ELPAW | SP-tag #834 | DYSLW | SP-tag #1134 | ELTWW |
| SP-tag #235 | SWDYY | SP-tag #535 | PWPAW | SP-tag #835 | DWSLW | SP-tag #1135 | SYTWW |
| SP-tag #236 | PWDYY | SP-tag #536 | YYPAW | SP-tag #836 | DLSLY | SP-tag #1136 | WATWW |
| SP-tag #237 | EYDYY | SP-tag #537 | WWPAW | SP-tag #837 | SYSLY | SP-tag #1137 | TYTWW |
| SP-tag #238 | WLDYY | SP-tag #538 | AAPAY | SP-tag #838 | SWSLY | SP-tag #1138 | SWTWW |
| SP-tag #239 | EWDYY | SP-tag #539 | SAPAY | SP-tag #839 | PWSLY | SP-tag #1139 | EYTWW |
| SP-tag #240 | SAELA | SP-tag #540 | TAPAY | SP-tag #840 | YYSLY | SP-tag #1140 | WLTWW |

(continued)

| Number | Sequence | Number | Sequence | Number | Sequence | Number | Sequence |
|---|---|---|---|---|---|---|---|
| SP-tag #241 | ELELA | SP-tag #541 | DAPAY | SP-tag #841 | ELSWA | SP-tag #1141 | EWTWW |
| SP-tag #242 | SYELA | SP-tag #542 | EAPAY | SP-tag #842 | SYSWA | SP-tag #1142 | SLTWY |
| SP-tag #243 | WAELA | SP-tag #543 | TLPAY | SP-tag #843 | PWSWA | SP-tag #1143 | DLTWY |
| SP-tag #244 | SWELA | SP-tag #544 | LLPAY | SP-tag #844 | EYSWA | SP-tag #1144 | SYTWY |
| SP-tag #245 | TWELA | SP-tag #545 | YAPAY | SP-tag #845 | WYSWA | SP-tag #1145 | EWTWY |
| SP-tag #246 | DWELA | SP-tag #546 | ELPAY | SP-tag #846 | SASWD | SP-tag #1146 | WATYA |
| SP-tag #247 | WWELA | SP-tag #547 | SYPAY | SP-tag #847 | SLSWD | SP-tag #1147 | SWTYA |
| SP-tag #248 | AAELE | SP-tag #548 | YLPAY | SP-tag #848 | TLSWD | SP-tag #1148 | TWTYA |
| SP-tag #249 | SAELE | SP-tag #549 | TWPAY | SP-tag #849 | DWSWD | SP-tag #1149 | WLTYA |
| SP-tag #250 | TAELE | SP-tag #550 | DWPAY | SP-tag #850 | EWSWD | SP-tag #1150 | EWTYA |
| SP-tag #251 | ALELE | SP-tag #551 | EWPAY | SP-tag #851 | WYSWD | SP-tag #1151 | WWTYA |
| SP-tag #252 | EAELE | SP-tag #552 | YYPAY | SP-tag #852 | AASWE | SP-tag #1152 | TLTYE |
| SP-tag #253 | TLELE | SP-tag #553 | WYPAY | SP-tag #853 | TLSWE | SP-tag #1153 | WYTYE |
| SP-tag #254 | YAELE | SP-tag #554 | TLPWA | SP-tag #854 | ELSWE | SP-tag #1154 | WWTYE |
| SP-tag #255 | ELELE | SP-tag #555 | EYPWA | SP-tag #855 | WASWE | SP-tag #1155 | AATYL |
| SP-tag #256 | SYELE | SP-tag #556 | WYPWA | SP-tag #856 | EWSWE | SP-tag #1156 | SATYL |
| SP-tag #257 | WAELE | SP-tag #557 | SAPWD | SP-tag #857 | WYSWE | SP-tag #1157 | TATYL |
| SP-tag #258 | TYELE | SP-tag #558 | EAPWD | SP-tag #858 | WWSWE | SP-tag #1158 | ALTYL |
| SP-tag #259 | YLELE | SP-tag #559 | PLPWD | SP-tag #859 | DASWL | SP-tag #1159 | YATYL |
| SP-tag #260 | PWELE | SP-tag #560 | ELPWD | SP-tag #860 | WYSWL | SP-tag #1160 | SYTYL |
| SP-tag #261 | DWELE | SP-tag #561 | SYPWD | SP-tag #861 | AASWW | SP-tag #1161 | SWTYL |
| SP-tag #262 | SAELL | SP-tag #562 | WAPWD | SP-tag #862 | SASWW | SP-tag #1162 | DWTYL |
| SP-tag #263 | PAELL | SP-tag #563 | EYPWD | SP-tag #863 | TASWW | SP-tag #1163 | SATYP |
| SP-tag #264 | DAELL | SP-tag #564 | SAPWE | SP-tag #864 | DASWW | SP-tag #1164 | TLTYP |
| SP-tag #265 | EAELL | SP-tag #565 | TAPWE | SP-tag #865 | EASWW | SP-tag #1165 | LLTYP |
| SP-tag #266 | PLELL | SP-tag #566 | PLPWE | SP-tag #866 | PLSWW | SP-tag #1166 | SYTYP |
| SP-tag #267 | LLELL | SP-tag #567 | YAPWE | SP-tag #867 | YASWW | SP-tag #1167 | WATYP |
| SP-tag #268 | YAELL | SP-tag #568 | ELPWE | SP-tag #868 | ELSWW | SP-tag #1168 | TYTYP |
| SP-tag #269 | SYELL | SP-tag #569 | AAPWL | SP-tag #869 | SYSWW | SP-tag #1169 | SWTYP |
| SP-tag #270 | PYELL | SP-tag #570 | SAPWL | SP-tag #870 | PYSWW | SP-tag #1170 | TWTYP |
| SP-tag #271 | SWELL | SP-tag #571 | TAPWL | SP-tag #871 | SWSWW | SP-tag #1171 | DWTYP |
| SP-tag #272 | TWELL | SP-tag #572 | DAPWL | SP-tag #872 | PWSWW | SP-tag #1172 | SATYS |
| SP-tag #273 | DWELL | SP-tag #573 | EAPWL | SP-tag #873 | EYSWW | SP-tag #1173 | ALTYS |
| SP-tag #274 | EWELL | SP-tag #574 | PLPWL | SP-tag #874 | DWSWW | SP-tag #1174 | TLTYS |
| SP-tag #275 | AAELP | SP-tag #575 | YAPWL | SP-tag #875 | EWSWW | SP-tag #1175 | YATYS |
| SP-tag #276 | SAELP | SP-tag #576 | ELPWL | SP-tag #876 | YYSWW | SP-tag #1176 | DWTYS |
| SP-tag #277 | DAELP | SP-tag #577 | SYPWL | SP-tag #877 | DASWY | SP-tag #1177 | AATYT |
| SP-tag #278 | EAELP | SP-tag #578 | WAPWL | SP-tag #878 | EASWY | SP-tag #1178 | SATYT |

(continued)

| Number | Sequence | Number | Sequence | Number | Sequence | Number | Sequence |
|---|---|---|---|---|---|---|---|
| SP-tag #279 | TLELP | SP-tag #579 | TYPWL | SP-tag #879 | PLSWY | SP-tag #1179 | TATYT |
| SP-tag #280 | DLELP | SP-tag #580 | SWPWL | SP-tag #880 | DLSWY | SP-tag #1180 | ALTYT |
| SP-tag #281 | ELELP | SP-tag #581 | TWPWL | SP-tag #881 | ELSWY | SP-tag #1181 | EATYT |
| SP-tag #282 | SYELP | SP-tag #582 | WLPWL | SP-tag #882 | EYSWY | SP-tag #1182 | TLTYT |
| SP-tag #283 | PYELP | SP-tag #583 | EWPWL | SP-tag #883 | WLSWY | SP-tag #1183 | YATYT |
| SP-tag #284 | YLELP | SP-tag #584 | YYPWL | SP-tag #884 | EWSWY | SP-tag #1184 | SYTYT |
| SP-tag #285 | PWELP | SP-tag #585 | WYPWL | SP-tag #885 | PASYA | SP-tag #1185 | WATYT |
| SP-tag #286 | EYELP | SP-tag #586 | WWPWL | SP-tag #886 | LLSYA | SP-tag #1186 | TYTYT |
| SP-tag #287 | WLELP | SP-tag #587 | AAPWP | SP-tag #887 | ELSYA | SP-tag #1187 | SWTYT |
| SP-tag #288 | EWELP | SP-tag #588 | SAPWP | SP-tag #888 | WASYA | SP-tag #1188 | YYTYT |
| SP-tag #289 | YYELP | SP-tag #589 | TAPWP | SP-tag #889 | SWSYA | SP-tag #1189 | WYTYT |
| SP-tag #290 | WYELP | SP-tag #590 | DAPWP | SP-tag #890 | PWSYA | SP-tag #1190 | AATYW |
| SP-tag #291 | WWELP | SP-tag #591 | EAPWP | SP-tag #891 | WLSYA | SP-tag #1191 | SATYW |
| SP-tag #292 | SAELS | SP-tag #592 | TLPWP | SP-tag #892 | PASYD | SP-tag #1192 | TATYW |
| SP-tag #293 | SLELS | SP-tag #593 | LLPWP | SP-tag #893 | DYSYD | SP-tag #1193 | DATYW |
| SP-tag #294 | DWELS | SP-tag #594 | YAPWP | SP-tag #894 | EYSYD | SP-tag #1194 | TLTYW |
| SP-tag #295 | AAELW | SP-tag #595 | ELPWP | SP-tag #895 | EWSYD | SP-tag #1195 | ELTYW |
| SP-tag #296 | SAELW | SP-tag #596 | SYPWP | SP-tag #896 | WWSYD | SP-tag #1196 | WATYW |
| SP-tag #297 | ALELW | SP-tag #597 | WAPWP | SP-tag #897 | DASYL | SP-tag #1197 | TYTYW |
| SP-tag #298 | ELELW | SP-tag #598 | SWPWP | SP-tag #898 | DWSYL | SP-tag #1198 | DYTYW |
| SP-tag #299 | SWELW | SP-tag #599 | PWPWP | SP-tag #899 | WYSYL | SP-tag #1199 | EYTYW |
| SP-tag #300 | PWELW | SP-tag #600 | EYPWP | SP-tag #900 | ALSYP | SP-tag #1200 | YYTYW |

**5. Determination of method for effective target library construction**

Since it was impossible to clone a library of 1200 vectors at one time, a method for effective target library construction was tested. One target protein gene was cloned into vectors carrying 10 signal peptides (Table 3). A ligation-transformation-plasmid prep was conducted on a mixture of the same amounts of 10 vectors. In order to investigate the distribution of the signal peptides in the plasmids, each of the plasmids was prepped from 100 colonies and the corresponding signal peptides were sequenced (Table 4).

[Table 3] 10 signal peptides used in library construction test

| No | Signal peptides | Sequence |
|---|---|---|
| 1 | Nephronectin | MDFLLAL VL V S SLYLQA |
| 2 | Neuropeptide B | MARSATLAAAALALCLL |
| 3 | Neurotrophin-4 | MLPLPSCSLPILLLFLL |
| 4 | Neutrophil defensin 1 | MRTLAILAAILLVALQA |
| 5 | Oxytocin | MAGSSLACCLLGLLALT |
| 5 | Pancreatic alpha amylase | MKFFLLLFTIGFCWAGR |
| 7 | PPY | MAAARLCLSLLLLSTCV |
| 8 | Pepsin | MKWLLLLGLVALSECGR |

(continued)

| No | Signal peptides | Sequence |
|----|-----------------|----------|
| 9 | PENK-A | MARFLTLCTWLLLLGPG |
| 10 | Resistin | MKALCLLLLPVLGLLVS |

[Table 4] Results of sequencing for plasmids prepped from 100 colonies

| Signal peptide - 1 Correspond-ing colony No. | Signal peptide - 2 Correspond-ing colony No. | Signal peptide - 3 Correspond-ing colony No. | Signal peptide - 4 Correspond-ing colony No. | Signal peptide - 5 Correspond-ing colony No. | Signal peptide - 6 Correspond-ing colony No. | Signal peptide - 7 Correspond-ing colony No. | Signal peptide - 8 Correspond-ing colony No. | Signal peptide - 9 Correspond-ing colony No. | Signal peptide - 10 Correspond-ing colony No. | N.D. Correspond-ing colony No. |
|---|---|---|---|---|---|---|---|---|---|---|
| 18 | 1 | 2 | 8 | 7 | 4 | 12 | 16 | 21 | 5 | 3 |
| 23 | 6 | 13 | 9 | 28 | 24 | 14 | 17 | 26 | 19 | 62 |
| 34 | 10 | 22 | 11 | 32 | 31 | 40 | 39 | 29 | 20 | 74 |
| 35 | 25 | 33 | 15 | 44 | 49 | 45 | 70 | 46 | 38 | |
| 50 | 27 | 42 | 30 | 64 | 60 | 53 | 71 | 56 | 48 | |
| 52 | 37 | 43 | 36 | 66 | 77 | 76 | 80 | 61 | 55 | |
| 54 | 51 | 47 | 41 | 75 | 83 | 85 | 87 | 78 | 57 | |
| 63 | 59 | 68 | 69 | 82 | 94 | 91 | | 79 | 58 | |
| 84 | 65 | 90 | 72 | | 100 | 95 | | | 96 | |
| 86 | 67 | 97 | 81 | | | 98 | | | 99 | |
| 88 | 73 | | 89 | | | | | | | |
| 93 | 92 | | | | | | | | | |
| Total 12 | Total 12 | Total 10 | Total 11 | Total 8 | Total 9 | Total 10 | Total 7 | Total 8 | Total 10 | Total 3 |

97 plasmids were sequenced, 10 SP-tags were all identified, and signal peptides were uniformly distributed (7-12%) (Fig. 8). Equal amounts of vectors were mixed for efficient library construction. Even in this case, the library maintained its diversities irrespective of the types of the SP-tags.

**6. Validation of method for screening signal peptides capable of increasing recombinant protein production**

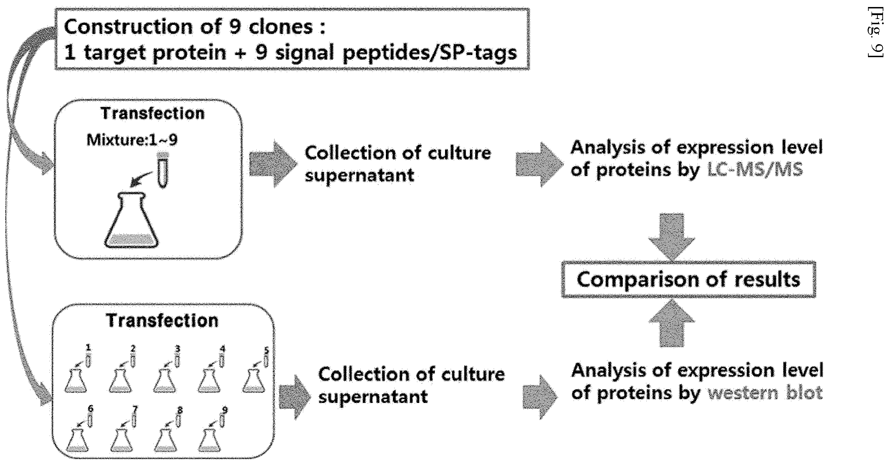

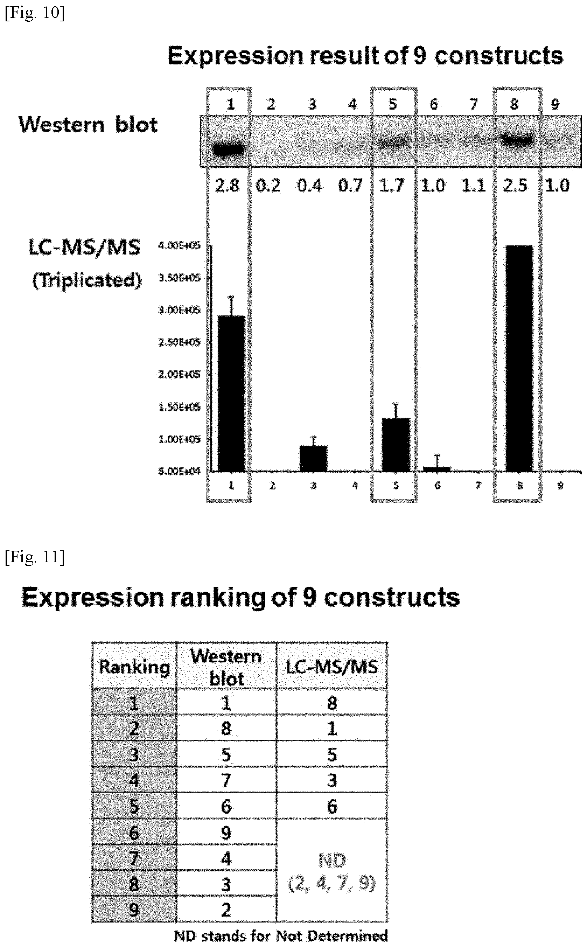

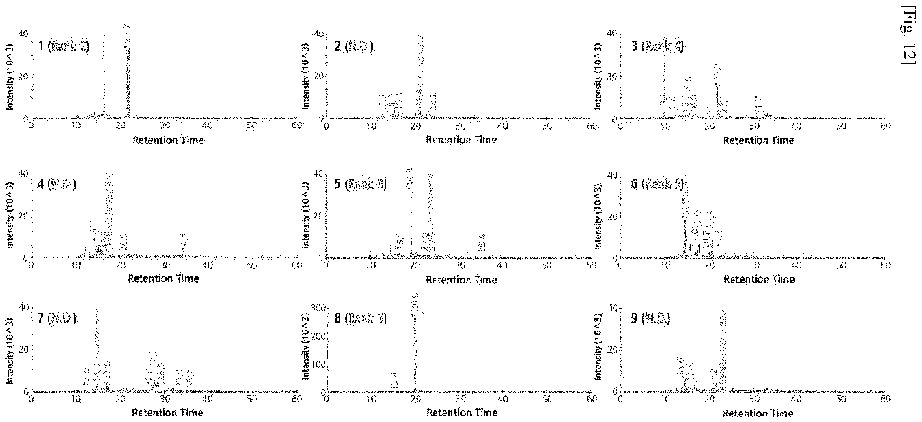

The effectiveness of a method for quantification of proteins expressed in a library by LC-MS/MS was verified by comparison with a method for quantification of independently expressed proteins by Western blot (Fig. 9). Several days after 9 signal peptide/SP-tag clones introduced with target proteins were mixed and transfected into animal cells, the SP-tagged proteins secreted to the cell culture media were relatively quantified by LC-MS/MS and ranked according to their relative amounts. Simultaneously, several days after 9 SP-tagged vectors were independently transfected in 9 different flasks, the expression levels of the SP-tagged proteins secreted to the cell culture media were measured by Western blot and compared (Fig. 10). The resulting LC-MS/MS peaks obtained based on the library expression revealed that Constructs Nos. 8, 1, and 5 showed the highest expression levels in this order and Constructs Nos. 3 and 6 followed in this order (Figs. 10 to 12). Constructs Nos. 2, 4, 7, and 9 could not be ranked because their expression levels were significantly low. The results of Western blot for the independently expressed constructs showed that Constructs Nos. 1, 8, and 5 were ranked as the top 3 constructs because of their highest expression levels and Constructs Nos. 7, 6, 9, 4, 3, and 2 followed in this order (Figs. 10 and 11). In light of the aim of the present invention to select signal peptides that can best increase the production of a target protein, the same top 3 constructs determined in both experiments verified the effectiveness of the method for screening signal peptides using the SP-tag library.

**7. Test for screening optimal signal peptides using SP-tags**

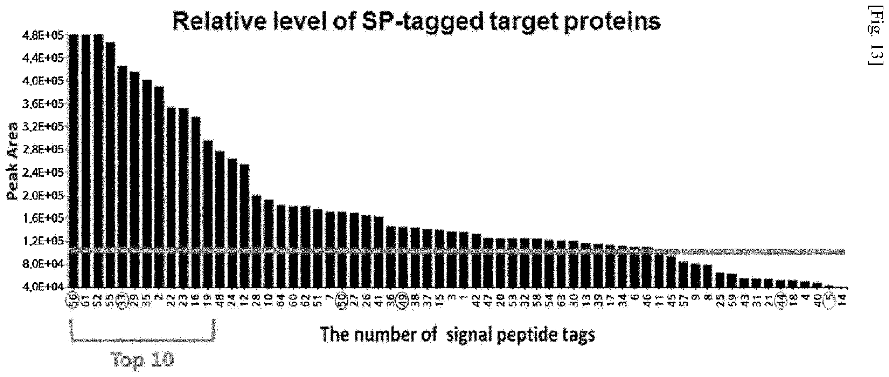

Human vascular endothelial growth factor (hVEGF) as a target protein was cloned into the signal peptide/SP-tag library to construct a target protein library. Three days after expression of the library in HEK 293 cells, the cell culture media were collected and the expressed protein was isolated and purified using His tags. After treatment of the protein samples with trypsin, the peak area values were calculated by LC-MS/MS to determine the relative amounts of the SP-tags (Fig. 13).

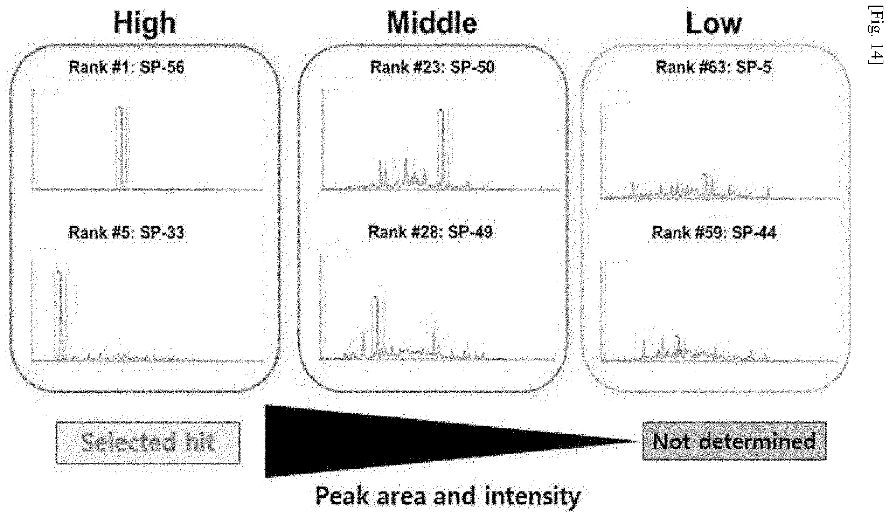

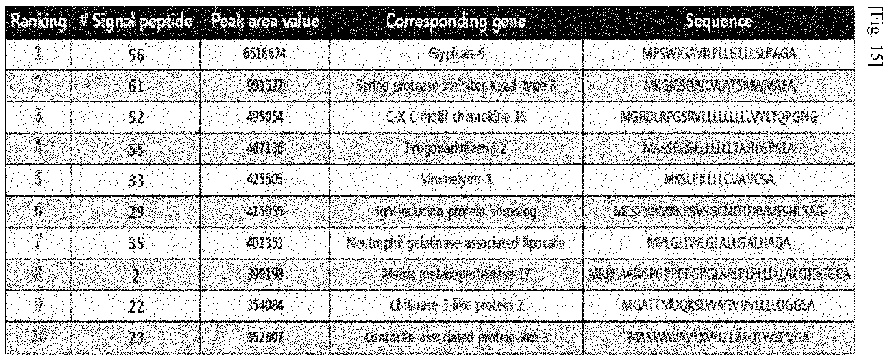

The resulting LC-MS/MS peaks revealed that there were distinct differences in peak height, sharpness, and noise intensity depending on whether the expression levels were high or low (Fig. 14). SP-tags with high expression levels appeared as clearly distinguishable single peaks, whereas SP-tags with low expression levels were not distinguished from noise signals. Signal peptides corresponding to the top 10 SP-tags whose expression levels were found to be high in Fig. 13 were selected (Fig. 15).

**8. Synthesis of standard peptides for SP-tag quantification**

To develop an MRM assay for screening 1,200 signal peptides, isotope labeled peptides (containing $^{15}N$ and $^{13}C$-labeled arginine residues) based on 1,200 barcoding sequences were synthesized as internal standards (JPT Peptide Technologies, Berlin, Germany). Each of the 1,200 internal standards was added at a concentration of 100 fmol/$\mu$l to the protein expression mixture prior to MRM analysis. The internal standards were used to accurately distinguish between non-specific peaks and target peaks based on their retention times (RT) during MRM analysis. A global standard peptide (EQVTNVGGAVVTGVTAVAQK) was synthesized based on an isotope-labeled peptide (containing $^{15}N$ and $^{13}C$-labeled lysine residues) to minimize errors between groups during analysis. The global standard peptide had a peptide sequence that was absent present in the samples. The global standard peptide was added at a concentration of 50 fmol/$\mu$l to the protein expression mixture prior to MRM analysis.

[Table 5] List of chemically synthesized SP tag peptides

| Number | Sequence | Number | Sequence | Number | Sequence | Number | Sequence |
|---|---|---|---|---|---|---|---|
| Peptide 001 | GAAPAPGHHHHHHR | Peptide 301 | GSYTLSGHHHHHHR | Peptide 601 | GWWPAAGHHHHHHR | peptide 901 | GEYPWPGHHHHHHR |
| Peptide_002 | GTAPAAGHHHHHHR | Peptide_302 | GSLSYTGHHHHHHR | Peptide_602 | GAAPWWGHHHHHHR | Peptide_902 | GLLPYWGHHHHHHR |
| Peptide_003 | GALSAAGHHHHHHR | Peptide_303 | GTWPAPGHHHHHHR | Peptide_603 | GSYTYPGHHHHHHR | Peptide_903 | GWWDADGHHHHHHR |
| Peptide_004 | GAASLAGHHHHHHR | Peptide_304 | GTAPWPGHHHHHHR | Peptide_604 | GYYTLAGHHHHHHR | Peptide_904 | GEWTWAGHHHHHHR |
| Peptide_005 | GSADAAGHHHHHHR | Peptide_305 | GELTLPGHHHHHHR | Peptide_605 | GYATYLGHHHHHHR | Peptide_905 | GWAEWTGHHHHHHR |
| Peptide_006 | GAATATGHHHHHHR | Peptide_306 | GDLDLPGHHHHHHR | Peptide_606 | GEWPAEGHHHHHHR | Peptide_906 | GEATWWGHHHHHHR |
| Peptide_007 | GTASASGHHHHHHR | Peptide_307 | GTLELPGHHHHHHR | Peptide_607 | GPAEWEGHHHHHHR | Peptide_907 | GDADWWUHHHHHHR |
| Peptide_008 | GSASATGHHHHHHR | Peptide_308 | GEWPAAGHHHHHHR | Peptide_608 | GALELWGHHHHHHR | Peptide_908 | GTAEWWGHHHHHHR |
| Peptide_009 | GPAPASGHHHHHHR | Peptide_309 | GWAPAEGHHHHHHR | Peptide_609 | GLLSLWGHHHHHHR | Peptide_909 | GYYSYPGHHHHHHR |
| Peptide_010 | GSAPAPGHHHHHHR | Peptide_310 | GPAEWAGHHHHHHR | Peptide_610 | GEYSAYGHHHHHHR | Peptide_910 | GPWDLYGHHHHHHR |
| Peptide_011 | GALPAAGHHHHHHR | Peptide_311 | GAAEWPGHHHHHHR | Peptide_611 | GSAEYYGHHHHHHR | Peptide_911 | GPYDWLGHHHHHHR |
| Peptide_012 | GAAPALGHHHHHHR | Peptide_312 | GWLSAPGHHHHHHR | Peptide_612 | GTADYYGHHHHHHR | Peptide_912 | GPLDYWGHHHHHHR |
| Peptide_013 | GSATAPGHHHHHHR | Peptide_313 | GSWPALGHHHHHHR | Peptide_613 | GELELEGHHHHHHR | Peptide_913 | GYYDAYGHHHHHHR |
| Peptide_014 | GALTAAGHHHHHHR | Peptide_314 | GSAPWLGHHHHHHR | Peptide_614 | GYYSLSGHHHHHHR | Peptide_914 | GYADYYGHHHHHHR |
| Peptide 015 | GAATLAGHHHHHHR | Peptide_315 | GYYSAAGHHHHHHR | Peptide_615 | GSYSLYGHHHHHHR | Peptide_915 | GDWDYPGHHHHHHR |

(continued)

| Number | Sequence | Number | Sequence | Number | Sequence | Number | Sequence |
|---|---|---|---|---|---|---|---|
| Peptide_016 | GEASAAGHHHHHHR | Peptide_316 | GYASAYGHHHHHHR | Peptide_616 | GEWDLAGHHHHHHR | Peptide_916 | GPWDYDGHHHHHHR |
| Peptide_017 | GTADAAGHHHHHHR | Peptide_317 | GELELAGHHHHHHR | Peptide_617 | GDWELAGHHHHHHR | Peptide_917 | GWYTLLGHHHHHHR |
| Peptide_018 | GSASLAGHHHHHHR | Peptide_318 | GEAELLGHHHHHHR | Peptide_618 | GEADWLGHHHHHHR | Peptide_918 | GYYSYTGHHHHHHR |
| Peptide_019 | GAASLSGHHHHHHR | Peptide_319 | GALELEGHHHHHHR | Peptide_619 | GWYPAPGHHHHHHR | Peptide_919 | GEWEYAGHHHHHHR |
| Peptide_020 | GDASASGHHHHHHR | Peptide_320 | GDLTLLGHHHHHHR | Peptide_620 | GYAPWPGHHHHHHR | Peptide_920 | GEYEWAGHHHHHHR |
| Peptide_021 | GSATATGHHHHHHR | Peptide_321 | GTLDLLGHHHHHHR | Peptide_621 | GSWDLLGHHHHHHR | Peptide_921 | GWAEYEGHHHHHHR |
| Peptide_022 | GTAPAPGHHHHHHR | Peptide_322 | GSWPADGHHHHHHR | Peptide_622 | GLLTWTGHHHHHHR | Peptide_922 | GYAEWEGHHHHHHR |
| Peptide_023 | GAAPAEGHHHHHHK | Peptide_323 | GSAPWDGHHHHHHR | Peptide_623 | GTLTLWGHHHHHHR | Peptide_923 | GEAEYWGHHHHHHR |
| Peptide_024 | GPLSAAGHHHHHHR | Peptide_324 | GTAPWTGHHHHHHR | Peptide_624 | GWATAWGHHHHHHR | Peptide_924 | GWYSLEGHHHHHHR |
| Peptide_025 | GSLPAAGHHHHHHR | Peptide_325 | GWLDAAGHHHHHHR | Peptide_625 | GAATWWGHHHHHHR | Peptide_925 | GSWEYLGHHHHHHR |
| Peptide_026 | GPASLAGHHHHHHR | Peptide_326 | GWADALGHHHHHHR | Peptide_626 | GTYSYTGHHHHHHR | Peptide_926 | GSYEWLGHHHHHHR |
| Peptide_027 | GSADAPGHHHHHHR | Peptide_327 | GAADWLGHHHHHHR | Peptide_627 | GSYTYTGHHHHHHR | Peptide_927 | GELSWYGHHHHHHR |
| Peptide_028 | GTATAPGHHHHHHR | Peptide_328 | GDLSLEGHHHHHHR | Peptide_628 | GYLELPGHHHHHHR | Peptide_928 | GDWTYLGHHHHHHR |
| Peptide_029 | GALDAAGHHHHHHR | Peptide_329 | GELTLTGHHHHHHR | Peptide_629 | GPYELLGHHHHHHR | Peptide_929 | GDYTWLGHHHHHHR |
| Peptide_030 | GSATLAGHHHHHHR | Peptide_330 | GPLSYPGHHHHHHR | Peptide_630 | GEWTAEGHHHHHHR | Peptide_930 | GDLTWYGHHHHHHR |

| Number | Sequence | Number | Sequence | Number | Sequence | Number | Sequence |
|---|---|---|---|---|---|---|---|
| Peptide_031 | GAASLTGHHHHHHR | Peptide_331 | GLLPAYGHHHHHHR | Peptide_631 | GEADWDGHHHHHHR | Peptide_931 | GEWSYDGHHHHHHR |
| Peptide_032 | GDATASGHHHHHHR | Peptide_332 | GEATWAGHHHHHHR | Peptide_632 | GDAEWDGHHHHHHR | Peptide_932 | GSYEWDGHHHHHHR |
| Peptide_033 | GTASADGHHHHHHR | Peptide_333 | GAADWDGHHHHHHR | Peptide_633 | GDWDLSGHHHHHHR | Peptide_933 | GEYTWTGHHHHHHR |
| Peptide_034 | GSLSASGHHHHHHR | Peptide_334 | GWLSATGHHHHHHR | Peptide_634 | GTLSWEGHHHHHHR | Peptide_934 | GTYTWEGHHHHHHR |
| Peptide_035 | GDAPAPGHHHHHHR | Peptide_335 | GTWSLAGHHHHHHR | Peptide_635 | GWWSASGHHHHHHR | Peptide_935 | GWYPAYGHHHHHHR |
| Peptide_036 | GTLPAAGHHHHHHR | Peptide_336 | GWASLTGHHHHHHR | Peptide_636 | GSASWWGHHHHHHR | Peptide_936 | GYYPAWGHHHHHHR |
| Peptide_037 | GTAPALGHHHHHHR | Peptide_337 | GTLSAWGHHHHHHR | Peptide_637 | GEYDLPGHHHHHHR | Peptide_937 | GWAPYYGHHHHHHR |
| Peptide 038 | GAATLPGHHHHHHR | Peptide_338 | GSATWLGHHHHHHR | Peptide_638 | GELDYPGHHHHHHR | Peptide_938 | GYAPYWGHHHHHHR |
| Peptide 039 | GEASAPGHHHHHHR | Peptide_339 | GPLDAYGHHHHHHR | Peptide_639 | GWYTAPGHHHHHHR | Peptide_939 | GPWDWPGHHHHHHR |
| Peptide 040 | GTAPADGHHHHHHR | Peptide_340 | GEWSASGHHHHHHR | Peptide_640 | GTWPAYGHHHHHHR | Peptide_940 | GELDYYGHHHHHHR |
| Peptide 041 | GSASLPGHHHHHHR | Peptide_341 | GSAEWSGHHHHHHR | Peptide_641 | GWATYPGHHHHHHR | Peptide_941 | GDLEYYGHHHHHHR |
| Peptide 042 | GALSLAGHHHHHHR | Peptide_342 | GTWSADGHHHHHHR | Peptide_642 | GYATWPGHHHHHHR | Peptide_942 | GWWTLPGHHHHHHR |
| Peptide 043 | GEADAAGHHHHHHR | Peptide_343 | GTATWTGHHHHHHR | Peptide_643 | GTAPYWGHHHHHHR | Peptide_943 | GWLPWTGHHHHHHR |
| Peptide 044 | GDLSAAGHHHHHHR | Peptide_344 | GSWSLSGHHHHHHR | Peptide_644 | GEYTLLGHHHHHHR | Peptide_944 | GTWPWLGHHHHHHR |
| Peptide 045 | GSADALGHHHHHHR | Peptide_345 | GPADYDGHHHHHHR | Peptide_645 | GELTLYGHHHHHHR | Peptide 945 | GTLPWWGHHHHHHR |

EP 4 026 904 A1

59

| Number | Sequence | Number | Sequence | Number | Sequence | Number | Sequence |
|---|---|---|---|---|---|---|---|
| Peptide 046 | GTATLAGHHHHHHR | Peptide_346 | GEYTAPGHHHHHHR | Peptide_646 | GTLEYLGHHHHHHR | Peptide_946 | GWYTAYGHHHHHHR |
| Peptide 047 | GAATLTGHHHHHHR | Peptide_347 | GEAPYTGHHHHHHR | Peptide_647 | GYAEWAGHHHHHHR | Peptide_947 | GYYTAWGHHHHHHR |
| Peptide 048 | GEASATGHHHHHHR | Peptide_348 | GTYSLPGHHHHHHR | Peptide_648 | GAAEYWGHHHHHHR | Peptide_948 | GEYDYDGHHHHHHR |
| Peptide 049 | GSADADGHHHHHHR | Peptide_349 | GSYTLPGHHHHHHR | Peptide_649 | GWYSALGHHHHHHR | Peptide_949 | GDYEYDGHHHHHHR |
| Peptide 050 | GDATATGHHHHHHR | Peptide_350 | GALTYLGHHHHHHR | Peptide_650 | GWLSYAGHHHHHHR | Peptide_950 | GTYEYEGHHHHHHR |
| Peptide 051 | GTLSASGHHHHHHR | Peptide_351 | GEAEYAGHHHHHHR | Peptide_651 | GYLSAWGHHHHHHR | Peptide_951 | GWWELAGHHHHHHR |
| Peptide 052 | GTASLSGHHHHHHR | Peptide_352 | GAAEYEGHHHHHHR | Peptide_652 | GEAEYEGHHHHHHR | Peptide_952 | GWLEWAGHHHHHHR |
| Peptide 053 | GAASAYGHHHHHHR | Peptide_353 | GSYELAGHHHHHHR | Peptide_653 | GEYSLEGHHHHHHR | Peptide_953 | GWAEWLGHHHHHHR |
| Peptide 054 | GEAPAPGHHHHHHR | Peptide_354 | GELSYAGHHHHHHR | Peptide_654 | GSYELEGHHHHHHR | Peptide_954 | GALEWWGHHHHHHR |
| Peptide 055 | GPAPAEGHHHHHHR | Peptide_355 | GSAELYGHHHHHHR | Peptide_655 | GTLDYEGHHHHHHR | Peptide_955 | GELEWEGHHHHHHR |
| Peptide 056 | GALPALGHHHHHHR | Peptide_356 | GDYTLAGHHHHHHR | Peptide_656 | GSWDYAGHHHHHHR | Peptide_956 | GWYSYSGHHHHHHR |
| Peptide 057 | GDLPAAGHHHHHHR | Peptide_357 | GTYDALGHHHHHHR | Peptide_657 | GDASWYGHHHHHHR | Peptide_957 | GSYSYWGHHHHHHR |
| Peptide 058 | GEAPATGHHHHHHR | Peptide_358 | GTADLYGHHHHHHR | Peptide_658 | GSADYWGHHHHHHR | Peptide_958 | GWADWEGHHHHHHR |
| Peptide 059 | GDADAPGHHHHHHR | Peptide_359 | GLLSYSGHHHHHHR | Peptide_659 | GWYTATGHHHHHHR | Peptide_959 | GEADWWGHHHHHHR |
| Peptide 060 | GAATLLGHHHHHHR | Peptide_360 | GWLPAPGHHHHHHR | Peptide 660 | GTWTYAGHHHHHHR | Peptide_960 | GDWSLWGHHHHHHR |

EP 4 026 904 A1

60

(continued)

| Number | Sequence | Number | Sequence | Number | Sequence | Number | Sequence |
|---|---|---|---|---|---|---|---|
| Peptide 061 | GELSAAGHHHHHHR | Peptide_361 | GPWPALGHHHHHHR | Peptide_661 | GWATYTGHHHHHHR | Peptide_961 | GSWDWLGHHHHHHR |
| Peptide 062 | GSAELAGHHHHHHR | Peptide_362 | GDYSAEGHHHHHHR | Peptide_662 | GYATWTGHHHHHHR | Peptide_962 | GWWTLTGHHHHHHR |
| Peptide 063 | GDLTAAGHHHHHHR | Peptide_363 | GSADYEGHHHHHHR | Peptide_663 | GTATYWGHHHHHHR | Peptide_963 | GTLTWWGHHHHHHR |
| Peptide 064 | GTLDAAGHHHHHHR | Peptide_364 | GEYTATGHHHHHHR | Peptide_664 | GPWELPGHHHHHHR | Peptide_964 | GTYPYYGHHHHHHR |
| Peptide 065 | GTADALGHHHHHHR | Peptide_365 | GTYDADGHHHHHHR | Peptide_665 | GELPWPGHHHHHHR | Peptide_965 | GWYELPGHHHHHHR |
| Peptide 066 | GALSLSGHHHHHHR | Peptide_366 | GEATYTGHHHHHHR | Peptide_666 | GPLPWEGHHHHHHR | Peptide 966 | GELPWYGHHHHHHR |
| Peptide 067 | GTLSATGHHHHHHR | Peptide_367 | GYYPAAGHHHHHHR | Peptide_667 | GYYPAEGHHHHHHR | Peptide_967 | GPLEYWGHHHHHHR |
| Peptide 068 | GSLTATGHHHHHHR | Peptide_368 | GSYTLTGHHHHHHR | Peptide_668 | GPYEYAGHHHHHHR | Peptide_968 | GDWSWDGHHHHHHR |
| Peptide 069 | GTASLTGHHHHHHR | Peptide_369 | GYAPAYGHHHHHHR | Peptide_669 | GEAPYYGHHHHHHR | Peptide_969 | GYYSLYGHHHHHHR |
| Peptide 070 | GSATLTGHHHHHHR | Peptide_370 | GTLTYSGHHHHHHR | Peptide_670 | GPAEYYGHHHHHHR | Peptide_970 | GEWPYDGHHHHHHR |
| Peptide 071 | GAAPAYGHHHHHHR | Peptide_371 | GAAPYYGHHHHHHR | Peptide_671 | GSWSYTGHHHHHHR | Peptide_971 | GEYPWDGHHHHHHR |
| Peptide 072 | GPLPAPGHHHHHHR | Peptide_372 | GPLELLGHHHHHHR | Peptide_672 | GWLDLPGHHHHHHR | Peptide_972 | GDYEWPGHHHHHHR |
| Peptide 073 | GYASASGHHHHHHR | Peptide_373 | GPWDAPGHHHHHHR | Peptide_673 | GPWDLLGHHHHHHR | Peptide_973 | GSYDYYGHHHHHHR |
| Peptide 074 | GSASAYGHHHHHHR | Peptide_374 | GDAPWPGHHHHHHR | Peptide_674 | GPLDWLGHHHHHHR | Peptide_974 | GYYTYTGHHHHHHR |
| Peptide 075 | GAASYSGHHHHHHR | Peptide 375 | GELDLPGHHHHHHR | Peptide 675 | GDYPYSGHHHHHHK | Peptide 975 | GEWTLYGHHHHHHR |

61

| Number | Sequence | Number | Sequence | Number | Sequence | Number | Sequence |
|---|---|---|---|---|---|---|---|
| Peptide 076 | GPATLPGHHHHHHR | Peptide_376 | GDLELPGHHHHHHR | Peptide_676 | GSYDYPGHHHHHHR | Peptide_976 | GDWDLYGHHHHHHR |
| Peptide 077 | GELPAAGHHHHHHR | Peptide_377 | GLLDLLGHHHHHHR | Peptide_677 | GTYTYPGHHHHHHR | Peptide_977 | GEYTWLGHHHHHHR |
| Peptide 078 | GEAPALGHHHHHHR | Peptide_378 | GWLTAPGHHHHHHR | Peptide_678 | GYYDLAGHHHHHHR | Peptide_978 | GELTYWGHHHHHHR |
| Peptide 079 | GALPAEGHHHHHHR | Peptide_379 | GTLPWAGHHHHHHR | Peptide_679 | GYADYLGHHHHHHR | Peptide_979 | GDLDYWGHHHHHHR |
| Peptide 080 | GAAELPGHHHHHHR | Peptide_380 | GTAPWLGHHHHHHR | Peptide_680 | GELPWTGHHHHHHR | Peptide 980 | GWWSAYGHHHHHHR |
| Peptide-081 | GPLSLAGHHHHHHR | Peptide_381 | GYATAYGHHHHHHR | Peptide_681 | GWLTLLGHHHHHHR | Peptide_981 | GWYSWAGHHHHHHR |
| Peptide 082 | GEADAPGHHHHHHR | Peptide_382 | GELTLLGHHHHHHR | Peptide_682 | GPWSWAGHHHHHHR | Peptide_982 | GWASYWGHHHHHHR |
| Peptide 083 | GSADLPGHHHHHHR | Peptide_383 | GEWSAPGHHHHHHR | Peptide_683 | GYAEYTGHHHHHHR | Peptide 983 | GYASWWGHHHHHHR |
| Peptide 084 | GTLPATGHHHHHHR | Peptide_384 | GSAPWEGHHHHHHR | Peptide_684 | GDADYYGHHHHHHR | Peptide_984 | GSWEYEGHHHHHHR |
| Peptide 085 | GLLDAAGHHHHHHR | Peptide_385 | GDATWPGHHHHHHR | Peptide_685 | GSAPWWGHHHHHHR | Peptide_985 | GSYEWEGHHHHHHR |
| Peptide 086 | GALDLAGHHHHHHR | Peptide_386 | GTADWPGHHHHHHR | Peptide_686 | GYYSLTGHHHHHHR | Peptide_986 | GEYTWDGHHHHHHR |
| Peptide 087 | GEATLAGHHHHHHR | Peptide_387 | GWAELAGHHHHHHR | Peptide_687 | GSYTYLGHHHHHHR | Peptide_987 | GDYEWTGHHHHHHR |
| Peptide 088 | GLLTASGHHHHHHR | Peptide_388 | GAAELWGHHHHHHR | Peptide_688 | GWAELEGHHHHHHR | Peptide 988 | GTYEWDGHHHHHHR |
| Peptide 089 | GTASLLGHHHHHHR | Peptide_389 | GPWSLSGHHHHHHR | Peptide_689 | GEAEWLGHHHHHHR | Peptide_989 | GEWPWPGHHHHHHR |
| Peptide 090 | GSATLLGHHHHHHR | Peptide_390 | GSWSLPGHHHHHHR | Peptide_690 | GALEWEGHHHHHHR | Peptide 990 | GWLPWLGHHHHHHR |

EP 4 026 904 A1

62

| Number | Sequence | Number | Sequence | Number | Sequence | Number | Sequence |
|---|---|---|---|---|---|---|---|
| Peptide 091 | GAASAWGHHHHHHR | Peptide_391 | GWLSLAGHHHHHHR | Peptide_691 | GEWSLLGHHHHHHR | Peptide_991 | GLLPWWGHHHHHHR |
| Peptide 092 | GDADADGHHHHHHR | Peptide_392 | GEAELEGHHHHHHR | Peptide_692 | GSWELLGHHHHHHR | Peptide_992 | GWYSYPGHHHHHHR |
| Peptide 093 | GTADAEGHHHHHHR | Peptide_393 | GYYSASGHHHHHHR | Peptide_693 | GELSLWGHHHHHHR | Peptide_993 | GSYPYWGHHHHHHR |
| Peptide 094 | GELSASGHHHHHHR | Peptide_394 | GSYSAYGHHHHHHR | Peptide_694 | GWWDAAGHHHHHHR | Peptide_994 | GEYELYGHHHHHHR |
| Peptide 095 | GSAELSGHHHHHHR | Peptide 395 | GYASYSGHHHHHHR | Peptide 695 | GEYSYSGHHHHHHR | Peptide 995 | GELEYYGHHHHHHR |
| Peptide 096 | GTLTATGHHHHHHR | Peptide_396 | GELSLEGHHHHHHR | Peptide_696 | GAADWWGHHHHHHR | Peptide_996 | GWWDLPGHHHHHHR |
| Peptide 097 | GPYSAAGHHHHHHR | Peptide_397 | GPYTLPGHHHHHHR | Peptide_697 | GTYTYTGHHHHHHR | Peptide_997 | GPLDWWGHHHHHHR |
| Peptide 098 | GSYPAAGHHHHHHR | Peptide_398 | GEWDAAGHHHHHHR | Peptide_698 | GEAEWDGHHHHHHR | Peptide_998 | GWYDAYGHHHHHHR |
| Peptide 099 | GYASAPGHHHHHHR | Peptide_399 | GAADWEGHHHHHHR | Peptide_699 | GEWSLDGHHHHHHR | Peptide_999 | GWADYYGHHHHHHR |
| Peptide 100 | GPASYAGHHHHHHR | Peptide_400 | GWLSADGHHHHHHR | Peptide_700 | GDWELSGHHHHHHR | Peptide_1000 | GEYDYEGHHHHHHR |
| Peptide 101 | GSAPAYGHHHHHHR | Peptide_401 | GSWDLAGHHHHHHR | Peptide_701 | GELTWTGHHHHHHR | Peptide_1001 | GEWTWPGHHHHHHR |
| Peptide 102 | GAADAYGHHHHHHR | Peptide_402 | GWASLDGHHHHHHR | Peptide_702 | GDLDWTGHHHHHHR | Peptide_1002 | GDWDWPGHHHHHHR |
| Peptide 103 | GLLPAPGHHHHHHR | Peptide_403 | GDASWLGHHHHHHR | Peptide_703 | GTLDWDGHHHHHHR | Peptide_1003 | GWWTLLGHHHHHHR |
| Peptide 104 | GPLPALGHHHHHHR | Peptide_404 | GSADLWGHHHHHHR | Peptide_704 | GSYPWPGHHHHHHR | Peptide 1004 | GYLPYYGHHHHHHR |
| Peptide 105 | GSYTAAGHHHHHHR | Peptide_405 | GTLTWAGHHHHHHR | Peptide_705 | GWYPALGHHHHHHR | Peptide_1005 | GWYSYTGHHHHHHR |

| Number | Sequence | Number | Sequence | Number | Sequence | Number | Sequence |
|---|---|---|---|---|---|---|---|
| Peptide 106 | GYASATGHHHHHHR | Peptide_406 | GTATWLGHHHHHHR | Peptide_706 | GYAPWLGHHHHHHR | Peptide_1006 | GTYSYWGHHHHHHR |
| Peptide 107 | GSASYSGHHHHHHR | Peptide_407 | GEYPALGHHHHHHR | Peptide_707 | GWWSATGHHHHHHR | Peptide_1007 | GSYTWYGHHHHHHR |
| Peptide 108 | GALTLPHHHHHHHR | Peptide_408 | GELPAYGHHHHHHR | Peptide 708 | GSWTAWGHHHHHHR | Peptide_1008 | GEWEWAGHHHHHHR |
| Peptide 109 | GWAPAAGHHHHHHR | Peptide_409 | GPLEYAGHHHHHHR | Peptide_709 | GTASWWGHHHHHHR | Peptide_1009 | GWAEWEGHHHHHHR |
| Peptide 110 | GELSAPGHHHHHHR | Peptide_410 | GLLPYSGHHHHHHR | Peptide_710 | GSATWWGHHHHHHR | Peptide_1010 | GEAEWWGHHHHHHR |
| Peptide 111 | GSAELPGHHHHHHR | Peptide_411 | GEWTASGHHHHHHR | Peptide_711 | GEYELPGHHHHHHR | Peptide_1011 | GWWSLEGHHHHHHR |
| Peptide 112 | GDLTAPGHHHHHHR | Peptide_412 | GTWSAEGHHHHHHR | Peptide_712 | GPLEYEGHHHHHHR | Peptide_1012 | GSWELWGHHHHHHR |
| Peptide 113 | GTLPADGHHHHHHR | Peptide_413 | GTAEWSGHHHHHHR | Peptide_713 | GWYDAPGHHHHHHR | Peptide_1013 | GELSWWGHHHHHHR |
| Peptide 114 | GTADLPGHHHHHHR | Peptide 414 | GSATWEGHHHHHHR | Peptide 714 | GDWPAYGHHHHHHR | Peptide 1014 | GDWTLWGHHHHHHR |
| Peptide 115 | GEADLAGHHHHHHR | Peptide_415 | GDATWTGHHHHHHR | Peptide_715 | GYADWPGHHHHHHR | Peptide_1015 | GTWDLWGHHHHHHR |
| Peptide 116 | GYAPAPGHHHHHHR | Peptide_416 | GSWTLSGHHHHHHR | Peptide_716 | GPADYWGHHHHHHR | Peptide_1016 | GDLTWWGHHHHHHR |
| Peptide 117 | GDLSLAGHHHHHHR | Peptide_417 | GEYDAPGHHHHHHR | Peptide_717 | GEYDLLGHHHHHHR | Peptide_1017 | GYYDYPGHHHHHHR |
| Peptide 118 | GSADLLGHHHHHHR | Peptide_418 | GEAPYDGHHHHHHR | Peptide_718 | GELDLYGHHHHHHR | Peptide_1018 | GEWSWDGHHHHHHR |
| Peptide 119 | GTLTLAGHHHHHHR | Peptide_419 | GSYDLPGHHHHHHR | Peptide_719 | GDLELYGHHHHHHR | Peptide_1019 | GDWEWSGHHHHHHR |
| Peptide 120 | GTATLLGHHHHHHR | Peptide_420 | GTLTYPGHHHHHHR | Peptide_720 | GPLPYYGHHHHHHR | Peptide_1020 | GWWPYAGHHHHHHR |

EP 4 026 904 A1

64

| Number | Sequence | Number | Sequence | Number | Sequence | Number | Sequence |
|---|---|---|---|---|---|---|---|
| Peptide 121 | GWATAAGHHHHHHR | Peptide_421 | GEYTLAGHHHHHHR | Peptide 721 | GSWTYPGHHHHHHR | Peptide 1021 | GWYPWAGHHHHHHR |
| Peptide 122 | GELSATGHHHHHHR | Peptide_422 | GYLTAEGHHHHHHR | Peptide_ 722 | GSYPWTGHHHHHHR | Peptide 1022 | GWAPWYGHHHHHHR |
| Peptide 123 | GEATLSGHHHHHHR | Peptide_423 | GELTAYGHHHHHHR | Peptide 723 | GWYTLAGHHHHHHR | Peptide_ 1023 | GYAPWWGHHHHHHR |
| Peptide 124 | GTASLEGHHHHHHR | Peptide_424 | GDLDAYGHHHHHHR | Peptide_ 724 | GWLTYAGHHHHHHR | Peptide_ 1024 | GYYTLYGHHHHHHR |
| Peptide 125 | GDLSADGHHHHHHR | Peptide_425 | GEATLYGHHHHHHR | Peptide_ 725 | GWATLYGHHHHHHR | Peptide_ 1025 | GSYEYYGHHHHHHR |
| Peptide 126 | GDASLDGHHHHHHR | Peptide_426 | GDADYLGHHHHHHR | Peptide_ 726 | GYATWLGHHHHHHR | Peptide_ 1026 | GTYDYYGHHHHHHR |
| Peptide 127 | GTLSLSGHHHHHHR | Peptide_427 | GTAEYLGHHHHHHR | Peptide_ 727 | GTYELEGHHHHHHR | Peptide_ 1027 | GDWELYGHHHHHHR |
| Peptide 128 | GSWSAAGHHHHHHR | Peptide_428 | GTYSLLGHHHHHHR | Peptide_ 728 | GELDYDGHHHHHHR | Peptide_ 1028 | GELDYWGHHHHHHR |
| Peptide 129 | GSASAWGHHHHHHR | Peptide_429 | GSYTLLGHHHHHHR | Peptide_ 729 | GDLDYEGHHHHHHR | Peptide_ 1029 | GYYPWPGHHHHHHR |
| Peptide 130 | GTAPAYGHHHHHHR | Peptide_430 | GLLSYTGHHHHHHR | Peptide_ 730 | GPYDYPGHHHHHHR | Peptide_ 1030 | GWWTYAGHHHHHHR |
| Peptide 131 | GPYSASGHHHHHHR | Peptide_431 | GWYSAAGHHHHHHR | Peptide_ 731 | GEWSAYGHHHHHHR | Peptide_ 1031 | GWYTWAGHHHHHHR |
| Peptide 132 | GSYPASGHHHHHHR | Peptide_432 | GWASYAGHHHHHHR | Peptide_ 732 | GEYSWAGHHHHHHR | Peptide_ 1032 | GWATYWGHHHHHHR |
| Peptide 133 | GPASYSGHHHHHHR | Peptide_433 | GYASAWGHHHHHHR | Peptide_ 733 | GSYEWAGHHHHHHR | Peptide_ 1033 | GEWDYDGHHHHHHR |
| Peptide 134 | GSAPYSGHHHHHHR | Peptide_434 | GAASYWGHHHHHHR | Peptide_ 734 | GEASWYGHHHHHHR | Peptide_ 1034 | GDWEYDGHHHHHHR |
| Peptide 135 | GALSAYGHHHHHHR | Peptide_435 | GSAEYEGHHHHHHR | Peptide_ 735 | GSAEWYGHHHHHHR | Peptide_ 1035 | GEYEWTGHHHHHHR |

EP 4 026 904 A1

65

| Number | Sequence | Number | Sequence | Number | Sequence | Number | Sequence |
|---|---|---|---|---|---|---|---|
| Peptide 136 | GSYDAAGHHHHHHR | Peptide_436 | GDADYDGHHHHHHR | Peptide_736 | GDATYWGHHHHHHR | Peptide_1036 | GDYDWEGHHHHHHR |
| Peptide 137 | GSADAYGHHHHHHR | Peptide_437 | GTADYEGHHHHHHR | Peptide_737 | GWYSLSGHHHHHHR | Peptide_1037 | GTYEWEGHHHHHHR |
| Peptide 138 | GTYTAAGHHHHHHR | Peptide_438 | GELSYSGHHHHHHR | Peptide_738 | GWLSYSGHHHHHHR | Peptide_1038 | GWWSYSGHHHHHHR |
| Peptide 139 | GYATATGHHHHHHR | Peptide_439 | GSYDLTGHHHHHHR | Peptide_739 | GSWSLYGHHHHHHR | Peptide_1039 | GSYSWWGHHHHHHR |
| Peptide 140 | GTATAYGHHHHHHR | Peptide_440 | GTYTLTGHHHHHHR | Peptide_740 | GWWPAPGHHHHHHR | Peptide_1040 | GWWELPGHHHHHHR |
| Peptide 141 | GAATYTGHHHHHHR | Peptide_441 | GTLTYTGHHHHHHR | Peptide_741 | GPWPAWGHHHHHHR | Peptide_1041 | GEWPWLGHHHHHHR |
| Peptide 142 | GELPAPGHHHHHHR | Peptide_442 | GEWPAPGHHHHHHR | Peptide_742 | GWAPWPGHHHHHHR | Peptide_1042 | GWLEWPGHHHHHHR |
| Peptide 143 | GLLPALGHHHHHHR | Peptide_443 | GEAPWPGHHHHHHR | Peptide_743 | GYYTLPGHHHHHHR | Peptide_1043 | GPWELWGHHHHHHR |
| Peptide 144 | GSYTASGHHHHHHR | Peptide_444 | GPWSLPGHHHHHHR | Peptide_744 | GSWTYTGHHHHHHR | Peptide_1044 | GELPWWGHHHHHHR |
| Peptide 145 | GSATYSGHHHHHHR | Peptide_445 | GYYSAPGHHHHHHR | Peptide_745 | GSYTWTGHHHHHHR | Peptide 1045 | GYYEWAGHHHHHHR |
| Peptide 146 | GPADLLGHHHHHHR | Peptide_446 | GSYPAYGHHHHHHR | Peptide 746 | GWLELPGHHHHHHR | Peptide_1046 | GWAEYYGHHHHHHR |
| Peptide 147 | GDADLPGHHHHHHR | Peptide_447 | GYAPYSGHHHHHHR | Peptide_747 | GELPWLGHHHHHHR | Peptide_1047 | GYAEYWGHHHHHHR |
| Peptide 148 | GELTAPGHHHHHHR | Peptide_448 | GSAPYYGHHHHHHR | Peptide_748 | GEYSYPGHHHHHHR | Peptide 1048 | GWYSYLGHHHHHHR |
| Peptide 149 | GEATLPGHHHHHHR | Peptide_449 | GELELPGHHHHHHR | Peptide_749 | GEWDLPGHHHHHHR | Peptide 1049 | GEYEYEGHHHHHHR |
| Peptide 150 | GTLSLPGHHHHHHR | Peptide_450 | GLLELLGHHHHHHR | Peptide_750 | GELPWDGHHHHHHR | Peptide_1050 | GEWDWPGHHHHHHR |

| Number | Sequence | Number | Sequence | Number | Sequence | Number | Sequence |
|---|---|---|---|---|---|---|---|
| Peptide 151 | GPWSAAGHHHHHHR | Peptide_451 | GDWPALGHHHHHHR | Peptide_751 | GLLDLWGHHHHHHR | Peptide_1051 | GWWDLLGHHHHHHR |
| Peptide 152 | GSWPAAGHHHHHHR | Peptide_452 | GDAPWLGHHHHHHR | Peptide_752 | GEYDAYGHHHHHHR | Peptide_1052 | GSWDYYGHHHHHHR |
| Peptide 153 | GWASAPGHHHHHHR | Peptide 453 | GYYDAAGHHHHHHR | Peptide 753 | GEADYYGHHHHHHR | Peptide 1053 | GSYDYWGHHHHHHR |
| Peptide_154 | GPASAWGHHHHHHR | Peptide_454 | GAADYYGHHHHHHR | Peptide_754 | GDAEYYGHHHHHHR | Peptide_1054 | GWYTYTGHHHHHHR |
| Peptide_155 | GAAELEGHHHHHHR | Peptide_455 | GELDLLGHHHHHHR | Peptide 755 | GWWTAPGHHHHHHR | Peptide_1055 | GYYTWTGHHHHHHR |
| Peptide_156 | GELSLAGHHHHHHR | Peptide_456 | GEWTAPGHHHHHHR | Peptide_756 | GTAPWWGHHHHHHR | Peptide_1056 | GYYTWTGHHHHHHR |
| Peptide_157 | GSAELLGHHHHHHR | Peptide_457 | GDWPADGHHHHHHR | Peptide_757 | GYYTLTGHHHHHHR | Peptide_1057 | GEWTLWGHHHHHHR |
| Peptide_158 | GTLDLAGHHHHHHR | Peptide_458 | GEAPWTGHHHHHHR | Peptide_758 | GYYTLTGHHHHHHR | Peptide_1058 | GWLEWTGHHHHHHR |
| Peptide_159 | GDATLLGHHHHHHR | Peptide_459 | GTAPWEGHHHHHHR | Peptide_759 | GEWTLLGHHHHHHR | Peptide_1059 | GWLEWTGHHHHHHR |
| Peptide_160 | GSLSLLGHHHHHHR | Peptide_460 | GALTWLGHHHHHHR | Peptide_760 | GDWDLLGHHHHHHR | Peptide_1060 | GWLEWTGHHHHHHR |
| Peptide_161 | GWADAAGHHHHHHR | Peptide_461 | GYYSATGHHHHHHR | Peptide_761 | GTWELLGHHHHHHR | Peptide_1061 | GEYPYYGHHHHHHR |
| Peptide_162 | GELDASGHHHHHHR | Peptide_462 | GTYSAYGHHHHHHR | Peptide_762 | GELTLWGHHHHHHR | Peptide_1062 | GWWSAWGHHHHHHR |
| Peptide_163 | GDLTADGHHHHHHR | Peptide_463 | GSYTAYGHHHHHHR | Peptide_763 | GLLEWTGHHHHHHR | Peptide_1063 | GEWSWEGHHHHHHR |
| Peptide_164 | GDADLTGHHHHHHR | Peptide_464 | GYATYSGHHHHHHR | Peptide_764 | GTLEWLGHHHHHHR | Peptide_1064 | GSWEWEGHHHHHHR |
| Peptide_165 | GEATLTGHHHHHHR | Peptide_465 | GTLELEGHHHHHHR | Peptide_765 | GWAEWAGHHHHHHR | Peptide_1065 | GWWSYPGHHHHHHR |

EP 4 026 904 A1

67

(continued)

| Number | Sequence | Number | Sequence | Number | Sequence | Number | Sequence |
|---|---|---|---|---|---|---|---|
| Peptide_166 | GSYPAPGHHHHHHR | Peptide_466 | GPYDLPGHHHHHHR | Peptide_766 | GAAEWWGHHHHHHR | Peptide_1066 | GYYDYDGHHHHHHR |
| Peptide_167 | GYAPALGHHHHHHR | Peptide_467 | GEAEWAGHHHHHHR | Peptide_767 | GDYSYDGHHHHHHR | Peptide_1067 | GPWSYWGHHHHHHR |
| Peptide_168 | GTLSLTGHHHHHHR | Peptide_468 | GAAEWEGHHHHHHR | Peptide_768 | GSYDYDGHHHHHHR | Peptide_1068 | GSWPYWGHHHHHHR |
| Peptide_169 | GWASATGHHHHHHR | Peptide_469 | GEWSLAGHHHHHHR | Peptide_769 | GEYSYTGHHHHHHR | Peptide_1069 | GPYSVWWGHHHHHHR |
| Peptide_170 | GSATAWGHHHHHHR | Peptide_470 | GSWELAGHHHHHHR | Peptide_770 | GWWSLAGHHHHHHR | Peptide_1070 | GSYPWWGHHHHHHR |
| Peptide_171 | GDAPAYGHHHHHHR | Peptide_471 | GWASLEGHHHHHHR | Peptide_771 | GEAEWEGHHHHHHR | Peptide_1071 | GEWEYLGHHHHHHR |
| Peptide_172 | GPADAYGHHHHHHR | Peptide_472 | GELSWAGHHHHHHR | Peptide_772 | GELSWEGHHHHHHR | Peptide_1072 | GEYEWLGHHHHHHR |
| Peptide_173 | GSWSASGHHHHHHR | Peptide_473 | GSAELWGHHHHHHR | Peptide_773 | GEWTLDGHHHHHHR | Peptide_1073 | GELEYWGHHHHHHR |
| Peptide_174 | GSYTAPGHHHHHHR | Peptide_474 | GDWTLAGHHHHHHR | Peptide_774 | GDASWWGHHHHHHR | Peptide_1074 | GWWDAYGHHHHHHR |
| Peptide_175 | GTAPYSGHHHHHHR | Peptide_475 | GTADWLGHHHHHHR | Peptide_775 | GSADWWGHHHHHHR | Peptide_1075 | GWADYWGHHHHHHR |
| Peptide_176 | GSATYPGHHHHHHR | Peptide_476 | GSYSYSGHHHHHHR | Peptide_776 | GWWTATGHHHHHHR | Peptide_1076 | GYADWWGHHHHHHR |
| Peptide_177 | GYATLAGHHHHHHR | Peptide_477 | GPYTLLGHHHHHHR | Peptide_777 | GWATWTGHHHHHHR | Peptide_1077 | GEWDYEGHHHHHHR |
| Peptide_178 | GAATYLGHHHHHHR | Peptide_478 | GLLTYPGHHHHHHR | Peptide_778 | GTATWWGHHHHHHR | Peptide_1078 | GDWEYEGHHHHHHR |
| Peptide_179 | GEYSAAGHHHHHHR | Peptide_479 | GDWSAEGHHHHHHR | Peptide_779 | GEWPAYGHHHHHHR | Peptide_1079 | GEYEWDGHHHHHHR |
| Peptide_180 | GSAEYAGHHHHHHR | Peptide_480 | GSADWEGHHHHHHR | Peptide_780 | GEYPWAGHHHHHHR | Peptide_1080 | GYYPWLGHHHHHHR |

68

(continued)

| Number | Sequence | Number | Sequence | Number | Sequence | Number | Sequence |
|---|---|---|---|---|---|---|---|
| Peptide_181 | GDATAYGHHHHHHR | Peptide_481 | GEWTATGHHHHHHR | Peptide_781 | GYAPWEGHHHHHHR | Peptide_1081 | GWWSYTGHHHHHHR |
| Peptide_182 | GSYSLAGHHHHHHR | Peptide_482 | GTWDADGHHHHHHR | Peptide_782 | GEAPYWGHHHHHHR | Peptide_1082 | GSYTWWGHHHHHHR |
| Peptide_183 | GYASLSGHHHHHHR | Peptide_483 | GDADWTGHHHHHHR | Peptide_783 | GWYSLPGHHHHHHR | Peptide_1083 | GDWPYYGHHHHHHR |
| Peptide_184 | GSLSAYGHHHHHHR | Peptide_484 | GTADWDGHHHHHHR | Peptide_784 | GWLSYPGHHHHHHR | Peptide_1084 | GPWDYYGHHHHHHR |
| Peptide_185 | GPWPAAGHHHHHHR | Peptide_485 | GSLSWDGHHHHHHR | Peptide_785 | GPWSLYGHHHHHHR | Peptide_1085 | GWWPAWGHHHHHHR |
| Peptide_186 | GWAPAPGHHHHHHR | Peptide_486 | GSWTLTGHHHHHHR | Peptide_786 | GSYPWLGHHHHHHR | Peptide_1086 | GWYTLYGHHHHHHR |
| Peptide_187 | GAAPWPGHHHHHHR | Peptide_487 | GWYPAAGHHHHHHR | Peptide_787 | GPLSWYGHHHHHHR | Peptide_1087 | GWLELWGHHHHHHR |
| Peptide_188 | GDASYSGHHHHHHR | Peptide_488 | GAAPWYGHHHHHHR | Peptide_788 | GYYSAYGHHHHHHR | Peptide_1088 | GEYSWYGHHHHHHR |
| Peptide_189 | GTYTASGHHHHHHR | Peptide_489 | GEYSLPGHHHHHHR | Peptide_789 | GYLELEGHHHHHHR | Peptide_1089 | GSYEYWGHHHHHHR |
| Peptide_190 | GSYTATGHHHHHHR | Peptide_490 | GSYELPGHHHHHHR | Peptide_790 | GELELYGTHHHHHHR | Peptide_1090 | GYYTWDGHHHHHHR |
| Peptide_191 | GTASYTGHHHHHHR | Peptide_491 | GELPYSGHHHHHHR | Peptide_791 | GSYPWDGHHHHHHR | Peptide_1091 | GDYTYWGHHHHHHR |
| Peptide_192 | GSATYTGHHHHHHR | Peptide_492 | GDYTLPGHHHHHHR | Peptide_792 | GTWTYPGHHHHHHR | Peptide_1092 | GTYDWYGHHHHHHR |
| Peptide_193 | GELPALGHHHHHHR | Peptide_493 | GTLPYDGHHHHHHR | Peptide_793 | GTYTWPGHHHHHHR | Peptide_1093 | GEWDLWGHHHHHHR |
| Peptide_194 | GLLPAEGHHHHHHR | Peptide_494 | GYAELLGHHHHHHR | Peptide_794 | GWYDALGHHHHHHR | Peptide_1094 | GELDWWGHHHHHHR |
| Peptide_195 | GPAELLGHHHHHHR | Peptide_495 | GALELYGHHHHHHR | Peptide_795 | GWADLYGHHHHHHR | Peptide_1095 | GWYPWPGHHHHHHR |

EP 4 026 904 A1

69

(continued)

| Number | Sequence | Number | Sequence | Number | Sequence | Number | Sequence |
|---|---|---|---|---|---|---|---|
| Peptide_196 | GPLDAEGHHHHHHR | Peptide_496 | GEYDALGHHHHHHR | Peptide_796 | GYADWLGHHHHHHR | Peptide_1096 | GPWPYWGHHHHHHR |
| Peptide_197 | GEADLPGHHHHHHR | Peptide_497 | GELDYAGHHHHHHR | Peptide_797 | GELDYEGHHHHHHR | Peptide_1097 | GPYPWWGHHHHHHR |
| Peptide_198 | GDAELPGHHHHHHR | Peptide_498 | GEADLYGHHHHHHR | Peptide_798 | GDLEYEGHHHHHHR | Peptide_1098 | GWATWWGHHHHHHR |
| Peptide_199 | GTLTLPGHHHHHHR | Peptide_499 | GYYPAPGHHHHHHR | Peptide_799 | GWADYDGHHHHHHR | Peptide_1099 | GEWTWEGHHHHHHR |
| Peptide_200 | GWATAPGHHHHHHR | Peptide_500 | GSYDLLGHHHHHHR | Peptide_800 | GDADYWGHHHHHHR | Peptide_1100 | GDWEWDGHHHHHHR |
| Peptide_201 | GAAPWTGHHHHHHR | Peptide_501 | GDLSLYGHHHHHHR | Peptide_801 | GEWTYAGHHHHHHR | Peptide_1101 | GTWEWEGHHHHHHR |
| Peptide_202 | GELTLAGHHHHHHR | Peptide_502 | GTLTLYGHHHHHHR | Peptide_802 | GEYTAWGHHHHHHR | Peptide_1102 | GYYELYGHHHHHHR |
| Peptide_203 | GDLDLAGHHHHHHR | Peptide_503 | GWATYAGHHHHHHR | Peptide_803 | GYAEWTGHHHHHHR | Peptide_1103 | GYLEYYGHHHHHHR |
| Peptide_204 | GEATLLGHHHHHHR | Peptide_504 | GYATAWGHHHHHHR | Peptide_804 | GTAEWYGHHHHHHR | Peptide_1104 | GSWSWWGHHHHHHR |
| Peptide_205 | GTLSLLGHHHHHHR | Peptide_505 | GAATYWGHHHHHHR | Peptide_805 | GWYSLTGHHHHHHR | Peptide_1105 | GEYDYYGHHHHHHR |
| Peptide_206 | GAAEWAGHHHHHHR | Peptide_506 | GEADYDGHHHHHHR | Peptide_806 | GSWTYLGHHHHHHR | Peptide_1106 | GWYPWTGHHHHHHR |
| Peptide_207 | GPWSASGHHHHHHR | Peptide_507 | GDADYEGHHHHHHR | Peptide_807 | GTYSLWGHHHHHHR | Peptide_1107 | GWWEYAGHHHHHHR |
| Peptide_208 | GWLSAAGHHHHHHR | Peptide_508 | GTAEYEGHHHHHHR | Peptide_808 | GSYTWLGHHHHHHR | Peptide_1108 | GWAEYWGHHHHHHR |
| Peptide_209 | GWASLAGHHHHHHR | Peptide_509 | GEYSLTGHHHHHHR | Peptide_809 | GSLTWYGHHHHHHR | Peptide_1109 | GYAEWWGHHHHHHR |
| Peptide_210 | GALSAWGHHHHHHR | Peptide_510 | GTYSLEGHHHHHHR | Peptide_810 | GPLDYYGHHHHHHR | Peptide_1110 | GWYSWLGHHHHHHR |

| Number | Sequence | Number | Sequence | Number | Sequence | Number | Sequence |
|---|---|---|---|---|---|---|---|
| Peptide_211 | GSAELEGHHHHHHK | Peptide_511 | GELSYTGHHHHHHR | Peptide_811 | GEWSYSGHHHHHHR | Peptide_1111 | GWLSWYGHHHHHHR |
| Peptide_212 | GDLDADGHHHHHHR | Peptide_512 | GWYSASGHHHHHHR | Peptide_812 | GSYEWSGHHHHHHR | Peptide_1112 | GEWEYEGHHHHHHK |
| Peptide_213 | GTYPAPGHHHHHHR | Peptide_513 | GSWSYAGHHHHHHR | Peptide_813 | GDWTYSGHHHHHHR | Peptide_1113 | GWWSYDGHHHHHHR |
| Peptide_214 | GELSLSGHHHHHHR | Peptide_514 | GWASYSGHHHHHHR | Peptide_814 | GTYDWSGHHHHHHR | Peptide_1114 | GWYSWDGHHHHHHR |
| Peptide_215 | GSLELSGHHHHHHR | Peptide_515 | GSYSWAGHHHHHHR | Peptide_815 | GDYPYDGHHHHHHR | Peptide_1115 | GSYDWWGHHHHHHR |
| Peptide_216 | GTLTLTGHHHHHHR | Peptide_516 | GSASYWGHHHHHHR | Peptide_816 | GSWPWPGHHHHHHR | Peptide_1116 | CTYTWWGHHHHHHR |
| Peptide_217 | GSWDAAGHHHHHHR | Peptide_517 | GTLPWPGHHHHHHR | Peptide_817 | GWWPALGHHHHHHR | Peptide_1117 | GEWPYYGHHHHHHR |
| Peptide_218 | GWASADGHHHHHHR | Peptide_518 | GYYTAPGHHHHHHR | Peptide_818 | GWAPWLGHHHHHHR | Peptide_1118 | GEYPWYGHHHHHHR |
| Peptide_219 | GTWTAAGHHHHHHR | Peptide_519 | GYAPYTGHHHHHHR | Peptide_819 | GYYTLLGHHHHHHR | Peptide_1119 | GWLDYYGHHHHHHR |
| Peptide_220 | GTATAWGHHHHHHR | Peptide_520 | GTAPYYGHHHHHHR | Peptide_820 | GEWELPGHHHHHHR | Peptide_1120 | GWYTYEGHHHHHHR |
| Peptide_221 | GAATWTGHHHHHHR | Peptide_521 | GEWPALGHHHHHHR | Peptide_821 | GPWELEGHHHHHHR | Peptide_1121 | GYYDWDGHHHHHHR |
| Peptide_222 | GEYPAAGHHHHHHR | Peptide_522 | GELPAWGHHHHHHR | Peptide_822 | GELPWEGHHHHHHR | Peptide_1122 | GEYTYWGHHHHHHR |
| Peptide_223 | GEAPAYGHHHHHHR | Peptide_523 | GEAPWLGHHHHHHR | Peptide_823 | GEYEYAGHHHHHHR | Peptide_1123 | GTWEYYGHHHHHHR |
| Peptide_224 | GPAEYAGHHHHHHR | Peptide_524 | GPLSLWGHHHHHHR | Peptide_824 | GWWDAPGHHHHHHR | Peptide_1124 | GPWSWWGHHHHHHR |
| Peptide_225 | GYLSAPGHHHHHHR | Peptide_525 | GYAEYAGHHHHHHR | Peptide_825 | GWAPWDGHHHHHHR | Peptide_1125 | GDYDYWGHHHHHHR |

(continued)

| Number | Sequence | Number | Sequence | Number | Sequence | Number | Sequence |
|---|---|---|---|---|---|---|---|
| Peptide_226 | GSYPALGHHHHHHR | Peptide_526 | GAAEYYGHHHHHHR | Peptide_826 | GDAPWWGHHHHHHR | Peptide_1126 | GEWELWGHHHHHHR |
| Peptide_227 | GYASLPGHHHHHHR | Peptide_527 | GSYPYSGHHHHHHR | Peptide_827 | GYYSLEGHHHHHHR | Peptide_1127 | GWLEWEGHHHHHHR |
| Peptide_228 | GPLSAYGHHHHHHR | Peptide_528 | GYYSLAGHHHHHHR | Peptide_828 | GSYEYLGHHHHHHR | Peptide_1128 | GELEWWGHHHHHHR |
| Peptide_229 | GALSYPGHHHHHHR | Peptide_529 | GEWDAPGHHHHHHR | Peptide_829 | GSLEYYGHHHHHHR | Peptide_1129 | GEWDWEGHHHHHHR |
| Peptide_230 | GSWTASGHHHHHHR | Peptide_530 | GEAPWDGHHHHHHR | Peptide_830 | GTYDYLGHHHHHHR | Peptide_1130 | GWYPWLGHHHHHHR |
| Peptide_231 | GSYPADGHHHHHHR | Peptide_531 | GDAEWPGHHHHHHR | Peptide_831 | GEWDLLGHHHHHHR | Peptide_1131 | GWLPYWGHHHHHHR |
| Peptide_232 | GPASYDGHHHHHHR | Peptide_532 | GPAEWDGHHHHHHR | Peptide_832 | GDWELLGHHHHHHR | Peptide_1132 | GYLPWWGHHHHHHR |
| Peptide_233 | GSADYPGHHHHHHR | Peptide_533 | GDWSLPGHHHHHHR | Peptide_833 | GELDLWGHHHHHHR | Peptide_1133 | GSWTWWGHHHHHHR |
| Peptide_234 | GTYTAPGHHHHHHR | Peptide_534 | GSWDLPGHHHHHHR | Peptide_834 | GPLPYWGHHHHHHR | Peptide_1134 | GYYEYEGHHHHHHR |
| Peptide_235 | GDADAYGHHHHHHR | Peptide_535 | GTLTWPCTHHHHHHR | Peptide_835 | GEYSYDGHHHHHHR | Peptide_1135 | GWWDYPGHHHHHHR |
| Peptide_236 | GAADYDGHHHHHHR | Peptide_536 | GWADLLGHHHHHHR | Peptide_836 | GSYEYDGHHHHHHR | Peptide_1136 | GWYDWPGHHHHHHR |
| Peptide_237 | GSYTLAGHHHHHHR | Peptide_537 | GSYDAYGHHHHHHR | Peptide_837 | GTYDYDGHHHHHHR | Peptide_1137 | GPYDWWGHHHHHHR |
| Peptide_238 | GYASLTGHHHHHHR | Peptide_538 | GSADYYGHHHHHHR | Peptide_838 | GWWTLAGHHHHHHR | Peptide_1138 | GYYPYYGHHHHHHR |
| Peptide_239 | GALTYSGHHHHHHR | Peptide_539 | GYYTATGHHHHHHR | Peptide_839 | GWATWLGHHHHHHR | Peptide_1139 | GWYSWEGHHHHHHR |
| Peptide_240 | GSATYLGHHHHHHR | Peptide_540 | GTYTAYGHHHHHHR | Peptide_840 | GYYPAYGHHHHHHR | Peptide_1140 | GEWSWYGHHHHHHR |

(continued)

| Number | Sequence | Number | Sequence | Number | Sequence | Number | Sequence |
|---|---|---|---|---|---|---|---|
| Peptide_241 | GEYSASGHHHHHHR | Peptide_541 | GYATYTGHHHHHHR | Peptide_841 | GYAPYYGHHHHHHR | Peptide_1141 | GEYSWWGHHHHHHR |
| Peptide_242 | GEASYSGHHHHHHR | Peptide_542 | GPYELPGHHHHHHR | Peptide_842 | GELDWDGHHHHHHR | Peptide_1142 | GSWEYWGHHHHHHR |
| Peptide_243 | GTYSADGHHHHHHR | Peptide_543 | GEWTALGHHHHHHR | Peptide_843 | GDLDWEGHHHHHHR | Peptide_1143 | GSYEWWGHHHHHHR |
| Peptide_244 | GSYDATGHHHHHHR | Peptide_544 | GDWDALGHHHHHHR | Peptide_844 | GTLEWEGHHHHHHR | Peptide_1144 | GTWDYWGHHHHHHR |
| Peptide_245 | GTATYTGHHHHHHR | Peptide_545 | GTWELAGHHHHHHR | Peptide_845 | GWWSAEGHHHHHHR | Peptide_1145 | GWWPWPGHHHHHHR |
| Peptide_246 | GPLTLLGHHHHHHR | Peptide_546 | GELTWAGHHHHHHR | Peptide_846 | GEWSAWGHHHHHHR | Peptide_1146 | GPWPWWGHHHHHHR |
| Peptide_247 | GPWPASGHHHHHHR | Peptide_547 | GTLEWAGHHHHHHR | Peptide_847 | GSWEWAGHHHHHHR | Peptide_1147 | GYYEWLGHHHHHHR |
| Peptide_248 | GSAPWPGHHHHHHR | Peptide_548 | GEATWLGHHHHHHR | Peptide_848 | GWASWEGHHHHHHR | Peptide_1148 | GWLEYYGHHHHHHR |
| Peptide_249 | GWLPAAGHHHHHHR | Peptide_549 | GDADWLGHHHHHHR | Peptide_849 | GEASWWGHHHHHHR | Peptide_1149 | GWYDYEGHHHHHHR |
| Peptide_250 | GAAPWLGHHHHHHR | Peptide_550 | GTAEWLGHHHHHHR | Peptide_850 | GSAEWWGHHHHHHR | Peptide_1150 | GEWDYYGHHHHHHR |
| Peptide_251 | GELPAEGHHHHHHR | Peptide_551 | GSWTLLGHHHHHHR | Peptide_851 | GDATWWGHHHHHHR | Peptide_1151 | GWWEWACTHHHHHHR |
| Peptide_252 | GEAELPGHH H H H HR | Peptide_552 | GWWSAAGHHHHHHR | Peptide_852 | GTADWWGHHHHHHR | Peptide_1152 | GEWEWEGHHHHHHR |
| Peptide_253 | GTLDLPGHHHHHHR | Peptide_553 | GWASAWGHHHHHHR | Peptide_853 | GWYTLPGHHHHHHR | Peptide_1153 | GDWSWWGHHHHHHR |
| Peptide_254 | GLLSLLGHHHHHHR | Peptide_554 | GAASWWGHHHHHHR | Peptide_854 | GTYPWLGHHHHHHR | Peptide_1154 | GSWDWWGHHHHHHR |
| Peptide_255 | GWADAPGHHHHHHR | Peptide_555 | GSYSYTGHHHHHHR | Peptide_855 | GYYTAYCHHHHHHR | Peptide_1155 | GWWTWTGHHHHHHR |

73

| Number | Sequence | Number | Sequence | Number | Sequence | Number | Sequence |
|---|---|---|---|---|---|---|---|
| Peptide_256 | GAADWPGHHHHHHR | Peptide_556 | GPLDLYGHHHHHHR | Peptide_856 | GEWPYSGHHHHHHR | Peptide_1156 | GWYEWPGHHHHHHR |
| Peptide_257 | GELDLAGHHHHHHR | Peptide_557 | GEAEWSGHHHHHHR | Peptide_857 | GSYEWPGHHHHHHR | Peptide_1157 | GEWPWYGHHHHHHR |
| Peptide_258 | GDAELLGHHHHHHR | Peptide_558 | GSAEWEGHHHHHHR | Peptide_858 | GDWTYPGHHHHHHR | Peptide_1158 | GEYPWWGHHHHHHR |
| Peptide_259 | GYLPAPGHHHHHHR | Peptide_559 | GDADWDGHHHHHHR | Peptide_859 | GWAELYGHHHHHHR | Peptide_1159 | GPYEWWGHHHHHHR |
| Peptide_260 | GPYPALGHHHHHHR | Peptide_560 | GEWSLSGHHHHHHR | Peptide_860 | GYAEWLGHHHHHHR | Peptide_1160 | GYYSYWCTHHHHHHk |
| Peptide_261 | GSLDLLGHHHHHHR | Peptide_561 | GDWTLSGHHHHHHR | Peptide_861 | GELEYEGHHHHHHR | Peptide_1161 | GWWDLYGHHHHHHR |
| Peptide_262 | GSAPWTGHHHHHHR | Peptide_562 | GTLSWDGHHHHHHR | Peptide_862 | GYYSYSGHHHHHHR | Peptide_1162 | GWLDYWGHHHHHHR |
| Peptide_263 | GWATALGHHHHHHR | Peptide_563 | GTWTLTGHHHHHHR | Peptide_863 | GPWPWPGHHHHHHR | Peptide_1163 | GWWTYEGHHHHHHR |
| Peptide_264 | GALTAWUHHHHHHR | Peptide_564 | GDLDYPGTHHHHHHR | Peptide_864 | GEWDYAGHHHHHHR | Peptide_1164 | GWYEWTGHHHHHHR |
| Peptide_265 | GAATWLGHHHHHHR | Peptide_565 | GWYSAPGHHHHHHR | Peptide_865 | GWADYEGHHHHHHR | Peptide_1165 | GEWIWYGHHHHHHR |
| Peptide_266 | GELTAEGHHHHHHR | Peptide_566 | GPWSYAGHHHHHHR | Peptide_866 | GEADWYGHHHHHHR | Peptide_1166 | GEYTWWGHHHHHHR |
| Peptide_267 | GTAELEGHHHHH HR | Peptide_567 | GSAPYWGHHHHHHR | Peptide_867 | GDAhWYGHHHHHHR | Peptide_1167 | GWWPWLGHH H H H H k |
| Peptide_268 | GTLSLEGHHHHHHR | Peptide_568 | GELEYAGHHHHHHR | Peptide_868 | GDWSYLGHHHHHHR | Peptide_1168 | GWLPWWGHHHHHHR |
| Peptide_269 | GDLSLDGHHHHHHR | Peptide_569 | GYAELEGHHHHHHR | Peptide_869 | GDYSLWGHHHHHHR | Peptide_1169 | GWYEYEGHHHHHHR |
| Peptide_270 | GEWSAAGHHHHHHR | Peptide_570 | GEAEYLGHHHHHHR | Peptide_870 | GSWDYLGHHHHHHR | Peptide_1170 | GYYEWEGHHHHHHR |

EP 4 026 904 A1

74

| Number | Sequence | Number | Sequence | Number | Sequence | Number | Sequence |
|---|---|---|---|---|---|---|---|
| Peptide_271 | GEASAWGHHHHHHR | Peptide_571 | GEYSLLGHHHHHHR | Peptide_871 | GSYDWLGHHHHHHR | Peptide_1171 | GEWEYYGHHHHHHR |
| Peptide_272 | GSAEWAGHHHHHHR | Peptide_572 | GSYELLGHHHHHHR | Peptide_872 | GDLSWYUHHHHHHR | Peptide_1172 | GEYEYWGHHHHHHR |
| Peptide_273 | GAASWEGHHHHHHR | Peptide_573 | GTYDLLGHHHHHHR | Peptide_873 | GWYTLTGHHHHHHR | Peptide_1173 | GPWDWWGHHHHHHR |
| Peptide_274 | GWLSASGHHHHHHR | Peptide_574 | GWYDAAGHHHHHHR | Peptide_874 | GTLTYWGTHHHHHHR | Peptide_1174 | GWWTLWGHHHHHHR |
| Peptide_275 | GSWSALGHHHHHHR | Peptide_575 | GWADYAGHHHHHHR | Peptide_875 | GYYELPGHHHHHHR | Peptide_1175 | GWLTWWGHHHHHHR |
| Peptide_276 | GTLPAYGHHHHHHR | Peptide_576 | GAADWYGHHHHHHR | Peptide_876 | GELPYYGHHHHHHR | Peptide_1176 | GWYPYYGHHHHHHR |
| Peptide_277 | GSASWDGHHHHHHR | Peptide_577 | GLLPWPGHHHHHHR | Peptide_877 | GPLEYYGHHHHHHR | Peptide_1177 | GYYPYWGHHHHHHR |
| Peptide_278 | GTWTASGHHHHHHR | Peptide_578 | GPLPWLGHHHHHHR | Peptide_878 | GEWSYTGHHHHHHR | Peptide_1178 | GWWSWEGHHHHHHR |
| Peptide_279 | GSWTATGHHHHHHR | Peptide_579 | GDAEYEGHHHHHHR | Peptide_879 | GDWDYSGHHHHHHR | Peptide_1179 | GEWSWWGHHHHHHR |
| Peptide_280 | GEYSAPGHHHHHHR | Peptide_580 | GELDYSGHHHHHHR | Peptide_880 | GDYDWSGHHHHHHR | Peptide_1180 | GYYTYWGHHHHHHR |
| Peptide_281 | GDYTAPGHHHHHHR | Peptide_581 | GSLDYEGHHHHHHR | Peptide_881 | GSYDWDGHHHHHHR | Peptide_1181 | GWYEWLGHHHHHHR |
| Peptide_282 | GTYPADGHHHHHHR | Peptide_582 | GTLTYEGHHHHHHR | Peptide_882 | GEYPYDGHHHHHHR | Peptide_1182 | GYLEWWGHHHHHHR |
| Peptide_283 | GTADYPGHHHHHHR | Peptide_583 | GDYDLTGHHHHHHR | Peptide_883 | GYYDLLGHHHHHHR | Peptide_1183 | GWWDYEGHHHHHHR |
| Peptide_284 | GAAEYLGHHHHHHR | Peptide_584 | GPYSYPGHHHHHHR | Peptide_884 | GPWEWAGHHHHHHR | Peptide_1184 | GDWEYWGHHHHHHR |
| Peptide_285 | GSYSLPGHHHHHHR | Peptide_585 | GYYPALGHHHHHHR | Peptide_885 | GEAPWWGHHHHHHR | Peptide_1185 | GEYDWWGHHHHHHR |

| Number | Sequence | Number | Sequence | Number | Sequence | Number | Sequence |
|---|---|---|---|---|---|---|---|
| Peptide_286 | GPLSYSGHHHHHHR | Peptide_586 | GYLPAYGHHHHHHR | Peptide 886 | GWWSLPGHHHHHHR | Peptide_1186 | GEWPWWGHHHHHHR |
| Peptide_287 | GSLSYPGHHHHHHR | Peptide_587 | GALPYYGHHHHHHR | Peptide_887 | GEYTLYGHHHHHHR | Peptide_1187 | GPWEWWGHHHHHHR |
| Peptide_288 | GLLSYAGHHHHHHR | Peptide_588 | GWYSATGHHHHHHR | Peptide_888 | GYLEYTGHHHHHHR | Peptide_1188 | GYYSWWGHHHHHHR |
| Peptide_289 | GEYDAAGHHHHHHR | Peptide_589 | GSWTYAGHHHHHHR | Peptide_889 | GSWPWLGHHHHHHR | Peptide_1189 | GWWTWEGHHHHHHR |
| Peptide_290 | GYADAEGHHHHHHR | Peptide_590 | GTYSAWGHHHHHHR | Peptide_890 | GPLSWWGHHHHHHR | Peptide_1190 | GEWTWWGHHHHHHR |
| Peptide_291 | GAADYEGHHHHHHR | Peptide_591 | GWASYTGHHHHHHR | Peptide_891 | GWYSAYGHHHHHHR | Peptide_1191 | GDWDWWGHHHHHHR |
| Peptide_292 | GSYDLAGHHHHHHR | Peptide_592 | GSYTAWGHHHHHHR | Peptide_892 | GEWELLGHHHHHHR | Peptide_1192 | GYYDYWGHHHHHHR |
| Peptide_293 | GDASYLGHHHHHHR | Peptide_593 | GTASYWGHHHHHHR | Peptide_893 | GELELWGHHHHHHR | Peptide_1193 | GWWEYEGHHHHHHR |
| Peptide_294 | GSADLYGHHHHHHR | Peptide_594 | GSATYWGHHHHHHR | Peptide 894 | GLLEWEGHHHHHHR | Peptide_1194 | GWYEWEGHHHHHHR |
| Peptide_295 | GTYTLAGHHHHHHR | Peptide_595 | GPLPWDGHHHHHHR | Peptide_895 | GSYEYEGHHHHHHR | Peptide_1195 | GEWEYWGHHHHHHR |
| Peptide_296 | GTLTAYGHHHHHHR | Peptide_596 | GYYDAPGHHHHHHR | Peptide_896 | GSWDWPGHHHHHHR | Peptide_1196 | GEYEWWGHHHHHHR |
| Peptide_297 | GALTYTGHHHHHHR | Peptide_597 | GSWSYSGHHHHHHR | Peptide_897 | GTWTWPGHHHHHHR | Peptide_1197 | GEWDWWGHHHHHHR |
| Peptide_298 | GTATYLGHHHHHHR | Peptide_598 | GWLTLPGHHHHHHR | Peptide_898 | GWWDLAGHHHHHHR | Peptide_1198 | GDWEWWGHHHHHHR |
| Peptide_299 | GTYSAEGHHHHHHR | Peptide_599 | GPWTLLGHHHHHHR | Peptide_899 | GDWELECTHHHHHHR | Peptide 1199 | GWYEYYGHHHHHHR |
| Peptide_300 | GSADYDGHHHHHHR | Peptide 600 | GLLPWTGHHHHHHR | Peptide 900 | GELDWEGHHHHHHR | Peptide 1200 | GYYEYWGHHHHHHR |

EP 4 026 904 A1

76

**10. Screening of signal peptides capable of increasing aflibercept production using aflibercept target library**

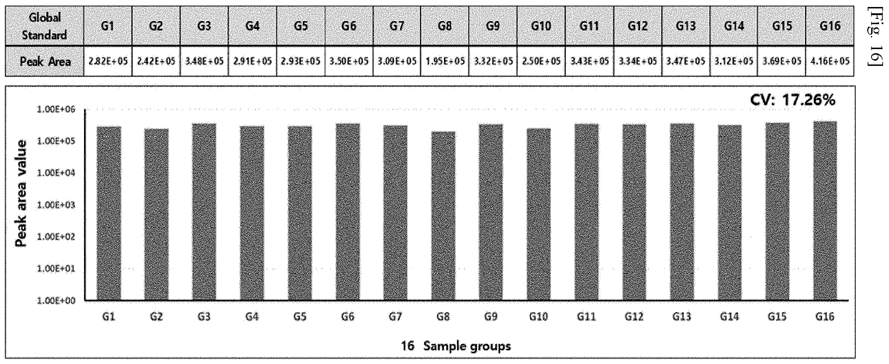

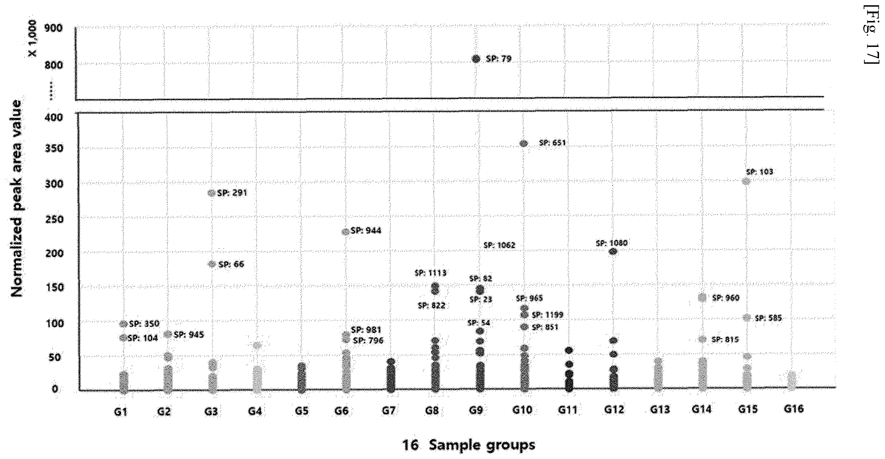

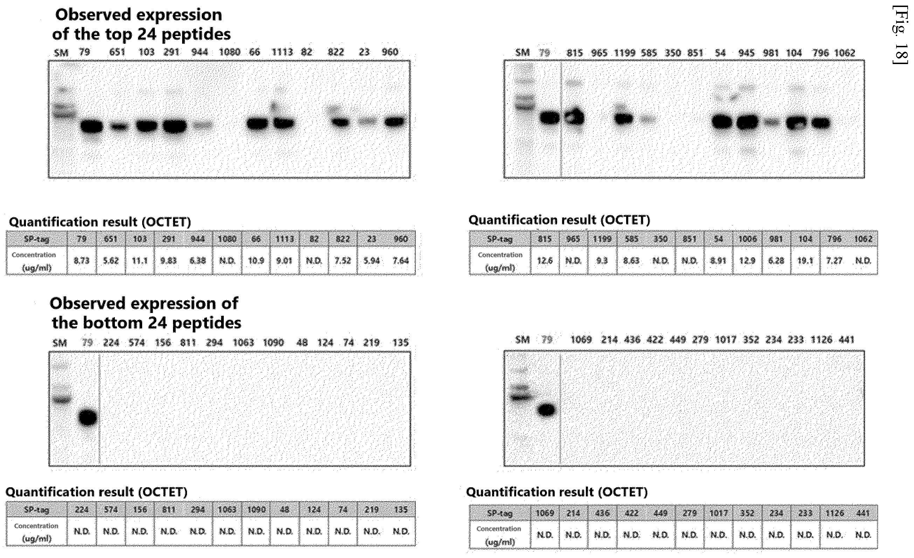

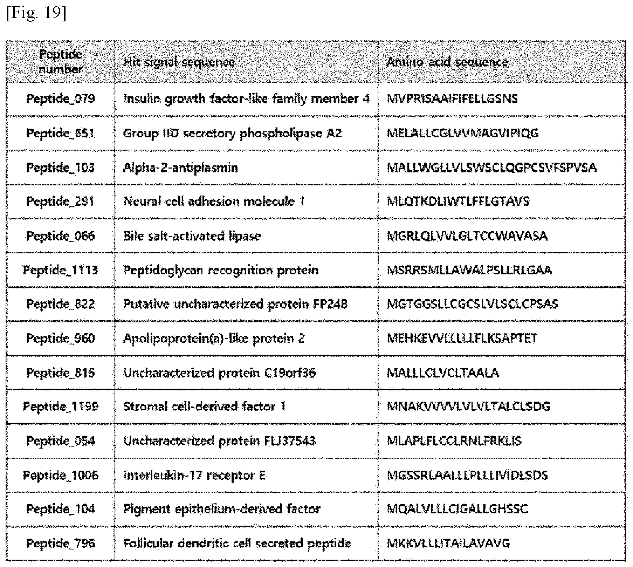

Aflibercept as a model target protein was cloned into the signal peptide/SP-tag library to construct a target protein library. Three days after expression of the library in HEK 293 cells, the cell culture media were collected and the expressed protein was isolated and purified using His tags. After treatment of the protein samples with trypsin, the peak area values were calculated by LC-MS/MS to determine the relative amounts of the SP-tags. To effectively analyze 1,200 barcoding peptides designed for signal peptide screening and 1,200 synthesized internal standards (a total of 2,400 peptides), the peptides were divided into a total of 16 groups, 150 target peptides per group, and MRM analysis was performed. Each group (150 target peptides) consisted of 75 barcoding peptides and 75 standard peptides. The global standard peptide was analyzed together for normalization to minimize differences between the 16 groups (Fig. 16). The system was found to be stable for the 16 groups, with a coefficient of variation (CV) of 17.26% (Fig. 16). The 16 groups (2,400 barcoding and standard peptides) were analyzed by LC-MS/MS. The peak area values were corrected with the global standard and are graphically shown in Fig. 17. The high values were selected as primary hits. To validate the screening system and select secondary hits, plasmids corresponding to the top 24 peptides and the bottom 24 peptides were individually expressed and their expression levels were compared by Western blot (Fig. 18). For quantitative analysis, the individually expressed samples were quantified using an OCTET system and compared in duplicate (Fig. 18). Based on these results, 14 signal peptides matched to the 14 SP-tags with high expression levels were selected (Fig. 19).

Although the particulars of the present invention have been described in detail, it will be obvious to those skilled in the art that such particulars are merely preferred embodiments and are not intended to limit the scope of the present invention. Therefore, the substantial scope of the present invention is defined by the appended claims and their equivalents.

**Claims**

1. A composition for screening various signal peptides to select specific ones that allow efficient secretion of a target protein to out of host cells, comprising polypeptides comprising signal peptide tags (SP-tags) or nucleic acid molecules encoding the polypeptides wherein each of the signal peptide tags comprises a barcoding sequence site consisting of three or more amino acids barcoding the corresponding signal peptide and the three or more amino acids are selected from the group consisting of leucine (L), proline (P), alanine (A), tryptophan (W), tyrosine (Y), threonine (T), serine (S), glutamate (E), and aspartate (D).

2. The composition according to claim 1, wherein each of the signal peptide tags further comprises a proteolytic cleavage site at the N-terminus (front end) of the barcoding sequence such that the barcoding sequence is cleaved by the protein.

3. The composition according to claim 2, wherein the proteolytic cleavage site is selected from the group consisting of trypsin cleavage sites, thrombin cleavage sites, enterokinase cleavage sites, Factor Xa cleavage sites, collagenase cleavage sites, and TEV protease cleavage sites.

4. The composition according to claim 3, wherein the proteolytic cleavage site is a trypsin cleavage site.

5. The composition according to claim 4, wherein the proteolytic cleavage site is selected from the group consisting of lysine (K) and arginine (R).

6. The composition according to claim 3, wherein the proteolytic cleavage site is linked to the barcoding sequence via a linker.

7. The composition according to claim 6, wherein the linker comprises one or more glycine (G) residues.

8. The composition according to claim 1, wherein each of the polypeptides comprising signal peptide tags further comprises an affinity tag at the end of the barcoding sequence to isolate and purify the barcoding sequence from culture media of the host cells.

9. The composition according to claim 8, wherein the affinity tag is selected from the group consisting of histidine tag (His-tag), myc-tag, FLAG-tag, small ubiquitin-like modifier tag (SUMO-tag), covalent yet dissociable NorpD peptide tag (CYD-tag), heavy chain of protein C tag (HPC-tag), calmodulin binding peptide tag (CBP-tag), and hemagglutinin-tag (HA-tag).

10. The composition according to claim 9, wherein the affinity tag is a His-tag composed of 2 to 15 histidine residues.

11. The composition according to claim 1, wherein each of the signal peptide tags is independently represented by Structure 1:
    <Structure 1> Trypsin cleavage site-linker-barcoding sequence-linker-affinity tag-trypsin cleavage site

**12.** The composition according to claim 11, wherein each of the signal peptide tags is independently represented by Structure 2:

<Structure 2> Lysine (K) or arginine (R)-glycine (G)-barcoding sequence-glycine (G)-affinity tag-lysine (K) or arginine (R)

**13.** The composition according to claim 1, wherein the target protein is fused to the N-termini of the signal peptide tags of the polypeptides.

**14.** The composition according to claim 13, wherein the signal peptides are fused to the N-terminus of the target protein.

**15.** A vector comprising each of the nucleic acid molecules encoding the polypeptides comprising signal peptide tags present in the composition according to any one of claims 1 to 14.

**16.** A method for selecting specific signal peptides that express a target protein in host cells and secrete the target protein to out of the host cells, the method comprising:

1) constructing vectors according to claim 15 for various signal peptides to establish a library;
2) transforming host cells with the vectors;
3) expressing the polypeptides comprising signal peptide tags from the transformed host cells; and
4) quantifying the polypeptides comprising signal peptide tags, the signal peptide tags or barcoding sequences of the signal peptide tags secreted to out of the transformed host cells.

**17.** The method according to claim 16, wherein the host cells are selected from the group consisting of CHO cells, HeLa cells, HEK293 cells, BHK cells, COS7 cells, COP5 cells, A549 cells, NIH3T3 cells, MDCK cells, and WI38 cells.

**18.** The method according to claim 16, wherein step 3) further comprises 3-1) isolating and purifying the polypeptides comprising signal peptide tags using affinity tags after expression.

**19.** The method according to claim 16, wherein step 3) further comprises 3-2) treating the polypeptides comprising signal peptide tags with trypsin.

**20.** The method according to claim 16, wherein in step 4), the quantification is performed by LC-MS/MS.

[Fig. 1]

EP 4 026 904 A1

Translated protein in the cell

Secretion

Secreted protein to out of the cell

| Signal peptide | Target protein | Signal peptide tag |

| Target protein | Signal peptide tag |

Identification of
the corresponding signal peptide

Sequence analysis of
signal peptide tag

[Fig. 2]

[Fig. 3]

[Fig. 4]

[Fig. 5]

[Fig. 6]

[Fig. 7]

[Fig. 8]

[Fig. 9]

Construction of 9 clones :
1 target protein + 9 signal peptides/SP-tags

Transfection
Mixture:1~9

Collection of culture supernatant

Analysis of expression level of proteins by LC-MS/MS

Comparison of results

Transfection

Collection of culture supernatant

Analysis of expression level of proteins by western blot

EP 4 026 904 A1

[Fig. 10]

Expression result of 9 constructs

[Fig. 11]

Expression ranking of 9 constructs

| Ranking | Western blot | LC-MS/MS |
|---|---|---|
| 1 | 1 | 8 |
| 2 | 8 | 1 |
| 3 | 5 | 5 |
| 4 | 7 | 3 |
| 5 | 6 | 6 |
| 6 | 9 | ND (2, 4, 7, 9) |
| 7 | 4 | |
| 8 | 3 | |
| 9 | 2 | |

ND stands for Not Determined

[Fig. 12]

Relative level of SP-tagged target proteins

[Fig. 13]

[Fig. 14]

EP 4 026 904 A1

[Fig. 15]

EP 4 026 904 A1

| Ranking | # Signal peptide | Peak area value | Corresponding gene | Sequence |
|---|---|---|---|---|
| 1 | 56 | 6518624 | Glypican-6 | MPSWIGAVILPLLGLLLSLPAGA |
| 2 | 61 | 991527 | Serine protease inhibitor Kazal-type 8 | MKGICSDAILVLATSMWMAFA |
| 3 | 52 | 495054 | C-X-C motif chemokine 16 | MGRDLRPGSRVLLLLLLLLLLVYLTQPGNG |
| 4 | 55 | 467136 | Progonadoliberin-2 | MASSRRGLLLLLLLTAHLGPSEA |
| 5 | 33 | 425505 | Stromelysin-1 | MKSLPILLLLCVAVCSA |
| 6 | 29 | 415055 | IgA-inducing protein homolog | MCSYYHMKKRSVSGCNITIFAVMFSHLSAG |
| 7 | 35 | 401353 | Neutrophil gelatinase-associated lipocalin | MPLGLLWLGLALLGALHAQA |
| 8 | 2 | 390198 | Matrix metalloproteinase-17 | MRRRAARGPGPPPPGPGLSRLPLPLLLLLALGTRGGCA |
| 9 | 22 | 354084 | Chitinase-3-like protein 2 | MGATTMDQKSLWAGVVVLLLLQGGSA |
| 10 | 23 | 352607 | Contactin-associated protein-like 3 | MASVAWAVLKVLLLLPTQTWSPVGA |

94

[Fig. 16]

| Global Standard | G1 | G2 | G3 | G4 | G5 | G6 | G7 | G8 | G9 | G10 | G11 | G12 | G13 | G14 | G15 | G16 |
|---|---|---|---|---|---|---|---|---|---|---|---|---|---|---|---|---|
| Peak Area | 2.82E+05 | 2.42E+05 | 3.48E+05 | 2.91E+05 | 2.93E+05 | 3.50E+05 | 3.09E+05 | 1.95E+05 | 3.32E+05 | 2.50E+05 | 3.43E+05 | 3.34E+05 | 3.47E+05 | 3.12E+05 | 3.69E+05 | 4.16E+05 |

CV: 17.26%

16 Sample groups

Peak area value

[Fig. 17]

[Fig. 18]

Observed expression of the top 24 peptides

Observed expression of the bottom 24 peptides

[Fig. 19]

| Peptide number | Hit signal sequence | Amino acid sequence |
|---|---|---|
| Peptide_079 | Insulin growth factor-like family member 4 | MVPRISAAIFIFELLGSNS |
| Peptide_651 | Group IID secretory phospholipase A2 | MELALLCGLVVMAGVIPIQG |
| Peptide_103 | Alpha-2-antiplasmin | MALLWGLLVLSWSCLQGPCSVFSPVSA |
| Peptide_291 | Neural cell adhesion molecule 1 | MLQTKDLIWTLFFLGTAVS |
| Peptide_066 | Bile salt-activated lipase | MGRLQLVVLGLTCCWAVASA |
| Peptide_1113 | Peptidoglycan recognition protein | MSRRSMLLAWALPSLLRLGAA |
| Peptide_822 | Putative uncharacterized protein FP248 | MGTGGSLLCGCSLVLSCLCPSAS |
| Peptide_960 | Apolipoprotein(a)-like protein 2 | MEHKEVVLLLLLFLKSAPTET |
| Peptide_815 | Uncharacterized protein C19orf36 | MALLLCLVCLTAALA |
| Peptide_1199 | Stromal cell-derived factor 1 | MNAKVVVVLVLVLTALCLSDG |
| Peptide_054 | Uncharacterized protein FLJ37543 | MLAPLFLCCLRNLFRKLIS |
| Peptide_1006 | Interleukin-17 receptor E | MGSSRLAALLLPLLLIVIDLSDS |
| Peptide_104 | Pigment epithelium-derived factor | MQALVLLLCIGALLGHSSC |
| Peptide_796 | Follicular dendritic cell secreted peptide | MKKVLLLITAILAVAVG |

## INTERNATIONAL SEARCH REPORT

| International application No. |
|---|
| **PCT/KR2020/011885** |

### A. CLASSIFICATION OF SUBJECT MATTER

**C12N 15/10**(2006.01)i; **G01N 30/72**(2006.01)i; **G01N 33/68**(2006.01)i; **C40B 40/08**(2006.01)i; **C40B 50/06**(2006.01)i

According to International Patent Classification (IPC) or to both national classification and IPC

### B. FIELDS SEARCHED

Minimum documentation searched (classification system followed by classification symbols)

C12N 15/10; C07K 7/08; C12N 15/09; C12Q 1/68; G01N 30/72; G01N 33/68; C40B 40/08; C40B 50/06

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 단백질 분비(protein secretion), 시그널 펩타이드(signal peptide), 바코딩 서열 (barcoding sequence)

### C. DOCUMENTS CONSIDERED TO BE RELEVANT

| Category* | Citation of document, with indication, where appropriate, of the relevant passages | Relevant to claim No. |
|---|---|---|
| A | 이주협. 단백질 발현증대를 위한 분비시스템 개발 위한 최적 Signal Peptide 선별 및 적용 위한 HT 시스템. 오송첨단의료산업진흥재단. 기술설명서. 04 April 2019. non-official translation (LEE, Juhyeop. HT system for Selection and Application of Optimal Signal Peptide for Development of Secretion System for Increasing Protein Expression. Osong Medical Innovation Foundation. Technical manual). (URL: https://www.kbiohealth.kr/board.es?mid=a10201030300&bid=0022&act=view&list_no=8878&tag=&nPage=1). See pages 2 and 3. | 1-20 |
| A | KR 10-1875355 B1 (THE INDUSTRY & ACADEMIC COOPERATION IN CHUNGNAM NATIONAL UNIVERSITY (IAC) et al.) 05 July 2018. See entire document. | 1-20 |
| A | WO 2015-133074 A1 (YAMAGUCHI UNIVERSITY) 11 September 2015. See entire document. | 1-20 |
| A | US 2018-0080021 A1 (THE BOARD OF TRUSTEES OF THE LELAND STANFORD JUNIOR UNIVERSITY) 22 March 2018. See entire document. | 1-20 |

☑ Further documents are listed in the continuation of Box C.   ☑ See patent family annex.

| | |
|---|---|
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| **17 December 2020** | **17 December 2020** |

| Name and mailing address of the ISA/KR | Authorized officer |
|---|---|
| **Korean Intellectual Property Office** <br> **Government Complex Daejeon Building 4, 189, Cheongsa-ro, Seo-gu, Daejeon, Republic of Korea 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2020/011885** |

| C. | DOCUMENTS CONSIDERED TO BE RELEVANT | |
| --- | --- | --- |
| Category* | Citation of document, with indication, where appropriate, of the relevant passages | Relevant to claim No. |
| A | US 2017-0137806 A1 (YEDA RESEARCH AND DEVELOPMENT CO., LTD.) 18 May 2017. See entire document. | 1-20 |

Form PCT/ISA/210 (second sheet) (July 2019)

## INTERNATIONAL SEARCH REPORT
### Information on patent family members

| International application No. |
| --- |
| **PCT/KR2020/011885** |

| Patent document cited in search report | | | Publication date (day/month/year) | Patent family member(s) | | | Publication date (day/month/year) |
| --- | --- | --- | --- | --- | --- | --- | --- |
| KR | 10-1875355 | B1 | 05 July 2018 | None | | | |
| WO | 2015-133074 | A1 | 11 September 2015 | JP | 6460538 | B2 | 30 January 2019 |
| | | | | JP | 2017-133074 | A1 | 06 April 2017 |
| US | 2018-0080021 | A1 | 22 March 2018 | None | | | |
| US | 2017-0137806 | A1 | 18 May 2017 | US | 2015-0307874 | A1 | 29 October 2015 |
| | | | | US | 2020-0263168 | A1 | 20 August 2020 |
| | | | | WO | 2014-108850 | A2 | 17 July 2014 |
| | | | | WO | 2014-108850 | A3 | 27 November 2014 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Acta Biochim Biophys Sin (Shanghai),* 2011, vol. 43 (2), 96-102
- *Biochem Biophys Res Commun.,* 01 January 2010, vol. 391 (1), 931-5
- **MANIATIS et al.** Molecular Cloning, A Laboratory Manual. Cold Spring Harbor Press, 1988
- **AUSUBEL et al.** Current Protocols in Molecular Biology. John, Wiley & Sons, Inc, 1994

```
<110>    Osong Hi-Tech Medical Industry Promotion Foundation
         The Industry & Academic Cooperation in Chungnam National University

<120>    High-Throughput Screening Method using Individual Barcoding
         System for Identifying Optimal Signal Peptides to Enhance the
         Productivity of Protein

<130>    HP9504

<150>    KR 10-2019-0108722
<151>    2019-09-03

<160>    3

<170>    KoPatentIn 3.0

<210>    1
<211>    3
<212>    PRT
<213>    Artificial Sequence

<220>
<223>    Barcoding Sequence 3


<220>
<221>    VARIANT
<222>    (1)
<223>    1st Leu may be substituted with Pro, Ala, Trp, Tyr, Thr, Ser, Glu
         or Asp


<220>
<221>    VARIANT
<222>    (2)
<223>    2nd Leu may be substituted with Pro, Ala, Trp, Tyr, Thr, Ser, Glu
         or Asp


<220>
<221>    VARIANT
<222>    (3)
<223>    3rd Leu may be substituted with Pro, Ala, Trp, Tyr, Thr, Ser, Glu
         or Asp


<400>    1
Leu Leu Leu
  1


<210>    2
<211>    4
<212>    PRT
<213>    Artificial Sequence

<220>
<223>    Barcoding Sequence 4


<220>
<221>    VARIANT
<222>    (1)
```

<223> 1st Leu may be substituted with Pro, Ala, Trp, Tyr, Thr, Ser, Glu or Asp

<220>
<221> VARIANT
<222> (2)
<223> 2nd Leu may be substituted with Pro, Ala, Trp, Tyr, Thr, Ser, Glu or Asp

<220>
<221> VARIANT
<222> (3)
<223> 3rd Leu may be substituted with Pro, Ala, Trp, Tyr, Thr, Ser, Glu or Asp

<220>
<221> VARIANT
<222> (4)
<223> 4th Leu may be substituted with Pro, Ala, Trp, Tyr, Thr, Ser, Glu or Asp

<400> 2
Leu Leu Leu Leu
1

<210> 3
<211> 5
<212> PRT
<213> Artificial Sequence

<220>
<223> Barcoding Sequence 5

<220>
<221> VARIANT
<222> (1)
<223> 1st Leu may be substituted with Pro, Ala, Trp, Tyr, Thr, Ser, Glu or Asp

<220>
<221> VARIANT
<222> (2)
<223> 2nd Leu may be substituted with Pro, Ala, Trp, Tyr, Thr, Ser, Glu or Asp

<220>
<221> VARIANT
<222> (3)
<223> 3rd Leu may be substituted with Pro, Ala, Trp, Tyr, Thr, Ser, Glu or Asp

<220>
<221> VARIANT
<222> (4)

```
<223>     4th Leu may be substituted with Pro, Ala, Trp, Tyr, Thr, Ser, Glu
          or Asp


<220>
<221>     VARIANT
<222>     (5)
<223>     5th Leu may be substituted with Pro, Ala, Trp, Tyr, Thr, Ser, Glu
          or Asp


<400>     3
Leu Leu Leu Leu Leu
  1             5
```